(19) Europäisches Patentamt
European Patent Office
Office européen des brevets

(11) EP 1 315 710 B1

(12) **FASCICULE DE BREVET EUROPEEN**

(45) Date de publication et mention de la délivrance du brevet:
**25.04.2007 Bulletin 2007/17**

(21) Numéro de dépôt: **01965342.7**

(22) Date de dépôt: **23.08.2001**

(51) Int Cl.:
***C07D 307/84*** (2006.01) ***C07D 307/80*** (2006.01)
***C07D 405/04*** (2006.01) ***C07D 413/04*** (2006.01)
***C07D 413/14*** (2006.01) ***C07D 405/12*** (2006.01)
***A61K 31/41*** (2006.01) ***C07D 407/04*** (2006.01)

(86) Numéro de dépôt international:
**PCT/FR2001/002657**

(87) Numéro de publication internationale:
**WO 2002/016340 (28.02.2002 Gazette 2002/09)**

(54) **AMINOALKOXYBENZOYL-BENZOFURANES OU BENZOTHIOPHENES, LEUR PROCEDE DE PREPARATION ET LES COMPOSITIONS LES CONTENANT**

AMINOALKOXYBENZOYL-BENZOFURANE ODER BENZOTHIOPHENE, VERFAHREN ZU IHRER HERSTELLUNG UND DIE ENTHALTENDEN ZUSAMMENSETZUNGEN

AMINOALKOXYBENZOYL-BENZOFURAN OR BENZOTHIOPHENE DERIVATIVES, METHOD FOR PREPARING SAME AND COMPOSITIONS CONTAINING SAME

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **23.08.2000 FR 0010833**

(43) Date de publication de la demande:
**04.06.2003 Bulletin 2003/23**

(73) Titulaire: **Sanofi-Aventis**
**75013 Paris (FR)**

(72) Inventeurs:
- **ASSENS, Jean-Louis**
  **F-34790 Grabels (FR)**
- **BERNHART, Claude**
  **F-34980 Saint Gely du Fesc (FR)**
- **CABANEL-HAUDRICOURT, Frédérique**
  **F-34570 Pignan (FR)**
- **NISATO, Dino**
  **F-34680 Saint Georges d'Orques (FR)**

(74) Mandataire: **Weber, Mathieu et al**
**Sanofi-Aventis**
**174 avenue de France**
**75013 Paris (FR)**

(56) Documents cités:
**EP-A- 0 338 746**
**EP-A- 0 471 609**
**EP-A- 0 617 030**
**EP-A- 0 752 249**
**EP-A- 0 835 871**
**WO-A-90/02743**
**WO-A-94/29289**
**WO-A-95/10513**
**US-A- 3 248 401**
**US-A- 4 806 663**

- **MARTIN M J ET AL: "Versatile Raloxifene Triflates" BIOORGANIC & MEDICINAL CHEMISTRY LETTERS, OXFORD, GB, vol. 7, no. 7, 8 avril 1997 (1997-04-08), pages 887-892, XP004136150 ISSN: 0960-894X**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 315 710 B1

Printed by Jouve, 75001 PARIS (FR)

## Description

La présente invention se rapporte, d'une manière générale, à de nouveaux dérivés hétérocycliques ainsi qu'à leur procédé de préparation.

En particulier, l'invention concerne de nouveaux dérivés de benzofurane ou de benzothiophène, lesquels peuvent être représentés par la formule générale :

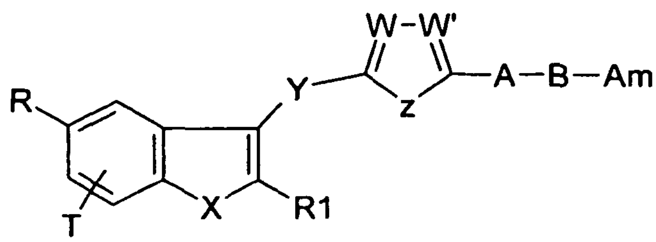

(1)

ainsi que leurs sels pharmaceutiquement acceptables, dans laquelle :

A représente -O- , -S- ou

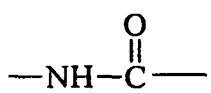

B représente un groupement alkylène, linéaire ou ramifié, en $C_I$-$C_5$, éventuellement substitué par un groupement hydroxyle,
T représente l'hydrogène
R représente :

- le groupement cyano ou hydroxyméthyle
- un groupement oxime de formule :

$R_4$-O-N=CH-

dans laquelle $R_4$ représente un groupement alkyle en $C_1$-$C_4$

- un groupement carboxyle de formule générale :

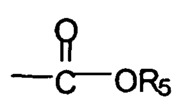

(a)

dans laquelle $R_5$ représente l'hydrogène ou un atome de métal alcalin, un groupement alkyle en $C_1$-$C_{10}$ ,linéaire ou ramifié, un groupement cycloalkyle en $C_3$-$C_6$, ou $R_5$ représente le groupement de formule générale :

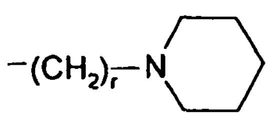

(a-1)

dans laquelle r représente 1 à 4

- un groupement carboxyle de formule générale :

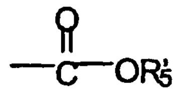

(b)

dans laquelle R'$_5$ représente un groupement pipéridinyle éventuellement N-substitué par un groupement alkyle en $C_1$-$C_4$ ou l'un des groupements de formule générale :

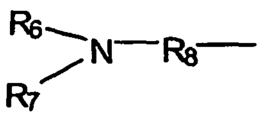

ou

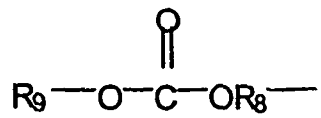

(c) (d)

dans lequel $R_6$ et $R_7$, identiques ou différents, représentent un groupement alkyle en $C_1$-$C_4$, $R_8$ représente un groupement alkylène, linéaire ou ramifié, en $C_1$-$C_6$, et $R_9$ représente l'hydrogène, un atome de métal alcalin, un groupement alkyle en $C_1$-$C_4$,

- un groupement aminocarbonyle de formule :

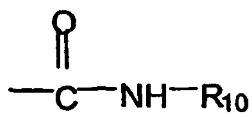

(e)

dans lequel $R_{10}$ représente l'hydrogène, un groupement alkyle en $C_1$-$C_4$, hydroxyle ou amino, un groupement (c) ci-dessus ou l'un des groupements :

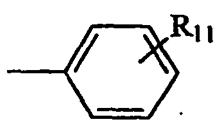

ou

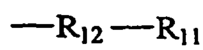

(f) (g)

dans lequel $R_{11}$ représente un groupement (a) et $R_{12}$ représente un radical alkylène en $C_1$-$C_6$.,

- un groupement de formule :

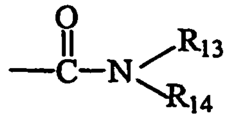

(h)

dans laquelle $R_{13}$ et $R_{14}$, identiques ou différents, représentent un radical alkyle en $C_1$-$C_4$, ou un groupement hydroxyalkyle en $C_1$-$C_4$

- l'un des groupements de formule ci-dessous :

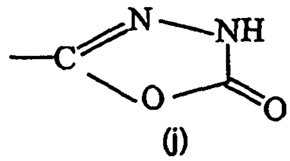

(j)

ou

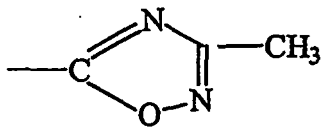

(k)

ou

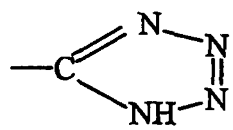

(l)

$R_1$ représente un groupement alkyle en $C_1$-$C_6$, cycloalkyle en $C_3$-$C_6$, phényle, benzyle ou un groupement de formule :

$$-(CH_2)_p-R_{11} \quad (m)$$

dans laquelle $R_{11}$ a la même signification que précédemment et p représente 1à4
Am représente un groupement azoté de formule :

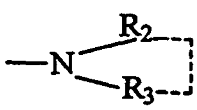

(Am₁)

ou

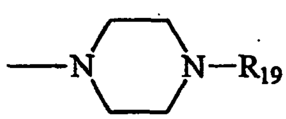

(Am₂)

ou

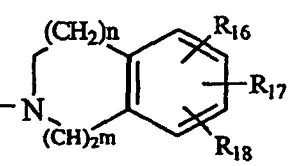

(Am₃)

dans laquelle :

$R_2$ représente l'hydrogène, un groupement alkyle en $C_1$-$C_6$, linéaire ou ramifié, éventuellement substitué par un groupement hydroxyle, un groupement (m), un groupement cycloalkyle en $C_3$-$C_6$ ou un groupement benzyle,
$R_3$ représente un groupement alkyle en $C_1$-$C_6$, linéaire ou ramifié, éventuellement substitué par un groupement hydroxyle, un groupement cycloalkyle en $C_3$-$C_6$, un groupement (m), un groupement benzyle ou un groupement phényle de formule :

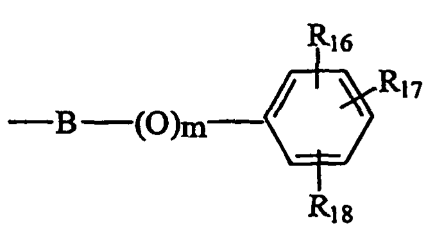

(n)

$R_{16}$, $R_{17}$ et $R_{18}$, identiques ou différents, représentent l'hydrogène, un groupement hydroxyle, nitro, amino, alkoxy en $C_1$-$C_4$ ou alkylsulfonamido en $C_1$-$C_4$,
$R_{19}$ représente l'hydrogène, un groupement alkyle en $C_1$-$C_5$, le groupement diphénylméthyle, un groupement mono-,

di- ou triméthylphényle, un groupement mono-, di- ou triméthoxyphényle, un groupement (a), un groupement (b) ou un groupement (c),
m et n représentent chacun 0 ou 1,
$R_2$ et $R_3$, lorsqu'ils sont pris ensemble, représentent un groupement alkylène en $C_3$-$C_{10}$, linéaire ou ramifié, éventuellement substitué par le groupement hydroxyle, par un groupement (a) ou par un groupement (m) et éventuellement interrompu par -O-,
ces alternatives $R_2$ et $R_3$ indépendants ou $R_2$ et $R_3$ pris ensemble étant représentées dans la formule ($Am_1$) par le symbole

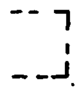

situé entre $R_2$ et $R_3$
W, W' et Z sont tels que :

- lorsque W et W', identiques représentent CH, Z représente -O- ou -S-
- lorsque W représente CH et W' représente C-$R_{20}$, Z représente

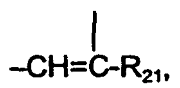

$R_{20}$ et $R_{21}$, étant identiques ou différents et représentant l'hydrogène, un atome d'halogène par exemple fluor, chlore ou brome, un radical alkyle en $C_1$-$C_4$ tel que méthyle, ou un radical alkoxy en $C_1$-$C_4$ tel que méthoxy,
X représente -O- ou -S-
Y représente un radical - CO -, - $CH_2$-, un radical de formule

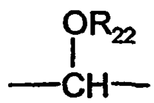

(73)

dans laquelle $R_{22}$ représente l'hydrogène, un radical alkyle en $C_1$-$C_4$ ou acyle de formule :

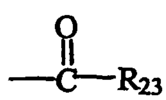

(74)

dans laquelle $R_{23}$ représente un radical alkyle en $C_1$-$C_4$,
ou Y représente

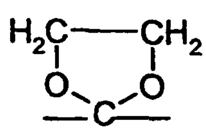

étant entendu que l'ensemble formé par les groupements R, $R_1$ et Am contient 0, 1 ou 2 groupements (a),
ces dérivés de benzofuranne ou de benzothiophène étant sous forme d'isomères individuels ou de mélange de ceux-ci, à l'exclusion du composé 2-phényl-3-[4-(2-pipéridin-1-yl-éthoxy)-benzoyl]-benzo[b]thiophène-6-carboxylate de méthyle de formule (72) suivante :

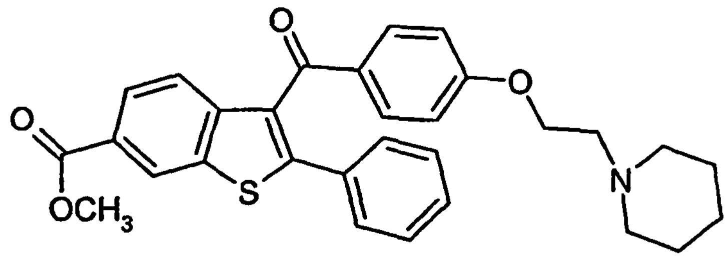

(72)

Des classes de composés préférés de l'invention peuvent être représentés par les composés de formule (1) dans laquelle :

- soit R représente un groupement isopropoxycarbonyle
- soit Am représente un groupement diéthylpipéridino.

De même, une classe particulière de composés de formule I est celle dans laquelle
Y représente un radical -CO-

Une autre classe de composés préférés de formule (1) est celle dans laquelle

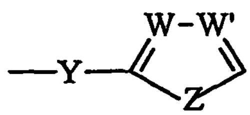

représente un radical benzoyle.

De même, une classe particulière de composés de formule (1) est celle dans laquelle l'entité :

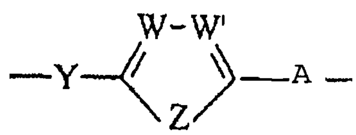

représente un radical 4-oxybenzoyle.

De même, les composés de formule (1) dans laquelle X représente - O - sont des composés préférés ainsi que ceux dans lesquels la chaîne :

- A - B - Am

se trouve en position 4.

Enfin, les composés de formule (1) dans laquelle $R_1$ représente n-butyle, B représente un groupement propylène et Am représente un groupement diéthylpipéridinyle, en particulier 3,5-diéthylpipéridinyle peuvent être considérés également comme préférés.

Des composés de formule (1) peuvent se représenter sous forme d'isomères optiques ou géométriques par exemple les composés en question dans lesquels Am représente un groupement diéthylpipéridinyle ou dans lesquels R représente un groupement - $R_4$ - O - N = CH -

En conséquence, l'invention se rapporte à la fois aux isomères individuels des composés de formule (1) ainsi qu'à leurs mélanges notamment le mélange racémique.

L'invention se rapporte également aux sels pharmaceutiquement acceptables des composés de formule (1) formés à partir d'un acide organique ou inorganique.

Comme exemples de sels organiques de ce genre, on peut citer les oxalate, maléate, fumarate, méthanesulfonate, benzoate, ascorbate, pamoate, succinate, hexamate, bisméthylènesalicylate, éthanedisulfonate, acétate, propionate, tartrate, salicylate, citrate, gluconate, lactate, malate, cinnamate, mandélate, citraconate, aspartate, palmitate, stéarate, itaconate, glycolate, p-aminobenzoate, glutamate, benzènesulfonate, p-toluènesulfonate et théophilline acétate ainsi que les sels formés à partir d'un acide aminé tel que le sel de lysine ou l'histidine.

Comme sels inorganiques de ce genre, on peut citer les chlorhydrate, bromhydrate, sulfate, sulfamate, phosphate et nitrate.

On a trouvé que les composés de l'invention possèdent de remarquables propriétés pharmacologiques notamment des propriétés anti-arythmiques puisqu'ils se sont révélés capables de supprimer ou de prévenir les troubles du rythme ventriculaire et auriculaire. La plupart des composés de l'invention ont des propriétés électrophysiologiques des classes 1,2,3 et 4 de la classification de Vaughan-Williams qui confèrent des propriétés bradycardisantes, antihypertensives et anti-adrénergiques $\alpha$ et $\beta$ non compétitives. De plus la plupart des composés ont également révélé des propriétés anti-oxydantes, une affinité pour les récepteurs sigma et une capacité à augmenter la synthèse du NO.

**[0015]** Par ailleurs, ces composés de l'invention manifestent des propriétés inhibitrices de différents agents hormonaux tels que par exemple l'angiotensine II, l'arginine vasopressine, le neuropeptide Y ou l'endothéline.

Ces propriétés sont capables de rendre les composés en question très utiles dans le traitement de certains syndromes pathologiques du système cardio-vasculaire en particulier dans le traitement de l'angine de poitrine, de l'hypertension, de l'arythmie en particulier atriale, ventriculaire ou supraventriculaire, de l'insuffisance circulatoire cérébrale. De même, les composés de l'invention pourront être utilisés dans le traitement de l'insuffisance cardiaque, de l'infarctus du myocarde compliqué ou non d'insuffisance cardiaque ou pour la prévention de la mortalité post-infarctus.

Dans le domaine antitumoral, les composés de l'invention pourront être utiles comme potentialisateurs d'anticancéreux.

En conséquence, l'invention se rapporte également à un médicament, caractérisé en ce qu'il comprend un composé dérivé de benzofurane ou de benzothiophène, ou un sel pharmaceutiquement acceptable de ce dernier, selon l'invention.

En conséquence, l'invention se rapporte également à des compositions pharmaceutiques ou vétérinaires contenant comme principe actif, au moins un composé de l'invention, en association avec un véhicule pharmaceutique ou excipient approprié.

Selon la voie d'administration choisie, la posologie journalière pour un être humain pesant 60 kg se situera entre 2 et 2000 mg de principe actif, en particulier entre 50 et 500 mg de principe actif.

Les composés de formule (1) peuvent être préparés selon les méthodes suivantes :

I. Les composés de formule (1) dans laquelle

- Y représente le groupement- CO -
- $R_1$ ne comporte pas de groupement carboxylique ou carboxylate de métal alcalin
- Am représente un groupement ($Am_1$) ou ($Am_2$), ce groupement ne comportant pas de groupement carboxylique ou carboxylate de métal alcalin ou Am représente un groupement ($Am_3$) dans lequel $R_{16}$ et/ou $R_{17}$ et/ou $R_{18}$ sont autres qu'un groupement amino ou alkylsulfonamido en $C_1$-$C_4$,

peuvent être obtenus :

A. - Dans le cas où R représente un groupement cyano ou hydroxyméthyle, un groupement (a) dans lequel $R_5$ représente un groupement alkyle en $C_1$-$C_{10}$ ou cycloalkyle en $C_3$-$C_6$ ou encore le groupement (k), en faisant réagir, en présence d'un agent basique tel qu'un carbonate ou hydroxyde de métal alcalin, un dérivé cétonique de formule générale :

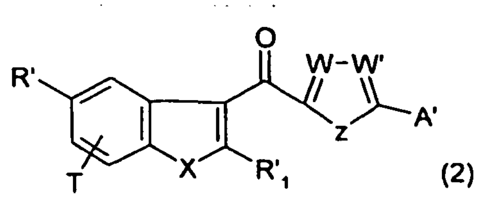

(2)

dans laquelle $R'_1$ représente un groupement alkyle en $C_1$-$C_6$, ou un groupement (m) ne comportant pas de groupement carboxylique ou carboxylate de métal alcalin, T, W, W, X et Z ont la même signification que précédemment, A' représente OH, SH ou $NH_2$ et R' représente le groupement cyano ou hydroxyméthyle, le groupement (k) ou un groupement $-CO_2R''_5$ dans lequel $R''_5$ représente un radical alkyle en $C_1$-$C_{10}$ ou cycloalkyle en $C_3$-$C_6$, avec un composé de formule générale :

$R_{24}$-B-Am' (3)

dans laquelle Am' représente un groupement ($Am_1$) ou ($Am_2$), ce groupement ne comportant pas de groupement

carboxylique ou carboxylate de métal alcalin ou encore un groupement ($Am_3$) dans lequel $R_{16}$ et/ou $R_{17}$ et/ou $R_{18}$ sont autres qu'un groupement amino ou un groupement alkylsulfonamido en $C_1$-$C_4$, B a la même signification que précédemment et $R_{24}$ représente :

un atome d'halogène tel que par exemple un atome de chlore, un radical alkylsulfonyloxy en $C_1$-$C_4$ ou arylsulfonyloxy en $C_6$-$C_{10}$, ce qui permet d'obtenir, sous forme de base libre, les composés désirés de formule I dans laquelle A représente -O-ou-S-

La réaction se déroule habituellement à la température de reflux du solvant utilisé ou à une température n'excédant pas 90°C, ce solvant pouvant être par exemple un solvant polaire tel que le N, N-diméthylformamide ou une cétone telle que par exemple la méthyléthylcétone.

B. - Dans le cas où R représente le groupement cyano, un groupement $R_4$ - O - N = CH -, un groupement (a) dans lequel $R_5$ représente un groupement alkyle en $C_1$-$C_{10}$ ou cycloalkyle en $C_3$-$C_6$ ou encore le groupement (k), en faisant réagir un composé de formule générale :

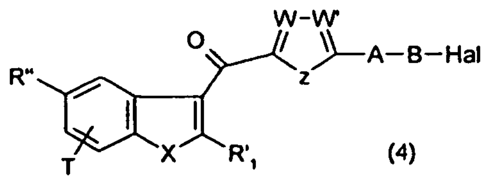

(4)

dans laquelle A, B, R'$_1$, T, X, W, W et Z ont la même signification que précédemment, et R" représente le groupement cyano, le groupement (k), un groupement $R_4$ - O - N = CH - ou un groupement - $CO_2$ R"$_5$ dans lequel R"$_5$ a la même signification que précédemment, et Hal représente un atome d'halogéne tel que par exemple chlore ou brome, avec un composé de formule générale :

H-Am' (5)

éventuellement sous forme de sel, par exemple de chlorhydrate, dans laquelle Am' a la même signification que précédemment, la réaction ayant lieu en présence d'un agent basique tel qu'un carbonate ou hydroxyde de métal alcalin ou un excès d'amine de formule (5) sous forme basique, ce qui fournit les composés désirés de formule (1) sous forme de base libre.

Généralement, la réaction se déroule à la température de reflux du milieu et dans un solvant polaire tel que le N,N-diméthylformamide, l'acétonitrile ou la méthyléthylcétone ou un solvant apolaire tel que le benzène ou le toluène.

C. - Dans le cas où R représente le groupement cyano, un groupement $R_4$ - O - N = CH -, un groupement (a) dans lequel $R_5$ représente un groupement alkyle en $C_1$-$C_{10}$ ou cycloalkyle en $C_3$-$C_6$ ou encore le groupement (k), en faisant réagir un composé de formule générale :

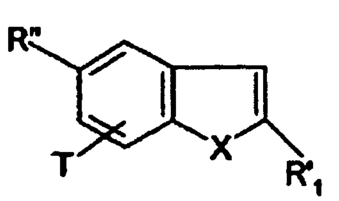

(6)

dans laquelle R", R'$_1$, T et X ont la même signification que précédemment, avec un halogénure de formule générale :

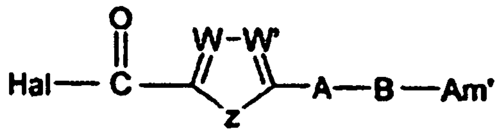

(7)

dans laquelle A, B, Am', W, W', Z et Hal ont la même signification que précédemment, la réaction ayant lieu éventuellement en présence d'un acide de Lewis tel que le chlorure d'aluminium, le chlorure stannique, le chlorure ferrique ou le trifluorométhanesulfonate d'argent, ce qui fournit les composés désirés de formule (1) sous forme de base libre.

Habituellement, la réaction ci-dessus se déroule dans un solvant apolaire tel qu'un composé halogéné par exemple le dichlorométhane ou le dichloroéthane et à une température comprise entre la température ambiante et la température de reflux.

Alternativement, on peut obtenir les composés de formule (1) dont le groupement Am représente un groupement ($Am_1$) dans lequel $R_2$ et $R_3$ sont différents, en transformant une amine secondaire de formule (1) comportant un groupement ($Am_1$) de formule $-NH-R_2$, en amine tertiaire par réaction au moyen d'un composéde formule générale:

$Hal-R_3$ (8)

dans laquelle Hal représente un atome d'halogène, de préférence brome et $R_3$ a la même signification que précédemment, la réaction ayant lieu de préférence à la température de reflux, en présence d'un agent basique tel qu'un carbonate ou hydroxyde de métal alcalin, ce qui fournit les composés désirés de formule (1) sous forme de base libre.

Les composés de formule (1) dans laquelle R représente un groupement oxime de formule $R_4$ - O - N = CH - peuvent se présenter sous forme de stéréoisomères.

Les méthodes décrites précédemment sous B) et C) permettent d'obtenir ces dérivés oxime sous forme de mélanges d'isomères. Toutefois, ces isomères peuvent être produits sous forme séparée par mise en oeuvre de méthodes connues telles que par exemple chromatographie ou précipitation.

D. - Dans le cas où R représente le groupement (j) en faisant réagir, ce préférence à la température de reflux du milieu, un composé de formule générale :

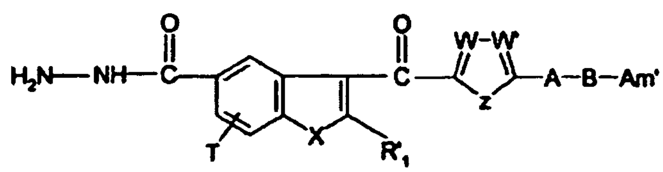

(9)

dans laquelle A, B, T, W, W', X et Z ont la même signification que précédemment, avec le phosgène, ce qui fournit les composés désirés de formule (1) sous forme de chlorhydrate que l'on peut traiter, si nécessaire, avec un agent basique tel qu'un hydroxyde de métal alcalin ou un carbonate de métal alcalin, ce qui fournit les composés désirés sous forme de base libre.

Les dérivés benzofuraniques ou benzothiophéniques de formule (1), qui répondent également à la formule générale :

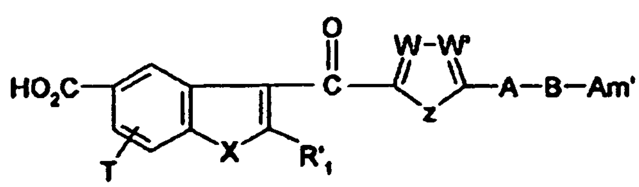

(10)

dans laquelle A, Am', B, $R'_1$, T, X, W, W' et Z ont la même signification que précédemment, sont eux-mêmes intermédiaires de synthèse pour la préparation d'autres composés de formule (1) dans laquelle Y représente

- CO - et Am représente un groupement ($Am_1$) ou ($Am_2$), ce groupement ne comportant pas de groupement carboxylique ou carboxylate de métal alcalin ou encore un groupement ($Am_3$) dans lequel $R_{16}$ et/ou $R_{17}$ et/ou $R_{18}$ sont autres qu'un groupement amino ou un groupement alkylsulfonamido en $C_1$-$C_4$ et dans laquelle $R_1$ ne comporte pas de groupement carboxylique ou carboxylate de métal alcalin.

A cet effet, on peut mettre en oeuvre les méthodes suivantes au départ des composés de formule (10) en question pour obtenir des composés souhaités de formule (1), c'est-à-dire :

E. - Dans le cas où R représente un groupement (b) dans lequel R'$_5$ représente un groupement (c) :

a) si ce groupement (c) est du type dialkylaminoalkyle primaire, on fait réagir, de préférence dans un solvant polaire tel que le N,N-diméthylformamide et habituellement à une température comprise entre 30 et 50°C, un composé de formule (10) après protection de la fonction amine lorsque Am' représente un groupement ($Am_1$) dans lequel $R_2$ représente l'hydrogène, et ce, avec un alcool de formule générale:

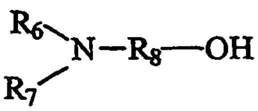

**(11)**

dans laquelle $R_6$ et $R_7$ ont la même signification que précédemment et $R_8$ représente un groupement alkylène linéaire en $C_1$-$C_6$, la réaction ayant lieu en présence de carbonyl diimidazole et de 1,8-diazabicyclo [5.4.0]undec-7ene, puis on déprotège , si nécessaire, le composé formé, ce qui fournit, sous forme de base libre, les composés désirés de formule (1).

b) si ce groupement (c) est du type dialkylaminoalkyle secondaire ou tertiaire, on fait réagir, de préférence dans un solvant aprotique tel qu'un hydrocarbure halogéné et généralement à la température de reflux du milieu, un composé de formule (10) après protection de la fonction amine lorsque Am' représente un groupement ($Am_1$) dans lequel $R_2$ représente l'hydrogène et ce, avec un agent halogénant tel que le chlorure de thionyle, pour obtenir un halogénure d'acyle que l'on traite ensuite, de préférence à la température ambiante, avec un alcool de formule (11) ci-dessus dans laquelle $R_6$ et $R_7$ ont la même signification que précédemment et $R_8$ représente un groupement alkylène secondaire ou tertiaire en $C_2$-$C_6$, puis on déprotège, si nécessaire, le composé formé, ce qui fournit les composés de formule (1) sous forme d'halohydrate ou sous forme de base libre, lorsque le composé de formule (10) est en excès, halohydrate que l'on peut traiter, si nécessaire, avec un agent basique tel qu'un hydroxyde de métal alcalin ou un carbonate de métal alcalin, pour obtenir les composés désirés sous forme de base libre.

F. - Dans le cas où R représente soit un groupement (a) dans lequel $R_5$ représente un groupement alkyle en $C_1$-$C_{10}$ ou cycloalkyle en $C_3$-$C_6$ soit un groupement (b) dans lequel R'$_5$ représente un groupement pipéridinyle éventuellement N-substitué par un groupement alkyle en $C_1$-$C_4$ ou dans lequel R'$_5$ représente un groupement (d) ne comportant pas de groupement carboxylique ou carboxylate de métal alcalin, on fait réagir un composé de formule (10), après protection de la fonction amine lorsque Am' représente un groupement ($Am_1$) dans lequel $R_2$ représente l'hydrogène, et ce, de préférence dans un hydrocarbure halogéné et généralement à la température de reflux du milieu, avec un agent halogénant tel que le chlorure de thionyle, pour obtenir un halogénure d'acyle que l'on traite ensuite avec un alcool de formule générale :

R'''$_5$-OH (12)

dans laquelle R'''$_5$ représente un groupement alkyle en $C_1$-$C_{10}$, cycloalkyle en $C_3$-$C_6$ ou un groupement (b) dans lequel R'$_5$ représente un groupement pipéridinyle éventuellement N-substitué par un groupement alkyle en $C_1$-$C_4$ ou R'$_5$ représente un groupement (d), ne comportant pas de groupement carboxylique ou carboxylate de métal alcalin, puis on déprotège, si nécessaire, le composé formé, ce qui fournit les composés désirés de formule (1) sous forme d'halohydrate ou sous forme de base libre lorsque le composé de formule (10) est en excès, halohydrate que l'on peut traiter, si nécessaire, avec un agent basique tel qu'un hydroxyde de métal alcalin ou un carbonate de métal alcalin, pour obtenir les composés désirés sous forme de base libre.

**G. -** Dans le cas où R représente un groupement (e) dans lequel $R_{10}$ représente un groupement (f) ne comportant pas de groupement carboxylique ou carboxylate de métal alcalin, on fait réagir un composé de formule (10) après protection de la fonction amine lorsque Am' représente un groupement ($Am_1$) dans lequel $R_2$ représente l'hydrogène et ce, de préférence dans un hydrocarbure halogéné et généralement à la température de reflux, avec un agent halogénant tel que le chlorure de thionyle, pour obtenir un chlorure d'acyle que l'on traite ensuite, de préférence à la température ambiante, avec un composé de formule :

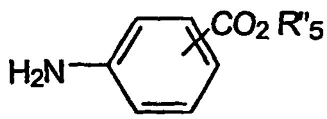

(13)

dans laquelle R"$_5$ a la même signification que précédemment, puis on déprotège si nécessaire, le composé formé ce qui fournit, sous forme de base libre, les composés désirés de formule (1) dans laquelle $R_{10}$ représente un groupement (f) dont le groupement $R_{11}$ représente un groupement (a) dans lequel $R_5$ représente un groupement alkyle en $C_1$-$C_4$ ou cycloalkyle en $C_3$-$C_6$.

H. - Dans le cas où R représente un groupement (e) dans lequel $R_{10}$ représente un groupement alkyle en $C_1$-$C_4$, un groupement amino ou un groupement (c), on fait réagir un composé de formule (10) après protection de la fonction amine lorsque Am' représente un groupement ($Am_1$) dans lequel $R_2$ représente l'hydrogène et ce, de préférence dans un hydrocarbure halogéné et généralement à la température de reflux, avec un agent halogénant tel que le chlorure de thionyle pour obtenir un halogénure d'acyle que l'on traite ensuite de préférence à la température ambiante, avec une amine de formule générale :

$R'_{10}$-$NH_2$ (14)

ou

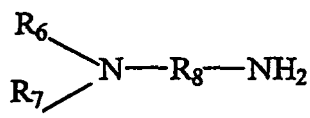

(15)

dans laquelle $R_6$, $R_7$ et $R_8$ ont la même signification que précédemment et $R'_{10}$ représente un radical alkyle en $C_1$-$C_4$ ou amino, puis on déprotège, si nécessaire, le composé formé, ce qui fournit éventuellement après traitement basique, le composé désiré de formule (1) sous forme d'halohydrate ou sous forme de base libre lorsque le composé de formule (10) est en excès, halohydrate que l'on peut traiter, si nécessaire, avec un agent basique tel qu'un hydroxyde de métal alcalin ou un carbonate de métal alcalin, pour obtenir les composés désirés sous forme de base libre.

**I**. - Dans le cas où R représente un groupement (e) dans lequel $R_{10}$ représente un groupement (g) ne comportant pas de groupement carboxylique ou carboxylate de métal alcalin, on fait réagir un composé de formule (10) après protection de la fonction amine lorsque Am' représente un groupement ($Am_1$) dans lequel $R_2$ représente l'hydrogène et ce, de préférence dans un solvant polaire ou apolaire tel que le N,N-diméthylformamide ou un hydrocarbure halogéné par exemple le dichlorométhane, avec un sel d'un composé de formule générale :

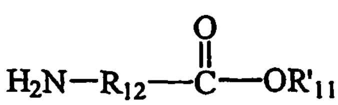

(16)

dans laquelle $R_{12}$ a la même signification que précédemment et $R'_{11}$ représente un radical alkyle en $C_1$-$C_4$ ou cycloalkyle en $C_3$-$C_6$, la réaction ayant lieu en présence d'un capteur d'acide tel qu'une amine par exemple la triéthylamine, puis on déprotège, si nécessaire, le composé formé, ce qui fournit, sous forme de base libre, les composés désirés de formule (1) dans laquelle $R_{10}$ représente un groupement (g) dont le groupement $R_{11}$ représente un groupement (a) dans lequel $R_5$ représente un groupement alkyle en $C_1$-$C_4$ ou cycloalkyle en $C_3$-$C_6$

J. - Dans le cas où R représente un groupement (h), en faisant réagir un composé de formule (10) après protection de la fonction amine lorsque Am' représente un groupement ($Am_1$) dans lequel $R_2$ représente l'hydrogène et ce, de préférence dans un hydrocarbure halogéné et généralement à la température de reflux du milieu, avec un agent halogénant tel que le chlorure de thionyle, pour obtenir un halogénure d'acyle que l'on traite ensuite avec une amine de formule générale :

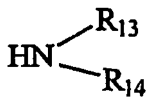

(17)

dans laquelle $R_{13}$ et $R_{14}$ ont la même signification que précédemment, puis on déprotège, si nécessaire le composé formé, ce qui fournit un sel du composé désiré de formule (1) que l'on traite par un agent basique approprié tel qu'un carbonate de métal alcalin, pour obtenir sous forme de base libre, les composés désirés de formule (1).

**K.-** Dans le cas où R représente un groupement (e) dans lequel $R_{10}$ représente le groupement hydroxyle, on fait réagir un composé de formule (10) après protection de la fonction amine lorsque Am' représente un groupement ($Am_1$) dans lequel $R_2$ représente l'hydrogène, avec un sel de benzyloxyamine par exemple le chlorhydrate, en présence d'un capteur d'acide, par exemple une amine telle que la triéthylamine et de benzotriazol-1-yloxy-tris(diméthylamino)phosphonium hexafluorophosphate, ci-après BOP, puis on déprotège, si nécessaire, le composé formé, ce qui fournit, des dérivés benzyloxyaminocarbonyle que l'on hydrogène en présence d'un catalyseur approprié, par exemple le charbon palladié ou le noir de platine, pour obtenir, sous forme de base libre, les composés désirés de formule (1).

Dans les procédés **E.** à **K.** ci-dessus, la protection de la fonction amine du composé de formule (10), c'est-à-dire la protection envisagée lorsque Am' représente un groupement ($Am_1$) dans lequel $R_2$ représente l'hydrogène, peut être obtenue par exemple par traitement au moyen d'un composé permettant la fixation d'un groupement aisément éliminable notamment au moyen de 9-fluorenylméthylchloroformiate et la déprotection s'opère par la suite par traitement avec une amine secondaire, par exemple la pipéridine ou la diéthylamine et ce dans un solvant approprié par exemple le N,N-diméthylformamide.

D'autres composés de formule (1) peuvent être utilisés comme intermédiaires synthèse de composés de l'invention notamment les dérivés cyano qui réponde également à la formule générale :

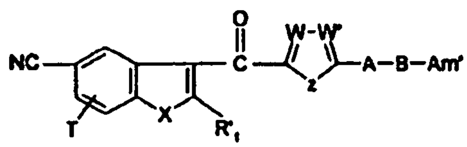

(18)

dans laquelle Am', A, B, $R'_1$, T, W, W', X et Z ont la même signification que précédemment.

Ainsi, on peut mettre en oeuvre les méthodes suivantes au départ des composés de formule (18) en question pour la préparation des composés de formule (1) dans laquelle Y représente - CO - , Am représente un groupement ($Am_1$) ou ($Am_2$), ce groupement ne comportant pas de groupement carboxylique ou carboxylate de métal alcalin ou encore un groupement ($Am_3$) dans lequel $R_{16}$ et/ou $R_{17}$ et/ou $R_{18}$ sont autres qu'un groupement amino ou un groupement alkylsulfonamido en $C_1$-$C_4$, et dans laquelle $R_1$ ne comporte pas de groupement carboxylique ou carboxylate de métal alcalin, c'est-à-dire :

L. - Dans le cas où R représente un groupement (e) dans lequel $R_{10}$ représente l'hydrogène, on hydrolyse un composé de formule (18) en présence d'un acide fort tel que par exemple l'acide sulfurique et généralement à la température ambiante, ce qui fournit, sous forme de base libre, les composés désirés de formule (1).

M. - Dans le cas où R représente le groupement (I), on fait réagir de préférence dans un solvant aprotique tel qu'un hydrocarbure aromatique par exemple le benzène

ou le toluène et habituellement à la température de reflux du milieu, un composé de formule (18) avec le tributylazido étain, ce qui fournit, sous forme de base libre, les composés désirés de formule (1).

II. Les composés de formule (1) dans laquelle :

- Y représente le groupement -CO-
- $R_1$ ne comporte pas de groupement carboxylique ou carboxylate de métal alcalin
- Am représente un groupement ($Am_1$) ne comportant pas de groupement carboxylique ou carboxylate de métal alcalin et dans lequel $R_{16}$ et/ou $R_{17}$ et/ou $R_{18}$ représentent le groupement amino ou un groupement alkylsulfonamido en $C_1$-$C_4$ ou encore Am représente un groupement ($Am_3$) dans lequel $R_{16}$ et/ou $R_{17}$ et/ou $R_{18}$ représentent le groupement amino ou un groupement alkylsulfonamido en $C_1$-$C_4$, peuvent être obtenus de la manière suivante :

a) Lorsque $R_{16}$ et/ou $R_{17}$ et/ou $R_{18}$ représentent le groupement amino, en hydrogénant, de préférence à température ambiante et à pression normale, un composé nitro de formule :

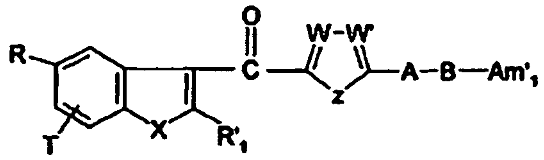

(19)

dans laquelle A, B, $R'_1$, T, W, W', X et Z ont la même signification que précédemment, R a la même signification que précédemment mais ne comporte pas de groupement carboxylique ou carboxylate de métal alcalin et $Am'_1$ représente soit un groupement ($Am_1$) ne comportant pas de groupement carboxylique ou carboxylate de métal alcalin et dans lequel $R_{16}$ et/ou $R_{17}$ et/ou $R_{18}$ représentent le groupement nitro, soit un groupement ($Am_3$) dans lequel $R_{16}$ et/ou $R_{17}$ et/ou $R_{18}$ représentent le groupement nitro et ce, en présence d'un catalyseur approprié tel que le nickel de Raney, l'oxyde de platine ou de palladium ou le zinc en milieu acide chlorhydrique et de préférence dans un solvant polaire, par exemple un alcool, ce qui fournit, sous forme de base libre, les composés désirés de formule (1).

b) Lorsque $R_{16}$ et/ou $R_{17}$ et/ou $R_{18}$ représentent un groupement alkylsulfonamido en $C_1$-$C_4$, en faisant réagir un composé amino de formule :

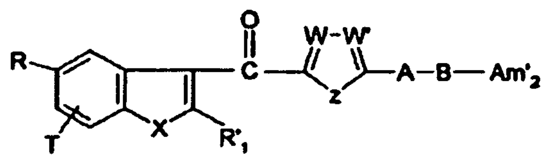

(20)

dans laquelle A, B, $R'_1$, T, W, W', X et Z ont la même signification que précédemment, R a la même signification que précédemment mais ne comporte pas de groupement carboxylique ou carboxylate de métal alcalin et $Am'_2$ représente soit un groupement ($Am_1$) ne comportant pas de groupement carboxylique ou carboxylate de métal alcalin et dans lequel $R_{16}$ et/ou $R_{17}$ et/ou $R_{18}$ représentent le groupement amino, soit un groupement ($Am_3$) dans lequel $R_{16}$ et/ou $R_{17}$ et/ou $R_{18}$ représentent le groupement amino, avec un halogénure de formule générale :

$Hal\text{-}SO_2\text{-}R'_{16}$ (21)

ou un anhydride de formule générale :

$(R'_{16}SO_2)_2O$ (22)

dans laquelle $R'_{16}$ représente un radical alkyle, linéaire ou ramifié, en $C_1$-$C_4$, la réaction se déroulant de préférence à la température ambiante et dans un solvant organique par exemple un solvant aprotique et éventuellement en présence d'un accepteur d'acide tel qu'une amine par exemple la triéthylamine, ce qui fournit, sous forme de base libre, les composés désirés de formule (1).

III. Les composés de formule (1) dans laquelle :

- Y représente le groupement - CO -
- l'ensemble formé par R, $R_1$ et Am, plus précisément R, $R_1$, et ($Am_1$) ou ($Am_2$), comportant 1 ou 2 groupements carboxylique ou carboxylate de métal alcalin, c'est-à-dire 1 ou 2 groupements (a) ci-ctessus dans lesquels $R_5$ représente l'hydrogène ou un atome de métal alcalin,
  peuvent être obtenus :

a) Lorsque, dans cette formule (1), un ou deux des groupements R, $R_1$, et ($Am_1$) ou ($Am_2$) comportent un groupement $-CO_2R_5$ dans lequel $R_5$ représente l'hydrogène ou un atome de métal alcalin, l'autre ou les autres groupements étant différents d'un groupement $-CO_2R_5$ dans lequel $R_5$ représente un radical alkyle en $C_1$-$C_{10}$ ou cycloalkyle en $C_3$-$C_6$, en saponifiant en présence d'un agent basique à savoir un hydroxyde de métal alcalin, par exemple l'hydroxyde de sodium, un composé de formule :

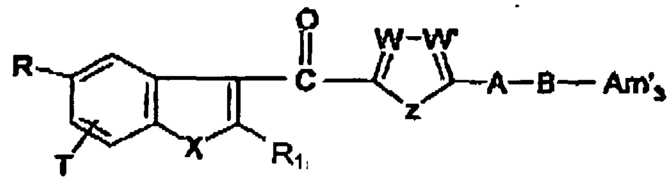

(23)

dans laquelle A, B, R, $R_1$, T, W, W', X et Z ont la même signification que précédemment, $Am'_3$ représente un groupement ($Am_1$) ou ($Am_2$) tels que définis précédemment et R, $R_1$ et ($Am_1$) ou ($Am_2$) sont tels que l'un ou deux d'entre eux comportent un groupement $-CO_2R''_5$ dans lequel $R''_5$ a la même signification que précédemment, l'autre ou les autres groupements étant différents d'un groupement $-CO_2R_5$ dans lequel $R_5$ représente un radical alkyle en $C_1$-$C_{10}$ ou cycloalkyle en $C_3$-$C_6$, ce qui fournit sous forme de base libre les composés de formule (1) dans laquelle un ou deux des groupements R, $R_1$ et ($Am_1$) ou ($Am_2$) comportent un groupement $-CO_2$ $R_5$ dans lequel $R_5$ représente un atome de métal alcalin, composé que l'on traite, si nécessaire, avec un acide fort par exemple l'acide chlorhydrique ce qui fournit, sous forme de base libre, les composés désirés de formée (1) dans laquelle $R_5$ représente l'hydrogène.

Toutefois lorsque dans cette formule (1) R représente le groupement cyano et l'un des groupements $R_1$, ($Am_1$) ou ($Am_2$) comporte un groupement carboxylique, on peut également traiter, au moyen d'oxyde de tributylétain, un composé de formule (1) dans laquelle Y représente -CO-, A, B, T, W, W', X et Z ont la même signification que précédemment, R représente le groupement cyano et $R_1$, ($Am_1$) et ($Am_2$) sont tels que l'un d'eux comporte un groupement $-CO_2R''_5$ dans lequel $R''_5$ a la même signification que précédemment, pour obtenir, sous forme de base libre, les composés de formule (1) désirés.

b) Lorsque, dans cette formule (1), deux des groupements R, $R_1$, ($Am_1$) ou ($Am_2$) comportent l'un un groupement $-CO_2R_5$ dans lequel $R_5$ représente l'hydrogène ou un atome de métal alcalin et l'autre un groupement $-CO_2R_5$ dans lequel $R_5$ représente un groupement alkyle en $C_1$-$C_{10}$ ou cycloalkyle en $C_3$-$C_8$, le troisième groupement étant différent d'un groupement $-CO_2R_5$ dans lequel $R_5$ représente un radical alkyle en $C_1$-$C_{10}$ ou cycloalkyle en $C_3$-$C_8$,

- soit par hydrogénation d'un composé de formule :

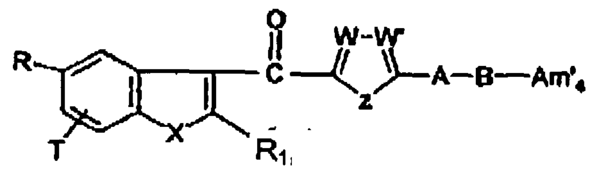

(24)

dans laquelle A, B, R, $R_1$, T, W, W', X et Z ont la même signification que précédemment, $Am'_4$ représente un groupement ($Am_1$) ou ($Am_2$) tels que définis précédemment et R, $R_1$ et ($Am_1$) ou ($Am_2$) sont tels que l'un d'entre eux comporte un groupement $-CO_2R''_5$ dans lequel $R''_5$ a la même signification que précédemment et un autre d'entre eux comporte un groupement benzyloxycarbonyle, le troisième groupement étant différent d'un groupement $-CO_2R_5$ dans lequel $R_5$ représente un radical alkyle en $C_1$-$C_{10}$ ou cycloalkyle en $C_3$-$C_6$ et ce, en présence d'un catalyseur approprié par exemple le charbon palladié ou le noir de platine et de préférence dans un solvant organique
- soit par hydrolyse d'un composé de formule:

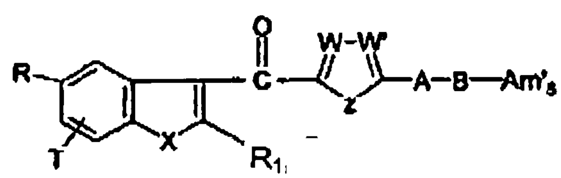

(25)

dans laquelle A, B, R, $R_1$, T, W, W', X et Z ont la même signification que précédemment, $Am'_5$ représente un groupement ($Am_1$) ou ($Am_2$) tels que définis précédemment et R, $R_1$ et ($Am_1$) ou ($Am_2$) sont tels que l'un d'entre eux comporte un groupement $-CO_2R''_5$ dans lequel $R''_5$ a la même signification que précédemment et un autre d'entre eux comporte un groupement t-butoxycarbonyle, le troisième groupement étant différent d'un groupement $-CO_2$ $R_5$ dans lequel $R_5$ représente un radical alkyle en $C_1$-$C_{10}$ ou cycloalkyle en $C_3$-$C_6$ et ce, en présence d'acide trifluoroacétique et de préférence dans un solvant organique par exemple un solvant aprotique tel que le chlorure de méthylène,
ce qui permet d'obtenir les composés souhaités de formule (1) dans laquelle deux des groupements R, $R_1$, et ($Am_1$) ou ($Am_2$) comportent l'un un groupement - $CO_2R_5$ dans lequel $R_5$ représente un groupement alkyle en $C_1$-$C_{10}$ ou cycloalkyle en $C_3$-$C_6$ et l'autre un groupement - $CO_2R_5$ dans lequel $R_5$ représente l'hydrogène, c'est-à-dire un groupement carboxylique, composés que l'on peut traiter, si nécessaire, avec un agent basique approprié par exemple un hydroxyde de métal alcalin, pour obtenir, sous forme de base libre, les composés souhaités de formule (1) dans laquelle deux des groupements R, $R_1$, et ($Am_1$) ou ($Am_2$) comportent l'un un groupement $-CO_2R_5$ dans lequel $R_5$ représente un atome de métal alcalin, l'autre un groupement de formule-$CO_2R_5$ dans lequel $R_5$ représente un radical alkyle en $C_1$-$C_{10}$ ou cycloalkyle en $C_3$-$C_8$, composé que l'on peut traiter eux-mêmes, si nécessaire, avec un acide fort par exemple l'acide chlorhydrique, pour obtenir, sous forme de base libre, les composés souhaités de formule (1) dans laquelle deux des groupements R, $R_1$, et ($Am_1$) ou ($Am_2$) comportent l'un un groupement carboxylique, l'autre un groupement-$CO_2R_5$ dans lequel $R_5$ représente un groupement alkyle en $C_1$-$C_{10}$ ou cycloalkyle en $C_3$-$C_6$.

IV. Les composés de formule (1) dans laquelle Y représente un groupement

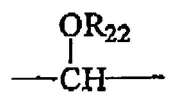

peuvent être obtenus :

a) Lorsque $R_{22}$ représente l'hydrogène, en réduisant au moyen d'un borohydrure de métal alcalin tel que le borohydrure de sodium et de préférence dans un solvant tel qu'un alcool ou un éther, un composé de formule (1) dans laquelle Y représente le groupement - CO -, ce qui fournit sous forme de base libre, les composés désirés de formule (1),
b) Lorsque $R_{22}$ représente un radical alkyle en $C_1$-$C_4$ ou un radical acyle de formule - CO - $R_{23}$, en faisant réagir l'alcool secondaire ainsi formé, c'est-à-dire un composé de formule (1) dans laquelle Y représente le groupement - CHOH - avec :

- soit un alcoolate de métal alcalin, puis avec un halogénure de formule générale :

$R_{23}$-Hal (26)

  dans laquelle Hal et $R_{23}$ ont la même signification que précédemment,
- soit un halogénure d'acyle de formule générale :

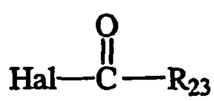

**(27)**

dans laquelle Hal et $R_{23}$ ont la même signification que précédemment, la réaction ayant lieu en présence d'un accepteur d'acide telle que la pyridine,
de manière à obtenir, sous forme de base libre, les composés désirés de formule (1).
Selon la structure du produit de départ, des mélanges de composés peuvent être obtenus lors de la réduction. Ces composés peuvent être séparés de leur mélange selon des techniques classiques par exemple par chromatographie d'élution.
V. Les composés de formule (1) dans laquelle Y représente le groupement $-CH_2$-peuvent être préparés, préférentiellement, en réduisant au moyen d'un borohydrure de métal alcalin tel que le borohydrure de sodium, en présence d'acide trifluoroacétique et de préférence dans un solvant tel qu'un alcool, un éther ou un hydrocarbure halogéné, un composé de formule (1) dans laquelle Y représente le groupement, -CHOH- ce qui fournit, sous forme de base libre, les composés désirés de formule (1).

Généralement, la réduction des composés de formule (1) dans laquelle Y représente un groupement - CO - ou - CHOH - s'effectue à une température de l'ordre de -10˚ à + 10˚C, de préférence à 0˚C.

Les composés de formule (1) obtenus sous forme de base libre selon l'une ou l'autre des méthodes décrites ci-dessus, peuvent ensuite être transformés en sels pharmaceutiquement acceptables par réaction avec acide organique ou inorganique approprié par exemple l'acide oxalique, maléique, fumarique, méthanesulfonique, benzoïque, ascorbique, pamoïque, succinique, hexamique, bisméthylènesalicylique, éthanedisulfonique, acétique, propionique, tartrique, salicylique, citrique, gluconique, lactique, malique, cinnamique, mandélique, citraconique, aspartique, palmitique, stéarique, itaconique, glycolique, p-aminobenzoïque, glutamique, benzènesulfonique, p-toluènesulfonique, théophylline acétique ou avec la lysine ou l'histidine.

Les composés de formule (2) dans laquelle R' représente un groupement cyano ou $-CO_2$ R"$_5$ ainsi que les composés de formule (4) dans laquelle R" représente un groupement cyano ou $-CO_2$ R"$_5$ peuvent être préparés au départ d'un composé de formule générale :

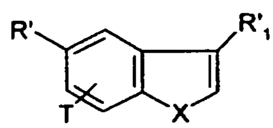

(28)

dans laquelle R' représente un groupement cyano ou $-CO_2$ R"$_5$ et R'$_1$, T et X ont la même signification que précédemment, composé que l'on traite avec un composé de formule générale:

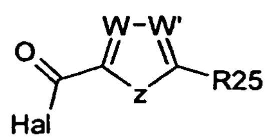

(29)

dans laquelle W, W', Z et Hal ont la même signification que précédemment et $R_{25}$ représente un groupement méthoxy, acétylthio ou nitro ou - A - B - Hal dans lequel A, B et Hal ont la même signification que précédemment et ce, en présence d'un acide de Lewis comme catalyseur par exemple le chlorure ferrique, le chlorure stannique ou le chlorure d'aluminium et dans un solvant tel qu'un hydrocarbure halogéné, de manière à obtenir une cétone de formule générale :

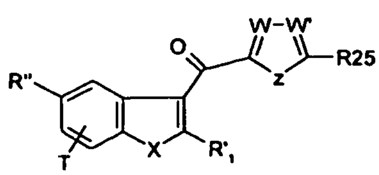

(30)

dans laquelle R' représente un groupement cyano ou $-CO_2$ R"$_5$ et R'$_1$, T, W, W', X, Z et $R_{25}$ ont la même signification que précédemment, ce qui fournit :

- lorsque $R_{25}$ représente un groupement - A - B - Hal, des composés désirés de formule (4),
- lorsque $R_{25}$ représente le groupement méthoxy, des composés que l'on O-déméthyle en présence d'un agent approprié tel que le chlorhydrate de pyridine, le tribromure de bore ou le chlorure d'aluminium, pour obtenir les composés de formule (2) dans laquelle A' représente OH,
- lorsque $R_{25}$ représente le groupement acétylthio, des composés que l'on traite au moyen d'un agent basique approprié tel qu'un hydroxyde de métal alcalin, pour obtenir les composés désirés de formule (2) dans laquelle A représente SH,
- lorsque $R_{25}$ représente le groupement nitro, des composés que l'on réduit par hydrogénation en présence d'un catalyseur approprié tel que la charbon palladié, pour obtenir les composés désirés de formule (2) dans laquelle A' représente $NH_2$.

Alternativement, les composés de formule (2) dans laquelle A' représente le groupement hydroxyle et R' représente un

groupement cyano ou $-CO_2$ $R''_5$ peuvent être obtenus au départ d'un composé de formule (28) que l'on traite avec le phosgène puis avec un composé de formule générale :

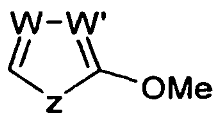
(31)

dans laquelle W, W' et Z ont la même signification que précédemment, la réaction ayant lieu en présence d'un acide de Lewis tel que par exemple le chlorure d'aluminium ou le chlorure stannique pour obtenir les cétones de formule (30) dans laquelle $R_{25}$ représente le groupement méthoxy.

On soumet alors ces cétones de formule (30) ainsi produites, à une O-déméthylation en présence d'un agent approprié tel que le chlorhydrate de pyridine, le tribromure de bore ou le chlorure d'aluminium, pour obtenir finalement les composés désirés.

Les composés de formule (2) dans laquelle A' représente le groupement hydroxyle et R' représente le groupement hydroxyméthyle peuvent être préparés au départ d'un composé de formule (2) dans laquelle R' représente un groupement - $CO_2$ $R''_5$, selon la suite d'étapes ci-après :

a) on traite l'ester de formule (2) en question, à la température de reflux du milieu, au moyen de glycol en présence d'acide p-toluènesulfonique pour former un diéther de formule générale :

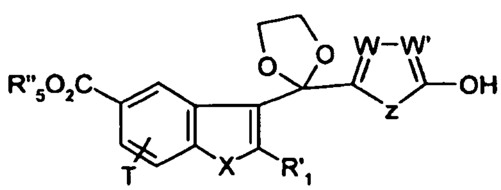
(32)

dans laquelle $R'_1$, $R''_5$, T, X, W, W' et Z ont la même signification que précédemment,
b) on réduit ce composé de formule (32) au moyen d'un hydrure de métal alcalin tel que l'hydrure de lithium aluminium et dans un solvant tel qu'un éther pour obtenir un dialcool de formule générale :

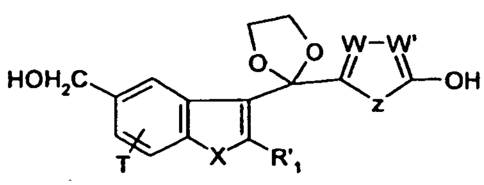
(33)

dans laquelle $R'_1$, T, X, W, W' et Z ont la même signification que précédemment,
c) on déprotège le dialcool ainsi obtenu, au moyen de pyridine p-toluènesulfonate, de préférence à la température de reflux du milieu, ce qui fournit les composés désirés.

Les composés de formule (2) dans laquelle R' représente le groupement (k) peuvent être obtenus en traitant un composé de formule (2) dans laquelle R' représente un groupement - $CO_2$ $R''_5$ avec l'acétamide oxime en présence d'un hydrure de métal alcalin, ce qui fournit les composés désirés.

Les composés de formule (4) dans laquelle R'' représente le groupement cyano, un groupement $-CO_2$ $R''_5$ ou le groupement (k) peuvent être préparés en faisant réagir, de préférence à la température de reflux, un composé de formule (2) dans laquelle R' représente un groupement cyano ou - $CO_2$ $R''_5$ avec un composé dihalogéné de formule générale :

Hal-B-Hal (34)

dans laquelle Hal représente un atome d'halogène, de préférence brome et B a la même signification que précédemment, la réaction ayant lieu en présence d'un agent basique tel qu'un carbonate ou un hydroxyle de métal alcalin, ce qui fournit

les composés désirés.

De même, les composés de formule (4) dans laquelle R" représente un groupement $R_4$ - O - N = CH - peuvent être obtenus :

a) en réduisant un ester de formule (28) dans laquelle R' représente un groupement (a) dans lequel $R_5$ représente un radical alkyle en $C_1$-$C_{10}$ ou cycloalkyle en $C_3$-$C_6$, au moyen d'un agent approprié tel qu'un hydrure par exemple l'hydrure de lithium aluminium, pour former un alcool de formule générale :

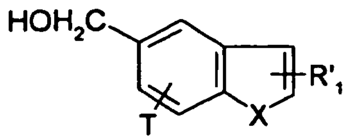
(35)

dans laquelle R'$_1$, T et X ont la même signification que précédemment,

b) en oxydant cet alcool de formule (35) avec le chlorure d'oxalyle de manière à former l'aldéhyde de formule générale :

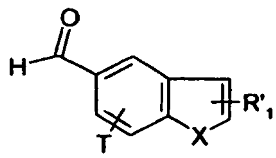
(36)

dans laquelle R'$_1$, T et X ont la même signification que précédemment,

c) en faisant réagir l'aldéhyde ainsi obtenue, avec un composé de formule générale :

$R_4$-O-$NH_2$ (37)

éventuellement sous forme d'un de ses sels, et ce dans un solvant capteur d'acide, par exemple la pyridine, pour former l'oxime de formule générale :

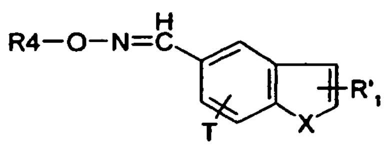
(38)

dans laquelle R'$_1$, T et $R_4$ ont la même signification que précédemment.

On traite alors le composé de formule (38) en question avec soit un composé de formule (29), de manière à obtenir les composés désirés soit d'abord avec le phosgène, ensuite avec un composé de formule (31) et finalement avec un agent approprié pour provoquer une O-déméthylation, par exemple le chlorure d'aluminium, le chlorhydrate de pyridine ou le tribromure de bore, ce qui fournit les composés désirés.

Les composés de formule (6), quant à eux, peuvent être obtenus comme suit :

A- Les composés de formule (6) dans laquelle R" représente un groupement -$CO_2$ R"$_5$ situé en position 5 et R'$_1$, situé en position 2, représente un groupement alkyle en $C_1$-$C_6$, cycloalkyle en $C_3$-$C_6$, phényle ou benzyle, peuvent être préparés selon la suite d'étapes ci-après :

a) on traite d'abord un benzoate de formule générale :

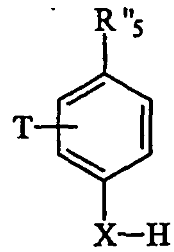

(39)

dans laquelle R"$_5$, T et X ont la même signification que précédemment, avec l'acide méthanesulfonique, en présence de pentoxyde de phosphore et d'hexaméthylènetétramine, pour donner un dérivé formyle de formule générale :

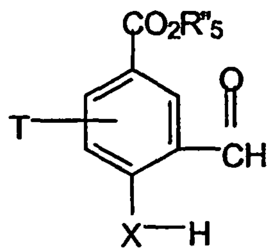

(40)

dans laquelle R"$_5$, T et X ont la même signification que précédemment,
b) on fait ensuite réagir ce composé de formule (40) avec un ester de formule générale:

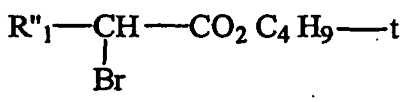

(41)

dans laquelle R", représente un groupement alkyle en $C_1$-$C_6$, cycloalkyle en $C_3$-$C_6$, phényle ou benzyle, ce qui fournit les composés de formule générale :

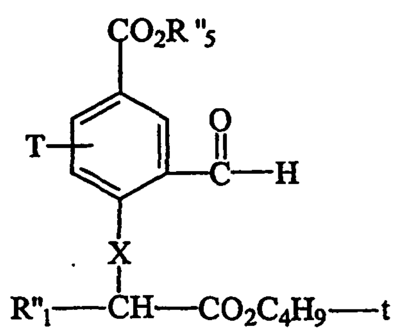

(42)

dans laquelle R"$_1$, R"$_5$, T et X ont la même signification que précédemment,
c) on traite cet ester de formule (42) avec l'acide formique ou l'acide trifluoracétique, ce qui fournit les acides de formule générale :

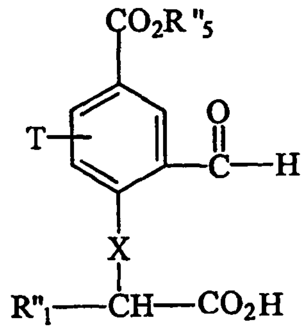

(43)

dans laquelle R"$_1$, R"$_5$, T et X ont la même signification que précédemment,

d) on cyclise ce composé en présence de chlorure de benzènesuflonyle ou de p-toluènsulfonyle et d'un accepteur d'acide tel que la triéthylamine, ce qui donne les composés souhaités de formule (6).

**B-** Les composés de formule (6) dans laquelle R" représente le groupement cyano situé en position 5 et R'$_1$, situé en position 2, représente un groupement alkyle en $C_1$-$C_6$, cycloalkyle en $C_3$-$C_6$, phényle ou benzyle, peuvent être préparés comme suit:

a) on traite d'abord un dérivé formyle de formule générale :

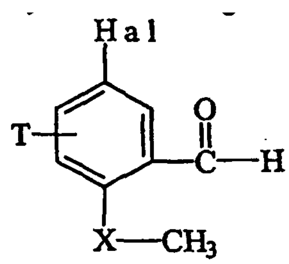

(44)

dans laquelle Hal, T et X ont la même signification que précédemment, avec le cyanure de zinc en présence d'un catalyseur approprié, par exemple un dérivé de palladium tel que le tétrakis-(triphénylphosphine)palladium, ce qui fournit les composés de formule générale :

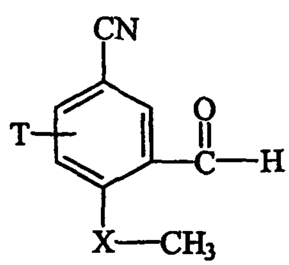

(45)

dans laquelle T et X ont la même signification que précédemment,

b) on déméthyle ensuite ce composé de formule (45) avec le chlorure de lithium, ce qui fournit les composés de formule générale :

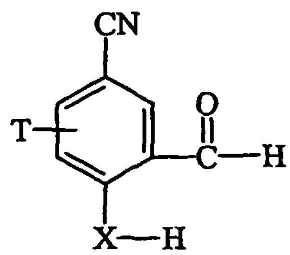

(46)

dans laquelle T et X ont a la même signification que précédemment,
c) on traite alors ce composé de formule (46) avec un ester de formule générale :

(47)

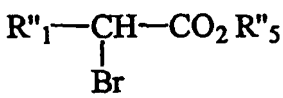

dans laquelle R"$_1$ et R"$_5$ ont la même signification que précédemment et ce, en présence d'un agent basique tel qu'un carbonate de métal alcalin, ce qui donne les composés de formule générale :

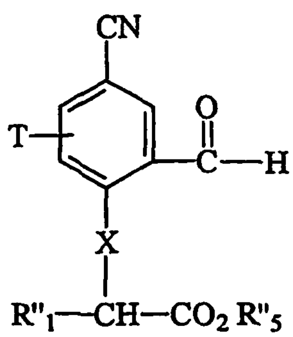

(48)

dans laquelle R"$_1$, R"$_5$, T et X ont la même signification que précédemment,
d) et e) on saponifie cet ester de formule (48) en présence d'un agent basique tel qu'un hydroxyde de métal alcalin et l'on cyclise l'acide ainsi obtenu en présence de chlorure de benzènesulfonyle ou de p-toluènesulfonyle et d'un accepteur d'acide tels que la triéthylamine, ce qui fournit les composés souhaités.

C- Les composés de formule (6) dans laquelle R" représente un groupement cyano ou - $CO_2$ R"$_5$ situé en position 5 et R'$_1$, situé en position 2, représente un groupement (m) dans lequel $R_{11}$ représente un groupement - $CO_2$ R"$_5$ peuvent être obtenus selon la suite d'étapes suivantes :

a) soit, on traite un dérivé cyano de formule générale :

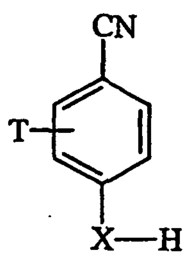

(49)

dans laquelle T et X ont la même signification que précédemment, avec l'iode en présence d'ammoniaque,

pour former un dérivé iodo de formule générale :

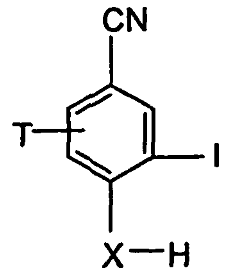

(50)

dans laquelle T et X ont la même signification que précédemment,
soit, on traite un composé de formule générale :

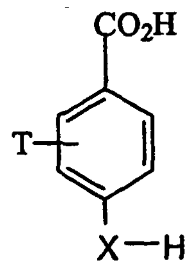

(51)

dans laquelle T et X ont la même signification que précédemment, d'abord avec un iodure de métal alcalin et un agent oxydant tel qu'un hypochlorite de métal alcalin par exemple l'hypochlorite de sodium, ensuite avec un agent halogénant tel que le chlorure de thionyle et finalement avec un alcool de formule générale :

$R''_5$-OH (52)

dans laquelle $R''_5$ a la même signification que précédemment, ce qui fournit un dérivé iodo de formule générale :

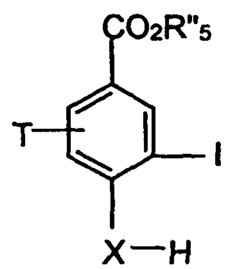

(53)

dans laquelle $R''_5$, T et X ont la même signification que précédemment,
b) on fait réagir le dérivé iodé de formule (50) ou (53) avec un ester acétylénique de formule générale :

$HC\equiv C-(CH_2)_p-CO_2\ R'_5$ (54)

dans laquelle $R''_5$ et p ont la même signification que précédemment et ce, en présence d'un catalyseur approprié tel qu'un dérivé de palladium par exemple le dichloro bis-(triphénylphosphine)palladium et d'iodure cuivreux et en présence de tétraméthylguanidine, ce qui fournit les composés désirés de formule (6).

D- Alternativement, les composés de formule (6) dans laquelle R" représente un groupement cyano ou $-CO_2\ R''_5$ situé en position 5 et $R'_1$, situé en position 2, représente un groupement alkyle en $C_3$-$C_6$, peuvent être préparés en faisant réagir un dérivé iodé de formule (53) avec un dérivé acétylénique de formule générale :

$$HC\equiv C-(CH_2)_{p-1}-CH_3 \quad (55)$$

dans laquelle p a la même signification que précédemment et ce, en présence d'un catalyseur approprié tel qu'un dérivé de palladium par exemple le tétrakis (triphénylphosphine)palladium et d'iodure cuivreux, ce qui fournit les composés désirés de formule (6).

**E**- Les composés de formule (6) dans laquelle R" représente un groupement cyano ou $-CO_2$ $R''_5$ situé en position 6 et $R'_1$, situé en position 2, a la même signification que précédemment peuvent être obtenus comme suit :

a) on fait réagir un composé de formule générale :

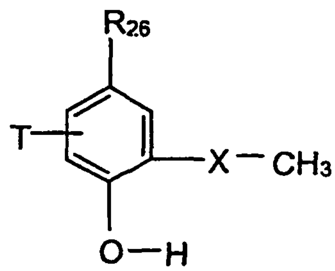

(56)

dans laquelle $R_{26}$, T et X ont la même signification que précédemment, avec l'anhydride de l'acide trifluorométhanesulfonique en présence de pyridine, pour obtenir un composé de formule générale :

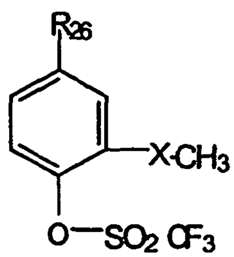

(57)

dans laquelle $R_{26}$, T et X ont la même signification que précédemment,

b) on fait réagir le composé ainsi formé avec un dérivé acétylénique de formule générale :

$HC\equiv C-R_1'$ (58)

dans laquelle $R'_1$ a la même signification que précédement et ce, en présence d'un catalyseur approprié par exemple un dérivé de palladium tel que le dichloro bis-(triphénylphosphine)palladium et d'un accepteur d'acide tel que la triéthylamine pour former les composés de formule générale :

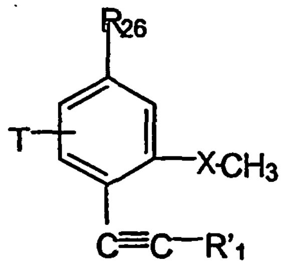

(59)

dans laquelle $R'_1$, $R_{26}$, T et X ont la même signification que précédemment,

c) on cyclise alors ce composé de formule (59) en présence de tribromure de bore à une température inférieure à -50°C, ce qui fournit les composés hétérocycliques de formule générale :

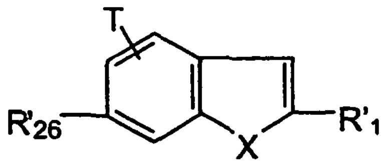

(60)

dans laquelle R'$_1$, T et X ont la même signification que précédemment et R'$_{26}$ représente le groupement cyano, ce qui fournit des composés désirés de formule (6), ou R'$_{26}$ représente un groupement carboxylique, ce qui fournit un acide,

d) on estérifie cet acide avec un alcool de formule (52), ce qui fournit des composés désirés de formule (6).

**F**- Les composés de formule (6) dans laquelle R" représente un groupement cyano ou $-CO_2$ R"$_5$ situé en position 4 et R'$_1$, situé en position 2, a la même signification que précédemment peuvent être obtenus comme suit :

a) on fait réagir un composé de formule générale :

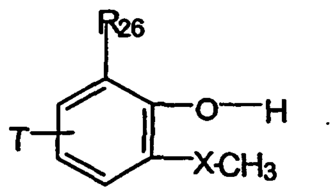

(61)

dans laquelle $R_{26}$, T et X ont la même signification que précédemment, avec l'anhydride trifluorométhanesulfonique en présence de pyridine, pour obtenir un composé de formule générale:

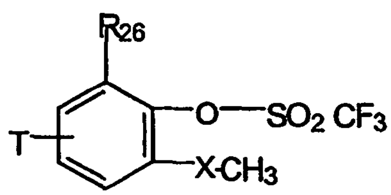

(62)

dans laquelle $R_{26}$, T et X ont la même signification que précédemment,

b) on fait réagir le composé ainsi formé avec un dérivé acétylénique de formule (58) et ce, en présence d'un catalyseur approprié tel qu'un dérivé de palladium par exemple le dichloro bis-(triphénylphosphine)palladium et d'un accepteur d'acide tel que la triéthylamine pour former les composés de formule générale :

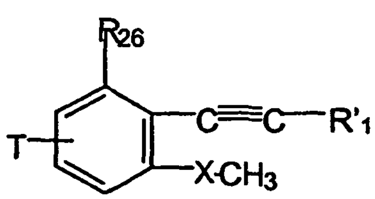

(63)

dans laquelle R'$_1$, $R_{26}$, T et X ont la même signification que précédemment,

c) on cyclise alors ce composé de formule (63) en présence de tribromure de bore, ce qui fournit les composés hétérocycliques de formule générale :

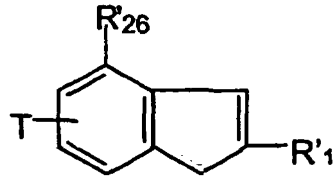

(64)

dans laquelle R'$_1$, T et X ont la même signification que précédemment et R'$_{26}$ représente le groupement cyano, ce qui fournit les composés désirés de formule (6)
ou R'$_{26}$ représente un groupement carboxylique, ce qui fournit un acide,
d) on estérifie cet acide avec un alcool de formule (52), ce qui fournit des composés désirés de formule (6).

**G**- Les composés de formule (6) dans laquelle R" représente un groupement cyano ou -$CO_2R''_5$ situé en position 7 et R'$_1$, situé en position 2, a la même signification que précédemment peuvent être obtenus comme suit :

a) on traite un alcool de formule générale :

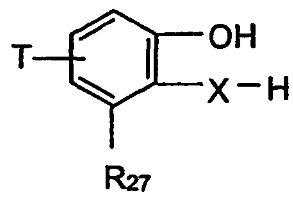

(65)

dans laquelle $R_{27}$ représente un groupement cyano ou formyle et T et X ont la même signification que précédemment et ce, avec l'iodure de méthyle en présence d'un hydrure de métal alcalin pour donner un composé de formule générale :

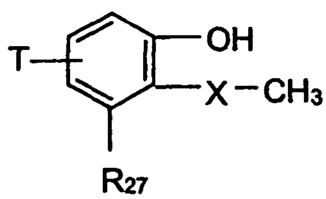

(66)

dans laquelle $R_{27}$, T et X ont la même signification que précédemment,
b) on fait réagir le composé ainsi formé avec l'anhydride trifluorométhanesulfonique pour former un composé de formule générale :

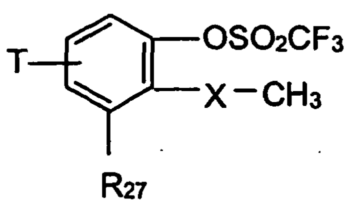

(67)

dans laquelle $R_{27}$, T et X ont la même signification que précédemment,
c) on traite le composé ainsi formé avec un composé de formule (58) en présence d'un catalyseur approprié tel qu'un dérivé de palladium, par exemple le dichloro bis-(triphénylphosphine)palladium, ce qui produit un composé de formule générale :

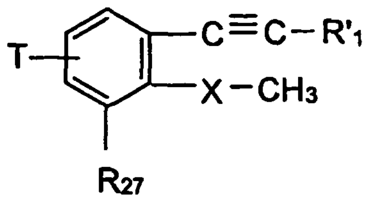

(68)

dans laquelle R'$_1$, $R_{27}$, T et X ont la même signification que précédemment,
d) on fait ensuite réagir le composé de formule (68) ainsi formé :

- lorsque $R_{27}$ représente le groupement cyano, avec le chlorure de lithium pour former les composés désirés de formule (6) dans laquelle R" représente le groupement cyano,
- lorsque $R_{27}$ représente le groupement formyle, avec un cyanure de métal alcalin en présence d'oxyde manganeux et d'acide acétique pour donner un composé de formule générale :

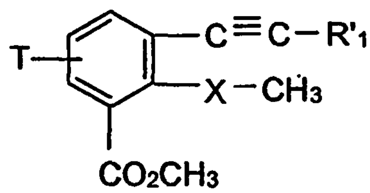

(69)

dans laquelle $R_{27}$, T et X ont la même signification que précédemment, que l'on cyclise avec le chlorure de lithium pour donner un mélange d'ester et d'acide de formule générale :

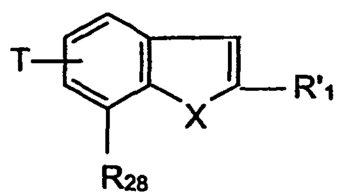

(70)

dans laquelle $R_{28}$ représente le groupement méthoxycarbonyle ou carboxylique et R'$_1$, T et X ont la même signification que précédemment, mélange que l'on traite avec le méthanol en présence d'un acide fort tel que l'acide sulfurique, ce qui fournit des composés désirés de formule (6) dans laquelle R" représente le groupement méthoxycarbonyle.

Les autres composés de formule (6), c'est-à-dire les composés de formule (6) dans laquelle R" situé en position 7 représente un groupement
$-CO_2R''_5$, à l'exception du groupement méthoxycarbonyle, peuvent être obtenus en saponifiant un ester de formule (6) dans laquelle R" situé en position 7 représente le groupement méthoxycarbonyle et ce, en présence d'un agent basique tel qu'un hydroxyde de métal alcalin pour donner un sel que l'on acidifie avec un acide fort tel que l'acide chlorhydrique pour donner un dérivé de 7-carboxy-benzofurane que l'on estérifie avec un alcool de formule générale :

$R'_a$-OH (71)

dans laquelle $R'_a$ représente un radical alkyle en $C_2$-$C_{10}$ ou cycloalkyle en $C_3$-$C_6$, ce qui fournit des composés désirés de formule (6).

H- Les composés de formule (6) dans laquelle R" représente le groupement (k) peuvent être obtenus par cyclisation d'un composé de formule (6) dans laquelle R" représente un groupement $-CO_2\ R''_5$ au moyen d'acétamide oxime et ce, en présence d'un hydrure de métal alcalin tel que l'hydrure de sodium, ce qui fournit les composés désirés.

I- Les composés de formule (6) dans laquelle R" représente un groupement $R_4$-O-N = CH- correspondent en fait aux composés de formule (38) dont la préparation a été décrite précédemment.

Les autres composés de départ ou intermédiaires intervenant dans les différents procédés décrits précédemment sont pour la plupart des composés connus ou pouvant être préparés par des méthodes connues.

Par exemple, les amines de formule (3) ou de formule (5) sont, pour certaines d'entre elles, connues et décrites notamment dans les brevets US 4831054 ou EP 471609 ou pouvant être préparés par les méthodes y décrites.

Par exemple, on peut préparer la 1-(2-chloroéthyl)-4-dicyclohexylméthylpipérazine en faisant réagir la N-dicyclohexylméthylpipérazine avec l'oxyde d'éthylène pour former la 1-(2-hydroxyéthyl)-4-dicyclohexylméthylpipérazine que l'on traite ensuite avec un agent de chloration tel que le chlorure de thionyle pour obtenir le composé souhaité.

De même, on peut obtenir la 1-(2-chloroéthyl)-3,5-diéthyl-pipéridine par un procédé analogue impliquant la formation de la 1-(2-hydroxyéthyl)-3,5-diéthylpipéridine à partir d'oxyde d'éthylène et de 2,5-diéthylpipéridine puis sa transformation par un agent halogénant tel que le chlorure de thionyle pour obtenir le composé souhaité.

On connaît déjà des dérivés de benzofuranne ou de benzothiophène comportant une chaîne monoalkylamino- ou dialkylamino-alkoxy-benzoyle et substitué sur l'homocycle par un groupement amino lui-même substitué ou non. De tels composés, qui ont été décrits dans le brevet EP 0471609, ont révélé d'intéressantes propriétés antiarythmiques se traduisant notamment par des effets pharmacologiques des classes 1,2,3 et 4 de Vaughan-Williams.

Toutefois, ces dérivés de benzofuranne et de benzothiophène présentent une faible soubitité en milieu aqueux ainsi qu'une faible disposnibilité par voie orale.

Or, on a maintenant découvert, dans le cadre de l'invention, que des dérivés de benzofurane ou benzothiophène comportant une chaîne aminoalkoxy-benzoyle ainsi que d'autres groupements fixés sur l'hétérocycle par l'intermédiaire d'un atome de carbone, présentent un profil pharmacologoique analogue à celui des composés antérieurs tout en offrant une meilleure stabilité métabolique, une solubilité plus importante et une biodisponibilité supérieure par voie orale.

Les résultats de tests pharmacologiques effectués en vue de déterminer les propriétés des composés de l'invention sur le système cardiovasculaire sont répertoriés ci-dessous.

I. Activité antiarythmique

Le but de ce test est de déterminer la capacité des composés de l'invention à assurer une protection contre les arythmies provoquées par reperfusion. A cet effet, on a utilisé la méthode rapportée par MANNING A.S. et coll. dans Circ. Res. 1984, 55 : 545-548 modifiée comme suit :

On anesthésie d'abord des rats, répartis en lots, avec du pentobarbital sodique (60mg/kg par voie intrapéritonéale) puis on les intube et les maintient sous respiration assistée.

On leur place ensuite une canule pour administration intraveineuse dans la veine jugulaire droite, on administre une dose intraveineuse du composé à étudier et 5 minutes plus tard, on place une boucle de ligature autour de l'artère coronaire descendante antérieure gauche et à proximité immédiate de son origine. On provoque alors l'occlusion de cette artère pendant 5 minutes par traction sur les extrémités de la ligature de manière à induire une reperfusion par relâchement de la tension.

On évalue alors les arythmies induites par cette reperfusion.

Un test analogue a été pratiqué par voie orale. Dans ce cas, le composé à étudier est administré 120 minutes avant la ligature de l'artère coronaire descendante antérieure gauche.

Les résultats de ces tests ont montré que les composés de l'invention protègent les animaux traités de manière significative allant jusqu'à 100% à des doses comprises entre 0,3 et 10mg/kg par voie intraveineuse et 10 à 90mg/kg par voie orale.

II. Propriétés antiadrénergiques

Le but de ce test est de déterminer la capacité des composés de l'invention à réduire l'augmentation de la pression sanguine induite par la phényléphrine (effet anti-$\alpha$) et l'accélération de la fréquence cardiaque induite par l'isoprénaline (effet anti-$\beta$) chez le chien préalablement anesthésié au pentobarbital et chloralose.

On détermine d'abord pour chaque chien la dose de phényléphrine (5 ou 10$\mu$g/kg) qui provoque une augmentation de la pression artérielle comprise entre. 25 et 40mm Hg et la dose d'isoprénaline (0,9 ou 1$\mu$g/kg) qui devra entraîner une augmentation de la fréquence cardiaque comprise entre 60 et 120 battements/minute.

On injecte alternativement toutes les 10 minutes les doses de phényléphrine et d'isoprénaline ainsi déterminées et après obtention de 2 réponses de référence successives, on administre une dose du composé à étudier par voie intraveineuse

- *Effet anti-$\alpha$*

On enregistre le pourcentage de réduction, par le composé de l'invention, de l'hypertension provoquée comparativement à l'hypertension de référence obtenue avant injection de ce composé (environ 100mm Hg).

- *Effet anti-β*

On enregistre le pourcentage de réduction, par le composé à étudier, de l'accélération provoquée de la fréquence cardiaque.

Les résultats de ces tests montrent qu'à des doses variant de 1 à 10 mg/kg, les composés de l'invention présentent des effets anti-α et/ou anti-β se traduisant par des réductions de l'hypertension provoquée et/ou de l'augmentation provoquée de la fréquence cardiaque, allant de 50% à presque 100%.

III. Fibrillation auriculaire

Le but de ce test est d'évaluer l'efficacité des composés de l'invention vis-à-vis de la fibrillation auriculaire induite par stimulation permanente du nerf vague chez le chien anesthésié selon la méthode décrite dans Circulation 1993 ;88 : 1030-1044.

Les composés à étudier sont administrés aux doses cumulées de 3 et 10 mg/kg en perfusions intraveineuses lentes de 10 minutes pendant un épisode de fibrillation auriculaire soutenu.

A la dose de 10 mg/kg les composés de l'invention convertissent généralement 100% des fibrillations auriculaires en rythme sinusal et en préviennent la ré-induction dans 50 à 100% des cas. A cette dose, on observe des augmentations significatives de la période cardiaque ainsi que des périodes réfractaires effectives auriculaires pour différentes valeurs basales de la période cardiaque.

IV. Effets inhibiteurs du système neuro-hormonal

Le but de ce test est de rechercher les effets inhibiteurs des composés de l'invention vis-à-vis des effets vasoconstricteurs induits par différents peptides tels que la noradrénaline (NA), l'angiotensine II (A-II), l'arginine vasopressine (AVP), le neuropeptide Y (NPY) et l'endothéline (ET) et également vis-à-vis des effets tachycardes induits par l'isoprénaline (Iso) chez le rat vigile.

Chez des rats mâles Sprague Dawley d'environ 300g, on implante, 24 heures avant le test, un cathéter artériel (artère carotide droite) pour la mesure de la pression artérielle et un cathéter veineux (veine jugulaire droite) pour l'injection des produits à étudier. Le lendemain, on place les rats dans des boîtes cylindriques et on relie le cathéter artériel à un capteur de pression par l'intermédiaire d'un joint tournant sur balancier. Ce capteur de pression est lui-même relié à un polygraphe pour enregistrement de la pression artérielle.

On recherche alors l'action des composés de l'invention, par voie intraveineuse, vis-à-vis des effets vasoconstricteurs induits par la NA (1 μg/kg), l'A-II (100 μg/kd) et l'AVP (40 μg/kg) aux doses respectives de soit 3, 10 et 30 mg/kg soit 1,3 à 10 mg/kg et uniquement à la dose de 10 mg/kg vis-à-vis des effets vasoconstricteurs induits par le NPY (6 μg/kg) et l'ET (0,5 μg/kg) ou des effets tachycardies induits par l'Iso (1 μg/kg).

On solubilise d'abord les différents agonistes peptiques dans du sérum physiologique à 0,9% et le composé à étudier dans un solvant approprié. On injecte ensuite ces peptides en bolus sous un volume de 0,05 ml/kg et ce, 30 et 10 minutes avant l'administration intraveineuse de 0,1 ml/kg d'une solution du composé à étudier ou de solvant. On répète ensuite ces injections de peptide 10, 30, 60 et 120 minutes après l'administration du composé à étudier. En fonction de la durée d'action du composé à tester, on peut éventuellement prolonger ces injections toutes les 30 minutes sans jamais dépasser 5 heures au total.

On évalue alors les variations de la pression artérielle après administration d'un peptide donné en mesurant, à différents temps, la différence entre l'effet maximal induit par l'agoniste peptide et la valeur basale de la pression artérielle.

Les résultats obtenus montrent que la NA, l'A-II, l'AVP, le NPY et l'ET induisent des augmentations respectives de la pression artérielle de 45 ±3, 40±3, 30±2 et 34±4 mmHg et l'Iso une augmentation de la fréquence cardiaque de 209±7 battements par minute.

En outre, on observe que les composés de l'invention antagonisent d'une manière dose-dépendante les effets vasoconstricteurs induits par la NA, l'A-II et l'AVP. Ils antagonisent également les effets induits par le NPY et par l'ET et l'augmentation de la fréquence cardiaque induite par l'Iso. Aux doses les plus élevées, l'inhibition maximale obtenue après 15 minutes varie entre 40 et 80% et la durée d'action est au moins supérieure ou égale à 30 minutes.

**V**. Toxicité

La toxicité des composés de l'invention s'est révélée compatible avec leur utilisation en thérapeutique.

Les compositions pharmaceutiques selon l'invention peuvent être présentées sous toute forme convenant à

l'administration en thérapie humaine ou vétérinaire. Par exemple les compositions pharmaceutiques de la présente invention peuvent être formulées pour l'administration orale, sublinguale, sous-cutanée, intramusculaire, intraveineuse, transdermique ou rectale. Pour ce qui concerne l'unité d'administration, celle-ci peut prendre la forme, par exemple, d'un comprimé, d'une dragée, d'une capsule, d'une gélule, d'une poudre, d'une suspension, d'un sirop ou encore de granules pour l'administration orale, d'un suppositoire pour l'administration rectale ou d'une solution ou suspension pour l'administration parentérale.

Les compositions pharmaceutiques de l'invention pourront comprendre, par unité d'administration, par exemple de 50 à 500mg en poids d'ingrédient actif pour l'administration orale, de 50 à 200mg d'ingrédient actif pour l'administration rectale et de 50 à 150mg d'ingrédient actif pour l'administration parentérale.

Suivant la voie d'administration choisie, les compositions pharmaceutiques ou vétérinaires de l'invention seront préparées en associant au moins un des composés de formule (1) ou un sel pharmaceutiquement acceptable de ce composé avec un excipient approprié, ce dernier pouvant être constitué par exemple d'au moins un ingrédient sélectionné parmi les substances suivantes : lactose, amidons, talc, stéarate de magnésium, polyvinylpyrrolidone, acide alginique, silice colloïdale, eau distillée, alcool benzylique ou agents édulcorants.

Lorsqu'il s'agit de comprimés, ceux-ci peuvent être traités de telle sorte qu'ils présentent une activité prolongée ou retardée et qu'ils libèrent d'une façon continue une quantité prédéterminée de principe actif.

Les Exemples illustrent la préparation des composés et compositions de l'invention :

## EXEMPLE 1

### 2-Butyl-3-[4-[3-dibutylaminopropoxy]benzoyl]-1-benzofurane-5-carboxylate de méthyle

**A.** 3-Formyl-4-hydroxy-benzoate de méthyle

Dans un ballon tricol, on place 2g d'anhydride phosphorique et 40ml d'acide méthanesulfonique. On chauffe à environ 85°C, on cesse alors le chauffage et on ajoute par fractions, à une température de 85°C à 90°C, un mélange intime de 7,6g (0,05 mole) de 4-hydroxy-benzoate de méthyle et de 10,22g (0,073 mole) d'hexaméthylènetétramine.

Dès l'addition terminée, on chauffe à 85°/90°C pendant 2 heures, on laisse refroidir à 70°C puis on ajoute 60ml d'eau. On laisse revenir progressivement à la température ambiante puis on extrait avec de l'acétate d'éthyle. On lave ensuite avec de l'eau, une solution aqueuse de bicarbonate sodique, de l'eau, une solution de sulfate acide de potassium à nouveau avec de l'eau jusqu'à neutralité et enfin avec une solution saturée de chlorure de sodium.

De cette manière, on obtient 6,73g de composé désiré sous forme brute.
Rendement : 75%
P. F. : 80-81 °C

**B.** 2-Bromo-hexanoate de tertiobutyle

On dissout 25,16g (0,129 mole) d'acide 2-bromo-hexanoïque dans 200ml de benzène contenant 1ml de N,N-diméthylformamide. On refroidit au moyen d'un mélange eau/glace et, on ajoute, goutte à goutte, à la température d'environ 7°C, 32,8g (2 équivalents) de chlorure d'oxalyle dans 50ml de benzène. On agite durant 1 heure à froid puis on laisse revenir à la température ambiante. On évapore le solvant. On dissout alors le chlorure d'acide obtenu dans 500ml de dichlorométhane puis on l'ajoute, à température inférieure à 10°C, à un mélange de 120g (12,5 équivalents) de tertiobutanol et de 24,9g (1,75 équivalent) de triéthylamine.

On laisse revenir à température ambiante et on lave avec 500ml d'eau puis avec 100ml d'acide chlorhydrique à 3%. On évapore le dichlorométhane puis on reprend le résidu avec de l'éther diéthylique et on lave avec de l'eau, une solution aqueuse de bicarbonate sodique, de l'eau, une solution de sulfate de potassium de l'eau et enfin avec une solution saturée de chlorure de sodium. On distille alors sous pression réduite.

De cette manière, on obtient 25g de composé désiré.
Rendement : 77%
P.E. : 104-107°C (20mm Hg)

**C.** 2-[(2-Formyl-4-méthoxycarbonyl)-phénoxyl-hexanoate de tertiobutyle

On dissout 6,73g (37mmoles) de 3-formyl-4-hydroxy-benzoate de méthyle et 10,32g (1,1 équivalent) de 2-bromo-hexanoate de tertiobutyle dans 100ml de N,N-diméthylformamide.

On ajoute alors 6,45g (1,25 équivalent) de carbonate de potassium et on chauffe au bain-marie (environ à 80°C) durant 3 heures. On évapore alors le N,N-diméthylformamide. On reprend avec de l'acétate d'éthyle et on lave avec une solution à 3% de sulfate acide de potassium, avec de l'eau et avec une solution saturée de chlorure de sodium.

On purifie par chromatographie sur silice (éluant : dichlorométhane/acétate d'éthyle 100/2).
De cette manière, on obtient 11,4g de composé désiré.
Rendement : 88%

**D**. Acide 2-[(2-formyl-4-méthoxycarbonyl)-phénoxy]-hexanoïque

On agite à température ambiante durant 24 heures, un mélange de 17,16g de 2-[(2-formyl-4-méthoxycarbonyl)-phénoxy]-hexanoate de tertiobutyle et de 100ml d'acide formique. On dilue avec de l'eau et on extrait avec de l'acétate d'éthyle. On lave avec de l'eau jusqu'à neutralité puis avec une solution saturée de chlorure de sodium.
De cette manière, on obtient 14,7g de composé désiré.
Rendement: 100%

**E**. 2-Butyl-1-benzofurane-5-carboxylate de méthyle

A un mélange de 141,6ml de triéthylamine dans 250ml de toluène, on ajoute 52,5ml de chlorure de benzènesulfonyle dissous dans 250ml de toluène. On chauffe à 80°C puis on ajoute, goutte à goutte, à une température inférieure ou égale à 90°C, 85,3g d'acide 2-[(2-formyl-4-méthoxycarbonyl)-phénoxy]-hexanoïque dissous dans 500ml de toluène.
Dès l'addition terminée, on poursuit le chauffage durant 0,5 heure, on laisse revenir à température ambiante et on dilue avec de l'acétate d'éthyle. On lave alors avec de l'eau, une solution de sulfate acide de potassium, de l'eau, une solution de bicarbonate sodique, de l'eau et une solution saturée de chlorure de sodium. On distille ensuite sous pression réduite.
De cette manière, on obtient 28,4g de composé désiré.
Rendement : 39% par rapport au 4-hydroxy-benzoate de méthyle
P.E. : 126-132°C (0,03mbar)

**F.** 2-Butyl-3-(4-méthoxy-benzoyl)-1-benzofurane-5-carboxylate de méthyle

Sous argon, on dissout 45,6g (0,28 mole) de chlorure ferrique dans 270ml de dichloréthane puis on ajoute, à environ 10°C, 32,51g (0,14 mole) de 2-butyl-1-benzofurane-5-carboxylate de méthyle dissous dans 180ml de dichloréthane. On additionne ensuite, entre 10 et 15°C, 48,2g (0,28mole) de chlorure d'anisoyle dissous dans 180ml de dichloréthane. On laisse revenir à température ambiante puis on agite durant 5 heures à cette température. On verse le mélange réactionnel sur un mélange glace/eau puis on filtre le précipité.
On décante le filtrat, on extrait la phase aqueuse avec du dichlorométhane. On lave les phases organiques avec une solution de bicarbonate sodique, de l'eau et une solution saturée de chlorure de sodium puis on purifie par cristallisation.
De cette manière, on obtient 48,84g de composé désiré.
Rendement : 95%
P. F. : 75-78°C

**G.** 2-Buyl-3-(4-hydroxy-benzoyl)-1-benzofurane-5-carboxylate de méthyle

On dissout 48,84g de 2-butyl-3-(4-méthoxy-benzoyl)-1-benzofurane-5-carboxylate de méthyle et 55g de chlorure d'aluminium dans 550ml de toluène. On chauffe à 60°C, au bain-marie, durant 2 heures puis on décante le toluène.
On dissout le résidu dans le tétrahydrofurane, on ajoute de la glace et on agite le mélange durant 2 heures. On décante et extrait la phase aqueuse avec de l'acétate d'éthyle. On réunit les 3 phases organiques et on lave avec de l'eau puis avec une solution aqueuse saturée de chlorure de sodium. On purifie alors par cristallisation dans l'éther diisopropylique.
De cette manière, on obtient 26,45g de composé désiré.
Rendement : 56%
P.F. : 152-153°C

**H**. 2-Butyl-3-[4-(3-dibutylamino)propoxy]benzoyl]-1-benzofurane-5-carboxylate de méthyle

On dissout, dans 35ml de méthyléthylcétone, 2,14g (6mmoles) de 2-butyl-3-(4-hydroxy-benzoyl)-1-benzofurane-5-carboxylate de méthyle, 1,25g (6mmoles) de 3-chloro-1-(dibutylamino)propane et 1g de carbonate de potassium. On porte au reflux durant 22 heures, on dilue dans l'eau et on extrait avec de l'acétate d'éthyle. On lave avec une solution saturée de chlorure de sodium et on purifie par chromatographie sur silice (éluant : hexane/acétate d'éthyle 5/5).
De cette manière, on obtient 2,95g de composé désiré sous forme de base libre.

Rendement : 94%
Spectre de résonance magnétique nucléaire (RMN) : conforme

**EXEMPLE 2**

**Oxalate de 2-butyl-3-[4-[3-dibutylamino)propoxy]benzoyl]-1-benzofurane-5-carboxylate de méthyle**

On dissout, dans du méthanol, 1,957g de 2-butyl-3-[4-[3-(dibutylamino)propoxy]benzoyl]-1-benzofurane-5-carboxylate de méthyle et 0,338g (1 équivalent) d'acide oxalique.
On évapore, reprend le résidu dans l'éther diéthylique, triture puis évapore. On sèche ensuite sous vide.
De cette manière, on obtient 2,13g de composé désiré.
Rendement : 93%
P.F. : 82-84°C
Spectre RMN : conforme

**EXEMPLE 4**

**Oxalate de 2-butyl-3-[4-[3-(dibutylamino)propoxy]benxoyl]-1-benzofurane-5-carboxylate de cyclohexyle**

On porte à reflux durant 1 heure, un mélange de 3,25g d'acide 2-butyl-3-[4-[3-(dibutylamino)propoxyl]benzoyl]-1-benzofurane-5-carboxylique et de 3ml de chlorure de thionyle dans 60ml de chloroforme. On concentre à sec puis on réalise des reprises dans l'éther diéthylique et des concentrations successives, ce qui fournit un chlorure d'acyle que l'on utilise sous forme brute par la suite. On ajoute alors 50ml de cyclohexanol et on chauffe à 100°C pendant 2 heures. On refroidit puis distille sous vide pour éliminer le cyclohexanol. On chromatographie sur silice la fraction non distillée (éluant : chlorométhane/méthanol : 98/2) et on reprend la fraction principale dans une solution aqueuse diluée de carbonate de sodium. On extrait à l'éther diéthylique, on sèche et on concentre, ce qui permet d'obtenir le produit désiré sous forme de base libre que l'on salifie par ajout d'acide oxalique dans l'éthanol absolu.
De cette manière, on recueille 2,203g de composé désiré.
Rendement : 50,6%
P.F. : 96°C
Spectre RMN : conforme

**EXEMPLE 5**

**Oxalate de 2-butyl-3-[4-[3-(dibutylamino)propoxy]benzoyl]-N-hydroxy-1-benzofurane-5-carboxamide**

**A.** 2-Butyl-3-[4-[3-(dibutylamino)propoxy]benzoyl]-N-benzyloxy-1-benzofurane-5-carboxamide

On fait réagir 2,45g d'acide 2-butyl-3-[4-[3-(dibutylamino)propoxy]benzoyl-1-benzofurane-5-carboxylique, 0,855g (1,1 équivalent) de chlorhydrate de benzyloxyamine, 2,36g (1,1 équivalent) de BOP et 2ml (environ 3 équivalents) de triéthylamine dans 70ml de dichlorométhane. On agite durant 1 heure à température ordinaire, on évapore et on reprend dans l'acétate d'éthyle. On lave ensuite les extraits avec de l'eau, une solution de sulfate acide de potassium, de l'eau, une solution de bicarbonate sodique, de l'eau jusqu'à neutralité puis avec une solution saturée de chlorure de sodium.
De cette manière, on obtient environ 3g de composé désiré brut

**B**. Oxalate de 2-butyl-3-[4-[3-(dibutylamino)propoxy]benzoyl]-N-hydroxy-1-benzofurane-5-carboxamide

On hydrogène 3,24g de 2-butyl-3-[4-[3-(dibutylamino)propoxy]benzoyl]-N-benzyloxy-1-benzofurane-5-carboxamide dans 100ml de méthanol en présence de charbon palladié à 5%, à température ambiante et à pression normale. On filtre sur terre de diatomées et on évapore. On purifie alors par chromatographie sur silice (éluant : dichlorométhane/méthanol/ammoniaque 90/10/0,5), ce qui fournit 1,82g (rendement : 66%) du produit désiré sous forme basique.
On introduit ensuite 1,565g de la base ainsi obtenue dans une solution de 0,270g d'acide oxalique dans le méthanol. On évapore, reprend par l'éther diéthylique et laisse cristalliser.
De cette manière, on obtient le composé désiré sous forme d'une poudre amorphe.
Spectre RMN : conforme

**EXEMPLE 6**

**2-Butyl-3-[4-[3-[(2,2-diméthyl-propyl)amino]propoxy]benzoyl]-1-benzofurane-5-carboxylate de méthyle**

On introduit 16,73g de 2-butyl-3-[4-(3-bromopropoxy)benzoyl]-1-benzofurane-5-carboxylate de méthyle, 15,52g (5 équivalents) de néopentylamine et 19,7g de carbonate de potassium dans 200ml de diméthylsulfoxyde.
On agite à température ambiante durant 18 heures, on évapore, on reprend dans l'eau et on extrait avec de l'acétate d'éthyle. On lave ensuite 2 fois avec de l'eau puis avec une solution saturée de chlorure de sodium. On purifie alors par chromatographie sur silice (éluant : dichlorométhane/méthanol 95/5) et on laisse cristalliser dans l'heptane.
De cette manière, on obtient 10,6g de composé désiré.
Rendement : 62,5%
P.F.: 61-63˚C
Spectre RMN : conforme

**EXEMPLE 7**

**Oxalate de 2-butyl-3-[4-[3-(dibutylamino]propoxy]benzoyl]-N,N-diéthyl-1-benzofurane-5-carboxamide**

On introduit 2,27g d'acide 2-butyl-3-[4-[3-(dibutylamino)propoxy]benzoyl]-1-benzofurane-5-carboxylique et 2,5ml de chlorure de thionyle dans 50ml de chloroforme.
On porte au reflux durant 1 heure et on évapore. On reprend le résidu dans l'éther diéthylique et on évapore deux fois. On dissout ensuite le résidu dans 50ml de dichlorométhane et on ajoute 1,61 g de N,N-diéthylamine dissoute dans 10ml de dichlorométhane.
On évapore, on reprend le résidu dans une solution de carbonate de potassium puis on extrait à l'éther diéthylique. On lave ensuite avec de l'eau et une solution saturée de chlorure de sodium et on purifie par chromatographie sur silice (éluant : dichlorométhane/méthanol 95/5), ce qui fournit 1,65g (rendement : 66%) de composé désiré sous forme de base libre.
On dissout ensuite, dans le méthanol, 1,62g de la base ainsi obtenue et 0,259g d'acide oxalique puis on évapore. On reprend le résidu dans l'éther diéthylique et on laisse cristalliser. On filtre, lave avec de l'éther diéthylique et sèche sous vide.
De cette manière, on obtient 1,54g de composé désiré sous forme d'un solide. Spectre RMN : conforme

**EXEMPLE 8**

**Oxalate de 2-butyl-3-[4-[3-dibutylamino)propoxy]benxoyl]-1-benzofurane-5-carboxyrlate de [2-(diméthyrlamino)éthanol]**

Sous atmosphère d'azote, on mélange 1,93g (3,8 mmoles) d'acide 2-butyl-3-[4-[3-(dibutylamino)propoxy]benzoyl]-1-benzofurane-5-carboxylique et 0,615g de carbonyl di-imidazole dans 20ml de N,N-diméthylformamide. On chauffe le milieu réactionnel à 40˚C durant 1 heure puis on ajoute 0,583g de 1,8-diazabicyclo[5.4.0]undec-7-ene et ensuite 0,678g (7,60 mmoles) de 2-(diméthylamino)éthanol. On maintient à 40˚C pendant 18 heures puis on concentre à sec. On extrait ensuite avec de l'acétate d'éthyle, lave l'extrait avec de l'eau et une solution saturée de chlorure de sodium et purifie par chromatographie sur silice (éluant : dichlorométhane/méthanol 90/10) pour obtenir 1,47g de produit désiré sous forme de base que l'on traite alors par une solution d'acide oxalique dans l'éthanol absolu.
De cette manière, on recueille 1,122g de composé désiré sous forme d'un solide amorphe.
Rendement : 66,8%
Spectre RMN : conforme

**EXEMPLE 9**

**3-{[(2-butyl-3-{4-[3-(dibutylamino)propoxy]benzoyl}-1-benzofurane-5-yl)carbonyl]amino}propanoate de méthyle**

Dans 50ml de dichlorométhane contenant 15ml de N,N-diméthylformamide, on introduit 2,54g (5mmoles) d'acide 2-buiyl-3-[4-[3-(dibutylamino)propoxy]benzoyl]-1-benzofurane-5-carboxylique, 0,768g (5,5mmoles) de chlorhydrate de 2-(méthoxycarbonyl)éthylamine, 2,3ml (16,5 mmoles) de triéthylamine et 2,43g (5,5mmoles) de BOP. On agite le milieu réactionnel à température ambiante durant 2 heures, on évapore et on extrait le résidu avec de l'acétate d'éthyle. On lave alors avec de l'eau, une solution de sulfate acide de potassium, de l'eau, une solution de bicarbonate sodium,

de l'eau jusqu'à neutralité et une solution saturée de chlorure de sodium. On purifie ensuite par chromatographie sur silice (éluant : dichlorométhane/méthanol 100/3).

De cette manière, on obtient 2,1 g de composé désiré.
Rendement: 71%
Spectre RMN : conforme

**EXEMPLE 10**

**Acide 3-{[(2-butyl-3-{4-[3-(dibutylamino)propoxy]benzoyl}-1-benzofurane-5-yl)carbonyl]amino}propionique**

On introduit 2g de 3-{[(2-butyl-3-{4-[3-(dibutylamino)propoxy]benzoyl}-1-benzofurane-5-yl)carbonyl]amino} propanoate de méthyle et 0,270g (2équivalents) d'hydroxyde de sodium dans un mélange de 50ml de dioxane, puis 10ml de méthanol et 10ml d'eau. On agite durant 2 heures à température ambiante, on évapore puis on reprend le résidu dans l'eau. On acidifie alors jusqu'à un pH d'environ 5 avec de l'acide chlorhydrique dilué, on extrait avec du dichlorométhane et on lave avec un solution saturée de chlorure de sodium. On purifie ensuite par chromatographie sur silice (éluant : dichlorométhane/méthanol 100/7).

De cette manière, on obtient 1,55g de composé désiré sous forme d'un solide amorphe.
Rendement : 79%
Spectre RMN : conforme

**EXEMPLE 11**

**2-Butyl-3-[4-[3-(dibutylamino)propoxy]benzoyl]-1-benzofurane-5-carboxamide**

On mélange 3,5g de 2-butyl-3-[4-[3-(dibutylamino)propoxy]benzoyl]-5-cyano-1-benzofurane dans 35ml d'acide sulfurique concentré.

On agite le milieu réactionnel à température ambiante durant 24 heures puis on verse sur de la glace. On alcalinise à froid avec de l'hydroxyde de sodium et on extrait au dichlorométhane. On lave alors avec une solution saturée de chlorure de sodium et on purifie par cristallisation dans l'heptane.

De cette manière, on obtient 2,68g de composé désiré.
Rendement : 74%
P.F. : 90 - 92°C
Spectre RMN : conforme

**EXEMPLE 12**

**2-Butyl-3-[4-[3-(dibutylamino)propoxy]benzoyl]-5-(1*H*-tétrazol-5-yl)-1-benzofurane**

On introduit 3,26g de 2-butyl-3-[4-[3-(dibutylamino)propoxy]benzoyl]-5-cyano-1-benzofurane et 4,5g (environ 2 équivalents) d'azide de tributylétain dans 80ml de toluène. On chauffe le mélange au reflux durant 90 heures, on évapore le solvant et on chromatographie le résidu sur silice (éluant : dichlorométhane / méthanol 92/8). On laisse ensuite cristalliser dans l'éther diisopropylique.

De cette manière, on obtient 3,05g de composé désiré.
Rendement : 86%
P.F. : 145-147°C
Spectre RMN : conforme

**EXEMPLE 13**

**2-Butyl-3-[4-[3-(dibutylamino)propoxy]benzoyl]-1-benzofurane-5-carbohydrazide**

On introduit 2,16g (4,3mmoles) d'acide 2-butyl-3-[4-[3-(dibutylamino)propoxy] benzoyl]-1-benzofurane-5-carboxylique et 2,5ml de chlorure de thionyle dans 50ml de chloroforme. On porte le mélange au reflux durant 1 heure et on évapore.

On reprend le résidu dans l'éther diéthylique et on évapore le solvant. On répète ces deux opérations. On reprend le chlorure d'acyle ainsi formé dans 15ml de tétrahydrofuranne et on ajoute cette solution goutte à goutte à une solution de 1ml d'hydrate d'hydrazine à 98% dans 15ml de tétrahydrofuranne. On agite le milieu réactionnel à température ambiante puis on évapore. On extrait avec de l'acétate d'éthyle et on lave à l'eau. On purifie ensuite par chromatographie

sur silice (éluant : dichlorométhane/méthanol/ammoniaque 85/5/0,2)

De cette manière, on obtient 1,13g de composé désiré.
Rendement: 41 %
Spectre RMN : conforme

**EXEMPLE 14**

**Chlorhydrate de 5-(2-butyl-3-[4-[3-(dibutylamino)propoxy]benzoyl]-1-benzofurane-5-yl)-1,3,4-oxadiazol-2-(3*H*)-one**

On dissout 1,13g de 2-butyl-3-[4-[3-(dibutylamino)propoxy]benzoyl]-1-benzofurane-5-carbohydrazide dans 20ml de chloroforme et on ajoute cette solution goutte à goutte à une solution de 2,2g de phosgène dans 30ml de chloroforme. On porte le mélange au reflux durant 6 heures. Après retour à température ambiante on lave d'abord avec de l'eau jusqu'à neutralité et ensuite avec une solution saturée de bicarbonate sodique.

De cette manière, on obtient 0,535g de composé désiré après cristallisation dans l'éther diéthylique.
Rendement : 45%
Spectre RMN : conforme

EXEMPLE 15

**Oxalate de 2-butyl-3-[4-[3-(dibutyrlamino)propoxy]benzoyl]-1-benzofurane-5-carbaldéhyde,(E) O-méthyloxime**

**A**. 2-Butyl-5-hydroxyméthyl-benzofurane

A 0,400g d'hydrure de lithium aluminium dans 20ml d'éther diéthylique, on ajoute goutte à goutte 2,32g (0,01mole) de 2-butyl-1-benzofurane-5-carboxylate de méthyle dans 20ml d'éther diéthylique puis on porte à la température de reflux de l'éther.

En fin d'addition, on agite durant 1 heure à température ambiante. On refroidit dans un mélange glace/eau, on hydrolyse au moyen d'une solution d'acide chlorhydrique 1 N et on décante. On extrait avec de l'éther diéthylique et on lave avec de l'eau et une solution saturée de chlorure de sodium.

De cette manière, on obtient 1,92g de composé désiré.
Rendement : 94%

**B.** 2-Butyl-1-benzofurane-5-carbaldéhyde

On refroidit à -60°C, 3,17g (0,025mole) de chlorure d'oxalyle dans 50ml de dichlorométhane puis on ajoute 3,67g (0,054mole) de diméthylsulfoxyde dans 20ml de dichlorométhane. On agite durant 10 minutes puis on ajoute 3,43g (17mmoles) de 2-butyl-5-hydroxyméthyl-1-benzofurane dans 50ml de dichlorométhane. On agite durant 15 minutes, on ajoute 15,7ml (0,113mole) de triéthylamine et on laisse revenir à température ambiante. On ajoute de l'eau et on décante. On extrait avec du dichlorométhane. On lave alors avec de l'eau, une solution de sulfate acide de potassium jusqu'à neutralité, avec de l'eau, une solution de carbonate de sodium, de l'eau et enfin avec une solution saturée de chlorure de sodium.

De cette manière, on obtient le composé désiré que l'on utilise sous forme brute.

**C.** 2-Butyl-1-benzofurane-5-carbaldéhlyde,(E) O-méthyloxime

On ajoute 2,07g de 2-butyl-1-benzofurane-5-carbaldéhyde, 1,38g de chlorhydrate de méthoxyamine et 1,57g de pyridine dans 25ml de méthanol. On agite durant 1,5 heure à température ambiante évapore et reprend dans l'éther diéthylique. On lave avec de l'eau, une solution de sulfate acide de potassium, de l'eau et une solution saturée de chlorure de sodium. On purifie ensuite par chromatographie sur silice (éluant : dichlorométhane/heptane 1/1)

De cette manière, on obtient 1,96g de composé désiré.
Rendement : 83%.

**D**. 2-Butyl-3-[4-(3-bromopropoxy)-benzoyl]-1-benzofurane-5-carbaldéhyde, (E) O-méthyloxime

On introduit, sous argon, 6,94g (2 équivalents) de chlorure ferrique à 40ml de dichloréthane. On ajoute alors, vers +10°C, 4,91g ((21 mmoles) de 2-butyl-1-benzofurane-5-carbaldéhyde,(E) O-méthyloxime dissous dans 25ml de dichloréthane puis on introduit, entre +10 et +15°C, 11,89g (2 équivalents) de 1-chlorocarbonyl-4-(3-bromopropoxy)-benzène dans 25ml de dichlorométhane. On laisse revenir à température ambiante puis on agite durant 5 heures à température ambiante: On verse alors sur un mélange glace/eau, on filtre le précipité et on décante le filtrat. On purifie alors par chromatographie sur silice (éluant : dichlorométhane)

De cette manière, on obtient le composé désiré à savoir 2,74g d'isomère (E) (rendement : 27,5%) et 2,05g d'isomère (Z).
P. F. : 78-81 ˚C

**E**. Oxalate de 2-butyl-3-[4-[3-(dibutylamino)propoxy]benzoyl]-1-benzofurane-5-carbaldéhyde,(E) O-méthyloxime

Dans 25ml d'acétonitrile, on introduit 2,3g (5mmoles) de 2-butyl-3-[4-(3-bromopropoxy)-benzoyl]-1-benzofurane-5-carbaldéhyde,(E) O-méthyloxime, 1,28g (2 équivalents) de dibutylamine, 1,38g (2 équivalents) de carbonate de potassium et 0,75g (1 équivalent) d'iodure de sodium. On porte au reflux durant 4 heures et on évapore. On extrait avec de l'acétate d'éthyle et on lave avec de l'eau et une solution saturée de chlorure de sodium. On purifie ensuite par chromatographie sur silice (éluant : dichlorométhane/méthanol 100/2,5), ce qui fournit 2,24g (rendement : 88%) de composé désiré sous forme basique.

On ajoute alors dans du méthanol 2,0g de base ainsi obtenue et 0,361g d'acide oxalique et on évapore. On reprend dans l'éther diéthylique et on laisse cristalliser.

De cette manière, on obtient 2,25g de composé désiré.
Rendement : 92%
P.F. : 97-99˚C
Spectre RMN : conforme

**EXEMPLE 16**

**Oxalate de 2-butyl-3-[4-[3-(dibutyrlamino)propoxy] benzoyl]-5-hydroxyméthyl-1-benzofurane**

**A.** 2-Butyl-3-[2-(4-hydroxyphényl)-1,3dioxolan-2-yl]-1-benzofurane-5-carboxylate de méthyle

On porte au reflux un mélange de 4,93g (14mmoles) de 2-butyl-3-(4-hydroxy-d'éthylène glycol-benzoyl)-1-benzofurane-5-carboxylate de méthyle, 2,17g et 0,500g d'acide p-toluènesulfonique dans 250ml de benzène. On dilue avec de l'éther diéthylique, lave avec une solution de bicarbonate de sodium, de l'eau et une solution de chlorure de sodium. On sèche et évapore.

De cette manière, on obtient le composé désiré que l'on utilise sous forme brute.

**B.** 2-Butyl-5-hydroxyméthyl-3-[2-(4-hydroxyphényl)-1,3-dioxolan-2-yl]-1-benzofurane

A 1,4g d'hydrure de lithium aluminium dans 50ml de tétrahydrofurane, on ajoute, goutte à goutte, le produit brut obtenu à l'étape précédente, dissous dans 50ml de tétrahydrofurane.

On agite durant 2 heures à température ambiante, on hydrolyse avec une solution diluée d'acide chlorhydrique, jusqu'à pH=3, et on décante. On extrait avec de l'éther diéthylique puis on lave avec de l'eau et une solution saturée de chlorure de sodium.

De cette manière, on obtient le composé désiré que l'on utilise sous forme brute. Toutefois celui-ci peut être purifié par chromatographie sur silice (éluant: dichlomléthane/méthanol 100/2,5)
P.F. : 100-101˚C

**C**. 2-Butyl-3-(4-hydroxy-benzoyl)-5-hydroxyméthyl-1-benzofurane

On porte au reflux durant 3 heures, une solution formée par le produit brut obtenu à l'étape précédente et 0,8g de pyridine p-toluènesulfonate dans 100ml d'acétone contenant 10ml d'eau. On évapore et on reprend dans un mélange éther diéthylique/eau. On filtre le précipité obtenu et on le lave avec de l'eau et de l'éther diéthylique, ce qui fournit un premier jet du produit souhaité.

On décante la phase organique, lave avec de l'eau, une solution de bicarbonate de sodium, de l'eau et une solution de chlorure de sodium.

On sèche, évapore, reprend le résidu avec de l'éther diéthylique et filtre ce qui fournit un deuxième jet du produit souhaité.

De cette manière, on obtient 3,37g de composé désiré après cristallisation dans l'éther diéthylique.
Rendement : 74%
P.F. : 180-182˚C

**D.** Oxalate de 2-butyl-3-[4-[3-(dibutylamino)propoxy] benzoyl]-5-hydroxyméthyl-1-benzofurane

On dissout 1,68g de 2-butyl-3-(4-hydroxy-benzoyl)-5-hydroxyméthyl-1-benzofurane, 1,18g de 1-chloro-3-(dibutylamino)propane et 0,960g de carbonate de potassium dans 70ml de méthyléthylcétone. On porte au reflux durant 6 heures, on ajoute de l'eau et on décante. On extrait avec de l'acétate d'éthyle et on lave avec une solution saturée de chlorure de sodium. On purifie ensuite par chromatographie sur silice (éluant : dichlorométhane/méthanol 95/5), ce qui fournit le composé souhaité sous forme de base libre.

On mélange ensuite 2,50g de la base ainsi obtenue et 0,455g d'acide oxalique dans du méthanol, on évapore et on reprend dans l'éther diéthylique. On laisse cristalliser, on filtre et on lave avec de l'éther diéthylique.

De cette manière, on obtient 2,57g de composé désiré.
Rendement : 85,5%
Spectre RMN : conforme

**EXEMPLE 17**

**2-Butyl-3-[4-[3-(*cis*-3,5-diéthyl-1-pipéridinyl)-propoxy]benzoyl-5-(3-méthyl-1,2,4-oxadiazol-5-yl]-1-benzofurane**

**A.** 2-Butyl-3-(4-hydroxy-benzoyl)-5-(3-méthyl-1,2,4-oxadiazol-5-yl)-1-benzofurane

Dans un ballon tricol, on place, sous argon, 1,018g acétamide oxime, 0,660g d'hydrure de sodium, 6g de tamis moléculaire et 60ml de tétrahydrofurane. On chauffe à 60°C pendant 1 heure, on ajoute 4,03g de 2-butyl-3-(4-hydroxy-benzoyl)-1-benzofurane-5-carboxylate de méthyle dissous dans 50ml de tétrahydrofurane et on porte au reflux durant 3 heures. On dilue avec de l'eau, ajoute une solution de chlorure de sodium et extrait à l'acétate d'éthyle. On lave avec une solution saturée de chlorure de sodium.

De cette manière, on obtient 3,4g de composé désiré sous forme cristallisée.
Rendement : 79%
P.F. : 180-182°C

**B**. 2-Butyl-3-[4-(3-bromopropoxy)benzoyl]-5-(3-méthyl-1,2,4-oxadiazol-5-yl)-1-benzofurane

On mélange 4,26g (0,0113mole) de composé obtenu à l'étape précédente, 11,44g (5 équivalents) de 1,3-dibromo-propane et 1,88g (1,2 équivalent) de carbonate de potassium dans 100ml de méthyléthylcétone. On porte au reflux pendant 3 heures et on évapore. On reprend dans l'eau et on extrait à l'acétate d'éthyle. On lave avec une solution saturée de chlorure de sodium et on purifie par chromatographie sur silice (éluant : dichlorométhane/méthanol 100/3)

De cette manière, on obtient 3,58g de composé désiré.
Rendement : 64%
P.F. : 85-87°C

**C**. 2-Butyl-3-[4-[3-(*cis*-3,5-diéthyl-1-pipéridinyl)-propoxy]benzoyl-5-(3-méthyl-1,2,4-oxadiazol-5-yl]-1-benzofurane

On introduit dans 100ml d'acétonitrile 3,4g de composé obtenu à l'étape précédente, 1,21g (1,1 équivalent) de chlorhydrate de *cis*-3,5-diéthylpipéridine, 1,03g (1 équivalent) d'iodure de sodium et 2,83g (3 équivalents) de carbonate de potassium. On porte au reflux durant 6 heures, on dilue avec de l'eau et on décante. On extrait avec de l'acétate d'éthyle et on lave avec une solution saturée de chlorure de sodium. On purifie ensuite par chromatographie sur silice (éluant : dichlorométhane/méthanol 100/2,5 et ammoniaque 20% : 2 gouttes).

De cette manière, on obtient 2,55g de composé désiré.
Rendement : 67%
P.F. : 81-83°C (après recristallisation dans l'heptane)
Spectre RMN : conforme

**EXEMPLE 20**

**2-Butyl-3-(4-{2-[méthyl(4-nitrophénéthyl)amino]éthoxy}benzoyl)-1-benzofurane-5-carboxyrlate de méthyle**

On introduit 4,3g (9,36 mmoles) de 2-butyl-3-[4-(3-bromopropoxy)-benzoyl]-1-benzofurane-6-carboxylate de méthyle dans 50ml d'acétonitrite puis on ajoute 1,7g (9,36 mmoles) de N-méthyl N-(4-nitrophényl)éthylamine, 1.4g (9,36 mmoles) d'iodure de sodium et 2,58g (18,7 mmoles) de carbonate de potassium.

On porte au reflux durant 18 heures, on concentre à sec et on extrait à l'acétate d'éthyle. On lave avec de l'eau et une solution saturée de chlorure de sodium puis on purifie par chromatographie sur silice (éluant : dichlorométhane/méthanol 99/1 puis 97/3).

De cette manière, on obtient 5,0g de composé désiré.
Rendement : 95,6%
Spectre RMN : conforme

## EXEMPLE 21

### 3-(4-{2-[(4-Aminophénéthyl)(méthyl)amino]éthoxy}benzoyl)-2-butyl-1-benzofurane-5-carboxylate de méthyle

On introduit 5,0g de composé obtenu à l'exemple précédent dans 100ml d'éthanol absolu et on ajoute une quantité catalytique de nickel de Raney.

On hydrogène ensuite sous pression normale et à température ambiante. Lorsque la réaction n'évolue plus, on filtre sur terre de diatomées et rince avec de l'éthanol absolu. On concentre et on purifie ensuite par chromatographie sur silice (éluant : dichlorométhane/méthanot 97/3 puis 95/5).

De cette manière, on obtient 3,57g de composé désiré.
Rendement : 75,5%
Spectre RMN : conforme

## EXEMPLE 22

### 2-Butyrl-3-{4-[2-(méthyl{4-[(méthyrlsutfonyl)amino]phénéthyl}amino) éthoxy]benzoyl}-1-benzofurane-5-carboxyrlate de méthyle

Dans 200ml de dichlorométhane, on introduit 3,57g (6,75 mmoles) de composé obtenu à l'étape précédente et 2,59g (14,9 mmoles) d'anhydride méthanesulfonique. On agite à température ambiante pendant 18 heures et on concentre à sec. On reprend par une solution de bicarbonate de sodium 2N et on extrait à l'acétate d'éthyle. On lave avec de l'eau et une solution de chlorure de sodium puis on purifie par chromatographie sur silice (éluant : dichlorométhane/méthanol 97/3 puis 50/50 et enfin méthanol pur).

De cette manière, on obtient 2,928g de composé désiré.
Rendement: 71,5%
Spectre RMN : conforme

## EXEMPLE 23

### Oxalate de 3-(5-cyano-3-{4-[3-(dibutylamino)propoxy]benzoyl}-1-benzofurane-2-yl)propanoate de méthyle

**A.** 3-Iodo-4-hydroxy-benzonitrile

On dissout 11,9g (0,1mole) de 4-hydroxy-benzonitrile dans 250ml de méthanol et on ajoute 250ml d'ammoniaque à 20%. Avec précaution, en raison du caractère explosif de la réaction, on ajoute alors, goutte à goutte, une solution de 31,75g d'iode dans 250ml de méthanol. Après addition, on agite durant 2 heures à température ambiante. On évapore le méthanol, dilue dans l'eau et acidifie jusqu'à pH = 2 à 3 avec une solution d'acide chlorhydrique. On extrait ensuite avec de l'acétate d'éthyle et on lave avec de l'eau, une solution de thiosulfate de sodium et une solution saturée de chlorure de sodium.

De cette manière on obtient 24,73g de composé désiré.
P.F. : 144-146°C

En utilisant le même procédé que précédemment, on a préparé le composé ci-dessous :
3-Iodo-4-hydroxy-benzoate d'isopropyle.

**B**. 3-(5-Cyano-1-benzofurane-2-yl)propanoate de méthyle

Dans 125ml de N,N-diméthylformamide, on introduit 19,38g (79mmoles) de composé obtenu à l'étape précédente, 9,81g (1,1 équivalent) de pent-4-inoate de méthyle, 0,750g (0,05 équivalent) d'iodure de cuivre, 2,77g de dichloro bis-(triphénylphosphine)palladium et 100ml (10 équivalents) de tétraméthylguanidine. On agite à température ambiante, durant 20 heures, dilue avec 1000ml d'eau et extrait avec 600ml d'acétate d'éthyle. On lave avec de l'eau, de l'acide chlorhydrique dilué, de l'eau, et une solution saturée de chlorure de sodium. On purifie ensuite par chromatographie

sur silice (éluant : dichlorométhane/acétate d'éthyle 100/1)

De cette manière, on obtient 9,93g de composé désiré.

Rendement : 59%

P.F. : 91-92°C

En suivant le même procédé que ci-dessus, on a obtenu le composé suivant :

3-(5-Isopropoxycarbonyl-1-benzofurane-2-yl)propanoate de méthyle.

P.F. : 62-64°C

**C**. 3-[5-Cyano-3-(4-méthoxy-benzoyl)-1-benzofurane-2-yl]propanoate de méthyle

A une solution de 11,46g (1,5 équivalent) de chlorure ferrique dans 80ml de dichloréthane maintenue sous argon, on ajoute vers +10°C, 9,91g de composé obtenu à l'étape précédente dans 50ml de dichloréthane puis, à la température de +10 à +15°C, on introduit 12,03g (1,5 équivalent) de chlorure d'anisoyle dans 50ml de dichloréthane. On laisse revenir à la température ambiante puis on agite, à cette température, durant 5 heures. On verse sur un mélange glace/eau, filtre le précipité et décante le filtrat. On extrait la phase aqueuse avec du dichlorométhane et lave les phases organiques que l'on purifie par chromatographie sur silice (éluant : dichlorométhane/acétate d'éthyle 100/2).

De cette manière, on obtient 10,78g de composé désiré.

Rendement : 64%

P.F. : 95-98°C

**D.** 3-[5-Cyano-3-(4-hydroxy-benzoyl)-1-benzofurane-2-yl]propanoate de méthyle

On introduit 9,73g de composé obtenu à l'étape précédente et 10,8g de chlorure d'aluminium dans 350 ml de toluène. On chauffe à 80°C pendant 6 heures puis on décante. On reprend le résidu dans du tétrahydrofurane et on ajoute doucement de la glace. On décante, extrait la phase aqueuse avec de l'acétate d'éthyle et réunit les 3 phases organiques. On lave avec de l'eau et on purifie alors par chromatographie sur silice (éluant : dichlorométhane/acétate d'éthyle 95/5 puis 90/10).

De cette manière, on obtient 5,57g de composé désiré.

Rendement : 95%

P.F. : 156-158°C

**E**. Oxalate de-3-(5-cyano-3-{4-[3-(dibutyamino)propoxy]benzoyl}-1-benzofurane-2-yl)propanoate de méthyle

Dans 70ml de méthyléthylcétone, on introduit 4,54g (13mmoles) de composé obtenu à l'étape précédente, 2,95g (1,1 équivalent) de 3-chloro-1-(dibutylamino)-propane et 2,16g (1,2 équivalent) de carbonate de potassium. On porte au reflux durant 6 heures, puis on dilue avec de l'eau et on extrait à l'acétate d'éthyle. On lave avec une solution saturée de chlorure de sodium et on purifie ensuite par chromatographie sur silice (éluant : heptane/acétate d'éthyle 5/5, dichlorométhane/méthanol 95/3 puis 90/10), ce qui fournit 6,65g du composé désiré sous forme de base libre. On prélève 2,32g de cette base et on l'introduit dans du méthanol. On y ajoute 0,416g d'acide oxalique et on évapore. On reprend dans l'éther diéthylique et on évapore. On purifie ensuite par cristallisation dans l'éther diéthylique.

De cette manière, on obtient 2,4g de composé désiré.

Rendement : 87%

P.F. : 76-79°C

Spectre RMN : conforme

En suivant le même procédé que ci-dessus mais en utilisant l'acide chlorhydrique en remplacement de l'acide oxalique, on obtient :

Chlorhydrate de 3-(4-{3-[*cis*-3,5-diéthylpipéridinyl]propoxy}benzoyl)-2-(3-méthoxy-3-oxopropyl)-1-benzofurane-5-carboxylate de méthyle **(Exemple 24).**

Rendement: 81%

P.F.: 166-169°C (après cristallisation dans l'éther diéthylique)

Spectre RMN : conforme

**EXEMPLE 25**

**Oxalate de 2-butyl-5-cyano-3-[4-[3-(dibutylamino)propoxy]-benzoyl]-1-benzofurane**

**A.** 2-Méthoxy-5-cyano-benzaldéhyde

On chauffe, sous argon, pendant 3 heures, un mélange de 40,63g de 2-méthoxy-5-bromo-benzaldéhyde, 13,35g de cyanure de zinc et 8,7g de tétrakis-(triphénylphosphine)palladium dans 240ml de N,N-diméthylformamide désoxygéné. On laisse revenir à température ambiante et on extrait avec 600ml de toluène. On lave ensuite avec 2 fois 600ml d'ammoniaque 2N puis avec une solution saturée de chlorure de sodium. On purifie alors par cristallisation dans l'éther diisopropylique.

De cette manière, on obtient 30,2g de composé désiré.
Rendement : 99%
P. F. : 115-118°C

**B.** 2-Hydroxy-5-cyano-benzaldéhyde

Dans 500ml de N,N-diméthylformamide, on introduit 31,08g de composé obtenu à l'étape précédente et 24,52g de chlorure de lithium. On porte au reflux durant 2 heures et on évapore le solvant. On reprend dans une solution de sulfate acide de potassium et on extrait à l'acétate d'éthyle. On lave alors avec de l'eau et une solution saturée de chlorure de sodium.

De cette manière, on obtient 24,5g de composé désiré
Rendement : 86%

**C.** 2-(2-Formyl-4-cyano-phénoxy)hexanoate de méthyle

On place 24,5g de composé obtenu à l'étape précédente, 47,52g de 2-bromo-hexanoate de méthyle et 28,8g de carbonate de potassium dans 400ml de N,N-diméthylformamide. On chauffe à environ 80°C durant 1,5 heure et on évapore le solvant, On reprend dans une solution de sulfate acide de potassium et on extrait avec de l'acétate d'éthyle. On lave alors avec de l'eau et une solution saturée de chlorure de sodium.

De cette manière, on obtient 53,6g de composé désiré sous forme brute.
P.F. : 77-79°C

**D**. Acide 2-(2-formyl-4-cyano-phénoxy)hexanoïque

On introduit 53,6g de composé brut obtenu à l'étape précédente et 8,6g d'hydroxyde de sodium dans 320ml de méthanol contenant 200ml de dioxanne et 160ml d'eau. On agite durant 1,5 heure à température ambiante et on évapore. On reprend dans l'eau contenant de l'acide chlorhydrique concentré et on extrait à l'acétate d'éthyle. On lave alors avec de l'eau et une solution saturée de chlorure de sodium.

De cette manière, on obtient 52,1g de composé désiré sous forme brute.

**E.** 2-Butyl-5-cyano-1-benzofurane

On introduit 47,66g de chlorure de benzènesulfonyle dans 100ml de toluène et on ajoute 68,23g de triéthylamine dans 50ml de toluène. On chauffe à 80°C puis on ajoute goutte à goutte, à une température inférieure à 90°C, 52,1g de composé obtenu à l'étape précédente dissous dans 400ml de toluène. Dès que l'addition est terminée, on poursuit le chauffage durant 0,5 heure puis on laisse revenir à température ambiante. On lave avec de l'eau et extrait la phase aqueuse avec du toluène. On agite les phases organiques réunies avec 100ml d'hydroxyde de sodium 2N et on décante. On extrait avec du toluène et on lave avec de l'eau, une solution de bisulfate de potassium, de l'eau et une solution saturée de chlorure de sodium. On purifie alors par chromatographie sur silice (éluant : dichlorométhane).

De cette manière, on obtient 19,94g de composé désiré.
Rendement global pour les 5 étapes : 53%

**F.** 2-Butyl-5-cyano-3-(4-méthoxy-benzoyl)-1-benzofurane

Sous argon, on introduit 32,43g (2 équivalents) de chlorure ferrique dans 200ml de dichloréthane. Aux environs de 10°C, on ajoute alors 19,92g de composé obtenu à l'étape précédente puis à une température comprise entre +10 et +15°C, on introduit 34,36g (2 équivalents) de chlorure d'anisoyle dissous dans 200ml de dichloréthane. On laisse

revenir à température ambiante et on agite à cette température durant 16 heures. On verse sur un mélange glace/eau et on filtre sur verre fritté. On décante le filtrat, extrait la phase aqueuse avec du dichlorométhane et mélange les phases organiques. On lave avec une solution diluée de bicarbonate sodique, de l'eau et une solution de chlorure de sodium saturée. On purifie ensuite par chromatographie sur silice (éluant : dichlorométhane).

De cette manière, on obtient 24,87g de composé désiré.
Rendement : 74,5%

**G.** 2-Butyl-5-cyano-3-(4-hydroxy-benzoyl)-1-benzofurane

Dans 200ml de toluène on introduit 16,27g de composé obtenu à l'étape précédente et 19,9g de chlorure d'aluminium. On chauffe à 60°C durant 2 heures et on décante le toluène. On dissout le résidu dans du tétrahydrofurane et on ajoute un mélange eau/glace. On agite durant 1 heure, décante et extrait la phase aqueuse avec de l'acétate d'éthyle. On mélange alors les 3 phases organiques et on lave avec de l'eau et une solution de chlorure de sodium. On purifie ensuite par cristallisation dans l'heptane.

De cette manière, on obtient 14,05g de composé désiré.
Rendement : 90%
P.F.: 152-153°C

**H** Oxalate de 2-butyl-5-cyano-3-[4-[3-(dibutylamino)propoxy]-benzoyl]-1-benzofurane

Dans 30ml de méthyléthylcétone, on introduit 2,02g de composé obtenu à l'étape précédente, 1,45g de 3-chloro-1-(dibutylamino)propane et 1,06g de carbonate de potassium. On porte au reflux durant 6 heures et on ajoute de l'eau. On décante et extrait la phase aqueuse avec de l'acétate d'éthyle. on lave les phases aqueuses réunies avec une solution saturée de chlorure de sodium et on extrait avec de l'acétate d'éthyle. On purifie ensuite par chromatographie sur alumine (éluant : heptane/acétate d'éthyle 95/5), ce qui fournit le composé désiré sous forme de base libre (2,25g ; rendement : 72%). On mélange alors 1,86g de ce composé basique et 0,343g d'acide oxalique dissous dans du méthanol. On évapore et on ajoute de l'éther diéthylique. On agite durant quelques heures, filtre et lave avec de l'éther diéthylique. On laisse alors cristalliser.

De cette manière, on obtient 2,11g d'oxalate de composé désiré.
Rendement : 96%
P.F.: 85-87°C
Spectre RMN : conforme

**EXEMPLE 26**

**3-{[(2-Butyl-3-{4-[3-(néopentylamino)propoxy]benxoyl}-1-benzofurane-5-yl)carbonyl]amino}propanoate de méthyle**

**A**. 2-Butyl-3-{4-[3-[(N-néogentyl-N-tertiobutoxycarbonyl)amino]propoxy]-benzoyl}-1-benzofurane-5-carboxylate de méthyle

On introduit, dans 100ml de chloroforme, 8,57g (18mmoles) de 2-butyl-3-[4-[3-(néopentylamino)propoxy]benzoyl]-5-méthoxycarbonyl-benzofurane et 4,29g (1,1 équivalent) d'anhydride de l'acide tertiobutoxycarboxylique. On porte au reflux durant 3 heures puis on évapore.

De cette manière, on obtient le produit désiré sous forme brute.

**B.** Acide 2-butyl-3-{4-[3-[(N-néopentyl-N-tertiobutoxycarbonyl)amino]propoxy]-benzoyl}-1-benzofurane-5-carboxylique

Dans 200ml de dioxanne contenant 40ml de méthanol et 40ml d'eau, on dissout le produit brut obtenu à l'étape précédente. On ajoute 1,45g (environ 2 équivalents) d'hydroxyde de sodium puis on agite à température ambiante durant 48 heures. On évapore, on reprend dans l'eau et on acidifie avec une solution d'hydrogénosulfate de potassium. On extrait avec de l'acétate d'éthyle et on lave avec de l'eau et une solution saturée de chlorure de sodium.

De cette manière, on obtient 11,23g de produit désiré sous forme brute.
Rendement: 100%

**C.** 3-{[(2-Butyl-3-{4-[3-[(N-néopentyl-N-tertiobutoxycarbony)-amino]propoxy]-benzoyl}-1-benzofurane-5-yl)carbonyl] amino}propanoate de méthyle

Dans 150ml de dichlorométhane, on introduit 7,48g de produit brut obtenu à l'étape précédente, 2g (1,1 équivalent) de chlorhydrate de 3-amino-propanoate de méthyle, 4,5g de triéthylamine et 6,32g (1,1 équivalent) de BOP. On agite durant 3 heures à température ambiante puis on évapore. On extrait avec de l'acétate d'éthyle et on lave avec de l'eau, une solution d'hydrogénosulfate de potassium, de l'eau, une solution de carbonate de sodium, de l'eau et enfin une solution saturée de chlorure de sodium. On purifie alors par chromatographie sur sulice (éluant : dichlorométhane/acétate d'éthyle 85/15).

De cette manière, on obtient 6,2g de produit désiré sous forme brute.
Rendement global des 3 étapes : 79,5%

**D**. 3-{[(2-Buyl-3-{4-[3-(néonentylamino)propoxy]benzoyl}-1-benzofurane-5-yl)carbonyl]amino}propanoate de méthyle

Dans 50ml de dichlorométhane, on dissout 6,15g de produit obtenu à l'étape précédente. On refroidit au moyen d'un bain de glace/eau et on ajoute 50ml d'acide trifluoroacétique. On agite à température ambiante durant 1 heure on évapore et on reprend dans l'éther diéthylique. On évapore à nouveau deux fois. On purifie alors par chromatographie sur silice (éluant : dichlorométhane/méthanol/ammoniaque 100/5/0,2).

De cette manière, on obtient 4,5g de composé désiré.
Rendement : 78%

**EXEMPLE 27**

**Oxalate de 2-{[(2-butyl-3-{4-[3-(dibutylamino)propoxy]benzoyl}-1-benzofurane-5-yl)carbonyl]amino}benzoate de méthyle**

On dissout 6,28g (0,012mole) d'acide 2-butyl-3-[4- [3-(dibutylamino)propoxy] benzoyl]-1-benzofurane-5-carboxylique dans 150ml de chloroforme. On ajoute alors 6ml de chlorure de thionyle et on porte au reflux durant 3 heures. On évapore puis on reprend 3 fois dans l'éther diéthylique. On introduit ensuite le chlorure d'acyle brut ainsi formé dans 130ml de dichlorométhane et on ajoute 9g (0,06mole) de 2-méthoxycarbonylaniline. On maintient le milieu réactionnel à température ambiante durant 12 heures et on évapore. On extrait avec de l'acétate d'éthyle et on lave avec une solution diluée de carbonate de potassium, de l'eau et une solution de chlorure de sodium. On purifie alors par chromatographie sur silice (éluant : dichlorométhane/méthanol 96/4), ce qui fournit 5,26g de produit désiré sous forme basique (rendement : 68%).On dissout alors 2g (3,1mmoles) de produit basique, ainsi obtenu, dans 15ml d'éthanol absolu et 0,281g (3,1mmoles) d'acide oxalique dans 10ml d'éthanol absolu. On mélange les deux solutions et on évapore. On reprend dans l'éther diéthylique et on laisse cristalliser. On filtre le précipité formé et on sèche.

De cette manière, on obtient 1,9g de composé désiré.
Rendement : 84%
P. F. : 89˚C
Spectre RMN : conforme

**EXEMPLE 28**

**Chlorhydrate de l'acide 2-{[(2-butyl-3-{4-[3-(dibutylamino)propoxy]benzoyl}-1-benzofurane-5-yl)carbonyl]amino}benzoique**

On dissout 3,26g (5,1 mmoles) d'oxalate de 2-{[(2-butyl-3-{4-[3-(dibutylamino)propoxy]benzoyl}-1-benzofurane-5-yl]carbonyl]amino}benzoate de méthyle dans 20ml de dioxanne.

On ajoute 0,407g (10 mmoles) d'hydroxyde de sodium dans 4ml d'eau contenant 4ml de méthanol. On abandonne alors le milieu à température ambiante durant 4 heures puis on filtre. On extrait avec de l'acétate d'éthyle puis on lave avec de l'eau, une solution d'acide chlorhydrique 1 N, de l'eau et une solution de chlorure de sodium. On purifie ensuite par chromatographie sur silice (éluant: dichlorométhane/méthanol 96/4).

De cette manière, on obtient 2,06 de produit désiré.
Rendement: 61%
P.F. : 109˚C
Spectre RMN : conforme

## EXEMPLE 29

**Oxalate de 2-butyl-3-(4-{3-[*cis*-3,5-diéthylpipéridinyl]propoxy}benzyl)-1-benzofurane-5-carboxyrlate d'isopropyle**

On introduit 3,19g (4,89 mmoles) de 2-butyl-3-(4-{3-[*cis*-3,5-diéthylpipéridinyl]propoxyybenzoyl)-1-benzofurane-5-carboxylate d'ispopropyle dans 50ml d'isopropanol puis on ajoute successivement 1 cuillerée de charbon palladié à 10% et quelques gouttes d'acide chlorhydrique concentré. On hydrogène ensuite sous pression normale durant 48 heures à 45˚C. On essore sur terre de diatomées, on rince avec de l'isopropanol, on filtre et on concentre. On purifie alors par chromatographie sur silice (éluant : dichlorométhane/méthanol), ce qui fournit 1,533g (rendement : 49,1%) du composé désiré sous forme de base.

On introduit alors 1,32g (2,41 mmoles) de composé basique ainsi obtenu dans une quantité minimale d'éthanol pour en assurer la dissolution et on ajoute 0,217g (2,41 mmole) d'acide oxalique. On concentre, triture dans l'éther diéthylique, essore et sèche. On purifie alors par cristallisation.

De cette manière, on obtient 1,203g de composé désiré.
Rendement : 78,3%
P.F. ; 148-149˚C
Spectre RMN : conforme

## EXEMPLE 32

**Chlorhydrate de l'acide 3-(5-cyano-3-[4-[3-(dibutylamino)propoxy]benzoyl]-1-benzofurane-2-yl)propanoique**

On chauffe à 100˚C pendant 2,5 heures, un mélange de 3,55g de 3-(5-cyano-3-[4-[3-(dibutylamino)propoxy] benzoyl]-1-benzofurane-2-yl)propanoate de méthyle et 4,08g (1 équivalent) d'oxyde de tributyl étain. On dilue avec de l'eau, on extrait avec de l'acétate d'éthyle et on concentre ce qui fournit 3,17g (rendement: 91,5%) de composé désiré sous forme basique.

On dissout alors 3,1g de ce composé basique dans l'acétate d'éthyle, on ajoute une solution de chlorure d'hydrogène dans l'éther diéthylique jusqu'à pH légèrement acide et on laisse cristalliser dans l'acétate d'éthyle. On filtre, on lave avec de l'acétate d'éthyle et on sèche sous vide.

De cette manière, on obtient 2,35g de composé désiré.
Rendement : 70,5%
P.F. : 165-168˚C (acétate d'éthyle)
Spectre RMN : conforme

## EXEMPLE 154

**Oxalate de (2-Butyl-3-(2-[4-(2-dibutylamino-ethoxy)-phenyl]-[1,3]dioxolan-2-yl)-benzofuran-5-yl)-methanol**

**A**. 3-(2-[4-(2-Bromo-ethoxy)-phenyl]-[1,3]dioxolan-2-yl]-2-butyl-benzofuran-5-carboxylate de méthyle

On porte à reflux 3 jours 10,0 g (0,022 moles) de 3-[4-(2-Bromo-ethoxy)-benzoyl]-2-butyl-benzofuran-5-carboxylate de méthyle, 3,39 g (0,055 moles) d'éthylèneglycol, 890 mg d'acide paratoluène sulfonique (ApTs) et 100 ml de benzène en éliminant l'eau formée à l'aide d'un appareil de Dean-Stark. Puis on évapore à sec et chromatographie le résidu sur une colonne de silice en éluant par du DCM (dichlorométhane) puis un mélange DCM/Acétate d'éthyle (99/1). On isole ainsi 5 g du composé désiré pur.

**B.** (3-(2-[4-(2-Bromo-ethoxyl-phenyl]-[1,3]dioxolan-2-yl)-2-bulyl-benzofuran-5yl)-methanol

Sous argon, on dissout 4,87 g (0,097 moles) du composé obtenu à l'étape A précédente dans 20 ml de THF anydre et on refroidit à -70˚ C. On ajoute, à cette température, 32 ml de solution toluénique de diisobutyl aluminium hydrure (DIBAL). Après la fin de l'addition on agite encore pendant 1 heure à -70˚C. On additionne alors, à -70˚ C, 1 ml de méthanol, puis à la fin du dégagement gazeux, on ajoute de l'eau. On filtre sur célite. On lave le gel et célite 3 fois par de l'acétate d'éthyle. On décante la phase organique puis on réextrait la phase aqueuse avec de l'acétate d' éthyle. Les phases organiques réunies sont lavées par de l'eau et une solution aqueuse de NaCl. On sèche sur $Na_2SO_4$. On purifie par chromatographie sur silice (éluant : DCM/Acétate d'éthyle à 92/8)

De cette manière, on obtient au total 3,25 g du composé désiré.

**C.** (2-Butyl-3-(2-[4-(2-dibutylamino-ethoxy)phenyl]-[1,3]dioxolan-2-yl)-benzofuran-5-yl)-methanol

On mélange 3,25 g du composé obtenu à l'étape précédente, 2,64 g de dibutylamine, 1,02 g d'iodure de sodium, 2,83 g de carbonate de potassium et 50 ml d'acétonitrile que l'on chauffe à reflux pendant 3 heures. On évapore à sec, on reprend le résidu par de l'acétate d'éthyle et on lave à l'eau, une solution aqueuse de NaCl. On sèche sur $Na_2SO_4$. On purifie par chromatographie sur silice (éluant : DCM/méthanol à 95/5).

De cette manière, on obtient 1,84 g du composé désiré.

**D**. Oxalate de 2-Butyl-3-(2-[4-(2-dibutylamino-ethoxy)-phenyl]-[1,3]dioxolan-2-yl)-benzofuran-5-yl)-methanol

On mélange 1,84 g du composé obtenu à l'étape C précédente et 313 mg d'acide oxalique dans 20 ml de méthanol. On évapore. Après trituration dans l'éther, on filtre la poudre sur fritté. On sèche sous vide.

De cette manière, on obtient 1,7 g du composé désiré.

Spectre RMN : conforme ; voir ci-dessous

**EXEMPLE 155**

**Oxalate de 2-butyl-3-[4-(3-dibutylamino-2-hyrdroxy-propoxy)-benzoyl]-benzofuran-5-carboxylate d'isopropyle**

**A.** 2-Butyl-3-(4-oxiranylmethoxy-benzoyl)-benzofuran-5-carboxylate d'isopronyle

On porte à reflux pendant 1 heure un mélange de 3,15 g de 2-butyl-3-(4-hydroxy-benzoyl)-benzofuran-5-carboxylate d'isopropyle, 25 ml d'isopropanol, 15 ml d'épibromhydrine, et 365 mg (1,1 equivalent) de NaOH. On évapore, reprend le résidu par de l'eau et on extrait 3 fois à l'acétate d'éthyle. On lave à l'eau puis avec une solution saturée de NaCl. On sèche sur $Na_2SO_4$ et on concentre. On purifie par chromatographie sur silice (éluant : acétate d'éthyle/dichlorométhane).

De cette manière, on obtient 3,15 g du composé désiré.

**B**. 2-Butyl-3-[4-(3-dibutylamino-2-hydroxy-propoxy)-benzoyl]-benzofuran-5-carboxylate d'isopropyle

On mélange 3,15 g du composé obtenu à l'étape A précédente, 3,15 g de dibutylamine et 20 ml d'acétonitrile, et on porte au reflux pendant 16 heures. On évapore le solvant et on purifie par chromatographie sur silice (éluant : acétate d'éthyle/dichlorométhane puis méthanolldichlorométhane).

De cette manière, on obtient 3,6 g du composé désiré.

**C.** Oxalate de 2-butyl-3-[4-(3-dibutylamino-2-hydroxy-propoxyl-benzoyl]-benzofuran-5-carboxylate d'isopropyle

On mélange 3,6 g du composé obtenu à l'étape B précédente et 585 mg d'acide oxalique dans du méthanol. On évapore. Après trituration dans l'éther, on filtre la poudre sur fritté. On sèche sous vide.

De cette manière, on obtient 2,1 g du composé désiré.

P.F. : 87-88˚C

Spectre RMN : conforme

**EXEMPLE 158**

**Chlorhydrate de 2-Butyl-3-[4-(3-piperidin-1-yl-propoxy)-benzoyl]-benzofuran-5-carboxylate de 2-piperidin-1-yl-éthyle**

**A.** 2-Butyl-3-[4-(3-pioeridin-1-yl-propoxy)-benzoyl]-benzofuran-5-carboxylate de 2-piperidin-1-yl-éthyle

On dissout 7,5 g (0,016 moles) d'acide 2-Butyl-3-[4-(3-piperidin-1-yl-propoxy)-benzoyl]-benzofuran-5-carboxilique dans 50 ml $CHCl_3$. On ajoute 15 ml de $SOCl_2$. On porte au reflux pendant 3 heures. On évapore et on reprend trois fois dans l'éther. On reprend dans 50 ml de DCM. On ajoute 2,34 ml de 2-(N-piperidyl)-1-éthanol (2,27 g ; 0,0176 moles) et on agite 24 heures à température ambiante. On évapore et reprend le résidu à l'acétate d'éthyle et on lave à l'eau, une solution de NaOH, $H_2O$ et une solution de NaCl. On purifie par chromatographie sur silice (éluant : DCM/Méthanal/$NH_4OH$ : 92/810,5).

De cette manière, on obtient 2,32 g du composé désiré.

Rendement : 25 %.

**B.** Chlorhydrate de 2-Butyl-3-[4-(3-piperidin-1-yl-propoxy)-benzoyl]-benzofuran-5-carboxylate de 2-piperidin-1-yl-éthyle

On dissout 2,3 g (0,004 moles) du composé obtenu à l'étape A précédente, dans un mélange d'éther et d'acétate d'éthyle. On ajoute une solution d'acide chlorhydrique dissout dans de l'éther. On évapore, on reprend dans l'éther et on filtre.

De cette manière, on obtient 2,02 g du composé désiré.
Rendement : 78 %.

En utilisant les procédés décrits aux exemples précédents, on a préparé les composés listés ci-après. Pour chacun de ces composés, les spectres RMN se sont révélés conformes aux structures décrites.

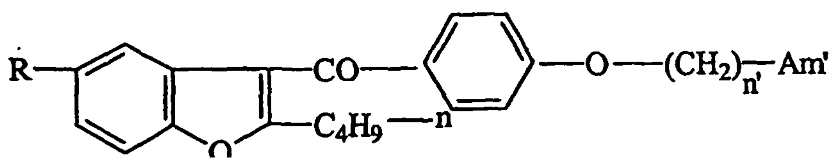

| R | Am' | n' | Caractéristiques | Ex. |
|---|---|---|---|---|
| 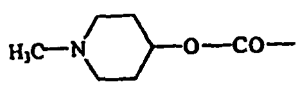 | $-N(C_4H_9)_2$ | 3 | Oxalate | 33 |
| $H_5C_2$-O-CO- | 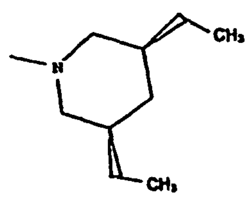 | 2 | Chlorhydrate P.F. : 133°C | 34 |
| $H_5C_2$O-CO- | 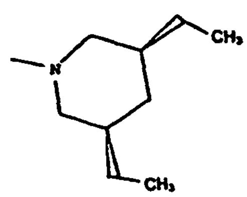 | 3 | Chlorhydrate P.F. : 164°C | 35 |
| 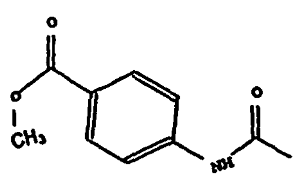 | $-N(C_4H_9)_2$ | 3 | Oxalate | 36 |
| $H_5C_2$-NH-CO- | $-N(C_4H_9)_2$ | 3 | Oxalate P. F. : 85°C | 37 |
| neo-$H_{11}C_5$-O-CO- | $-N(C_4H_9)_2$ | 3 | Oxalate P.F. : 102°C | 38 |

(suite)

| R | Am' | n' | Caractéristiques | Ex. |
|---|---|---|---|---|
| 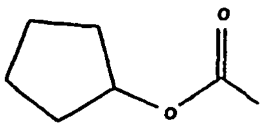 | $-N(C_4H_9)_2$ | 3 | Oxalate P.F. : 99˚C | 39 |
| $i-H_9C_4-O-CO-$ | $-N(C_4H_9)_2$ | 3 | Oxalate P.F. : 98˚C | 40 |
| $(i-H_7C_3)_2-CH-CO-$ | 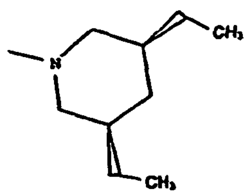 | 3 | Oxalate P.F. : 105˚C | 41 |
| $t-H_9C_4-CO_2-$ | 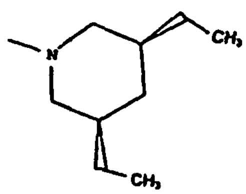 | 3 | Oxalate P.F. : 166˚C | 42 |
| 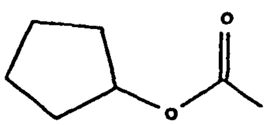 | | 3 | Oxalate (solide blanc amorphe) | 43 |
| $n-H_7C_3-CO_2-$ | $-N(C_4H_9)_2$ | 3 | Oxalate (solide amorphe) | 44 |
| 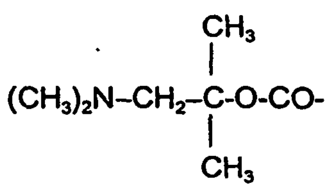 | $-N(C_4H_9)_2$ | 3 | Oxalate (solide amorphe) | 45 |
| $(CH_3)_2N-(CH_2)_{27}NH-CO-$ | $-N(C_4H_9)_2$ | 3 | Oxalate | 46 |
| $H_5C_2-CO_2-$ | 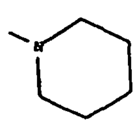 | 3 | Chlorhydrate P.F. : 161-163˚C | 47 |
| $H_5C_2-CO_2-$ | 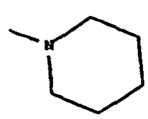 | 2 | Chlorhydrate P.F. : 147-1503˚C | 48 |

(suite)

| R | Am' | n' | Caractéristiques | Ex. |
|---|---|---|---|---|
| $(CH_3)_2$ N-$(CH_2)_2CO_2$- | 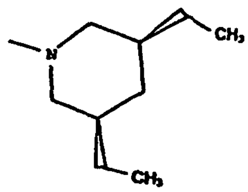 | 3 | Dioxalate (solide) | 49 |
| $(CH_3)_2$ N-$(CH_2)_2$-$CO_2$- | 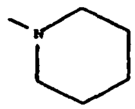 | 3 | Dichlorhydrate (solide) | 50 |
| $CH_3O_2C$-$(CH_2)_2$-NH-CO- | -$N(C_4H_9)_2$ | 3 | Oxalate (solide amorphe) | 51 |
| $HOCH_2$- | 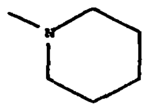 | 2 | Oxalate (solide amorphe) | 52 |
| $HOCH_2$- | 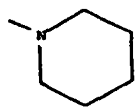 | 3 | Chlorhydrate P.F. : 173-175˚C | 53 |
| 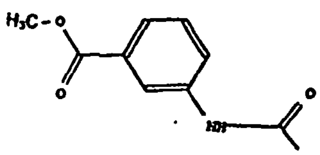 | -$N(C_4H_9)_2$ | 3 | Oxalate P.F. : 80˚C | 54 |
| 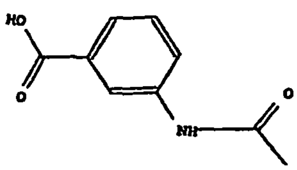 | -$N(C_4H_9)_2$ | 3 | Base P.F. : 130˚C | 55 |
| 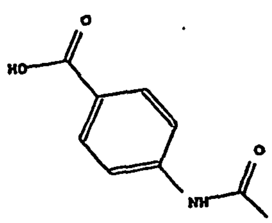 | -$N(C_4H_9)_2$ | 3 | Chlorhydrate P.F. : 142˚C | 56 |
| 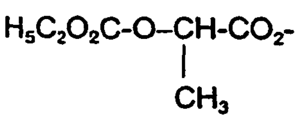 | -$N(C_4H_9)_2$ | 3 | Oxalate P.F. : 86˚C | 57 |

(suite)

| R | Am' | n' | Caractéristiques | Ex. |
|---|---|---|---|---|
| 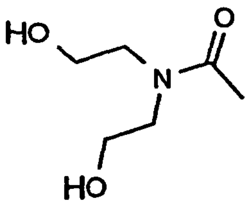 | $-N(C_4H_9)_2$ | 3 | Solide P.F. : 89-91 °C | 150 |
| $H_5C_2-CO_2-$ | 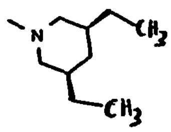 | 2 | Chlorhydrate Solide P.F. : 133-134°C | 156 |
| 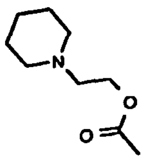 | 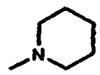 | 3 | Chlorhydrate Solide | 158 |
| HOOC- | 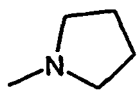 | 3 | Chlorhydrate Amorphe | 162 |

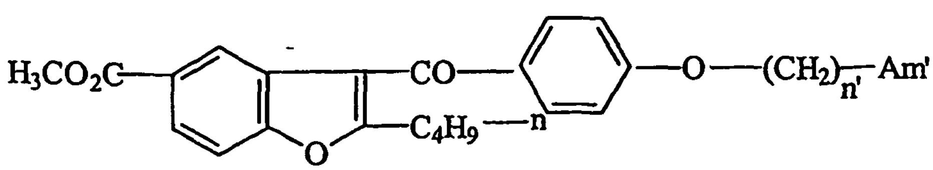

| Am' | n' | Caractéristiques | Ex. |
|---|---|---|---|
| $-N(C_4H_9-n)_2$ | 2 | Oxalate P.F. : 88°C | 58 |
| 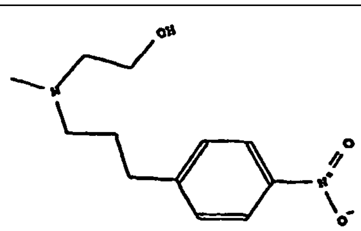 | 2 | Oxalate P.F. : 124°C | 59 |
| 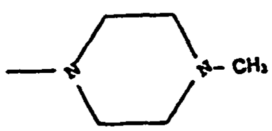 | 3 | Base P.F. : 66-68°C | 60 |

(suite)

| Am' | n' | Caractéristiques | Ex. |
|---|---|---|---|
| 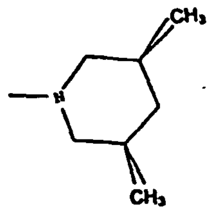 | 2 | Chlorhydrate P.F. : 153°C | 61 |
| 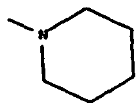 | 2 | Chlorhydrate P.F. : 122°C | 62 |
| 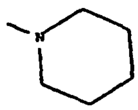 | 3 | Chlorhydrate P.F. : 147°C | 63 |
| $-N(C_2H_5)_2$ | 2 | Chlorhydrate P.F. : 112°C | 64 |
| $-N(C_2H_5)_2$ | 3 | Chlorhydrate P.F. : 105°C | 65 |
| 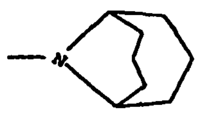 | 2 | Oxalate P.F.: 185°C | 66 |
| 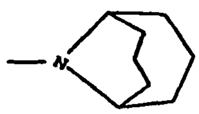 | 3 | Oxalate P.F.: 142°C | 67 |
| 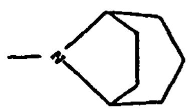 | 3 | Oxalate P.F. : 155°C | 68 |
| 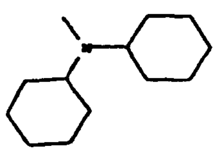 | 3 | Oxalate (poudre amorphe) | 69 |
| 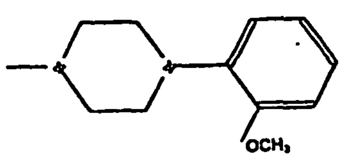 | 3 | Oxalate P.F. : 116°C | 70 |

(suite)

| Am' | n' | Caractéristiques | Ex. |
|---|---|---|---|
| 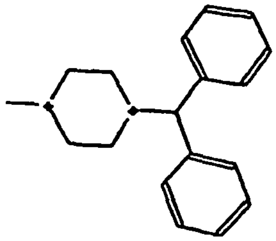 | 2 | Base P.F. : 124°C | 71 |
| 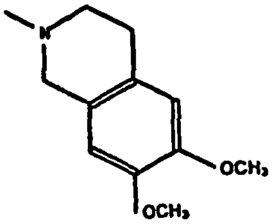 | 3 | Oxalate P.F. : 159°C | 72 |
| 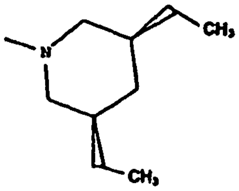 | 3 | Chlorhydrate P.F. : 135°C | 73 |
| 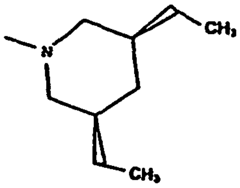 | 2 | Chlorhydrate P.F. : 161°C | 74 |
| 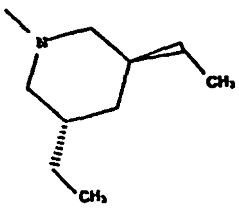 | 2 | Chlorhydrate P.F. : 133°C | 75 |
| 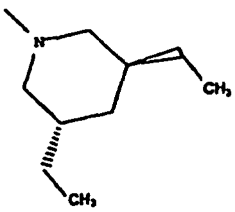 | 3 | Oxalate (poudre amorphe) | 76 |
| 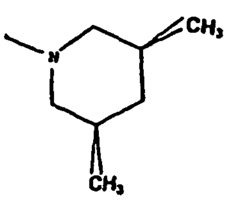 | 3 | Chlorhydrate P.F. : 141°C | 77 |

(suite)

| Am' | n' | Caractéristiques | Ex. |
|---|---|---|---|
| 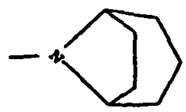 | 3 | Oxalate (solide) | 78 |
| 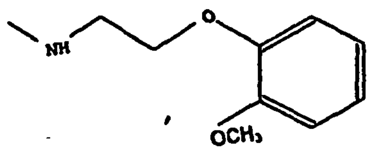 | 3 | Oxalate P.F. : 150-152°C | 79 |
| 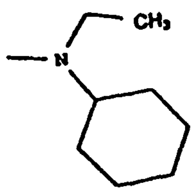 | 3 | Oxalate (solide) | 80 |
| 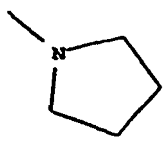 | 2 | Chlorhydrate P.F. : 108-110°C | 81 |
| 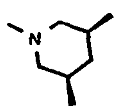 | 2 | Chlorhydrate Solide P.F. : 153-156°C | 157 |
| 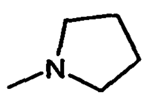 | 3 | Chlorhydrate Solide P.F. : 131-133°C | 159 |

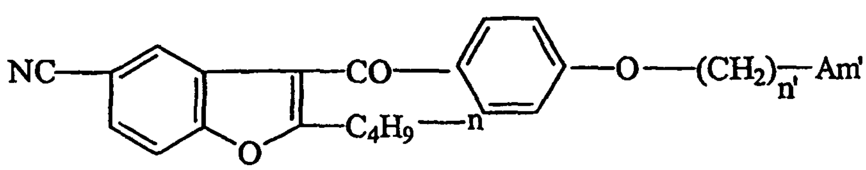

| Am' | n' | Caractéristiques | Ex. |
|---|---|---|---|
| 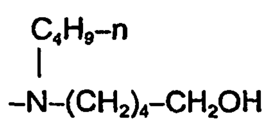 | 3 | Oxalate (solide amorphe) | 82 |
| -$NHC_4H_9$-n | 3 | Chlorhydrate P.F. : 176°C | 83 |

(suite)

| Am’ | n’ | Caractéristiques | Ex. |
|---|---|---|---|
| 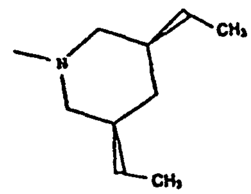 | 2 | Oxalate P. F. : 148˚C | 84 |
| 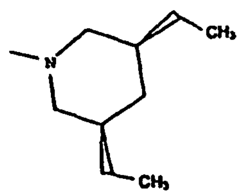 | 3 | Oxalate P.F. : 161 ˚C | 85 |
| 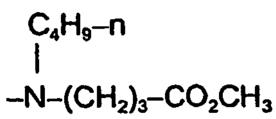 | 3 | Oxalate (solide amorphe) | 86 |

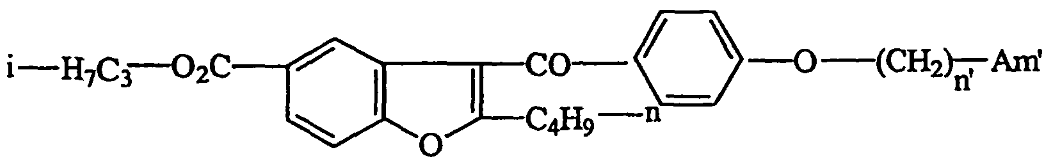

| Am’ | n’ | Caractéristiques | Ex. |
|---|---|---|---|
| $-N(C_4H_9-n)_2$ | 3 | Oxalate P.F. : 94˚C | 87 |
| 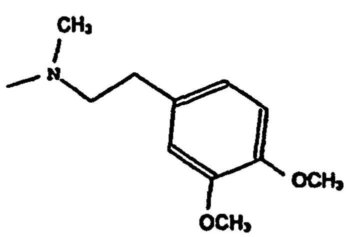 | 3 | Oxalate P.F. : 85˚C | 88 |
| 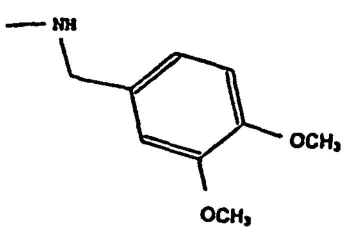 | 3 | Chlorhydrate (solide amorphe) | 89 |

(suite)

| Am' | n' | Caractéristiques | Ex. |
|---|---|---|---|
| 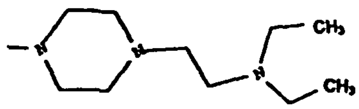 | 3 | Trichlorhydrate P.F.:242°C | 90 |
| 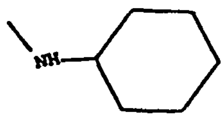 | 3 | Oxalate P. F. : 163°C | 91 |
| 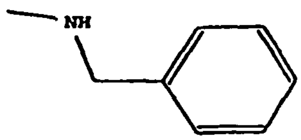 | 3 | Chlorhydrate (solide amorphe) | 92 |
| 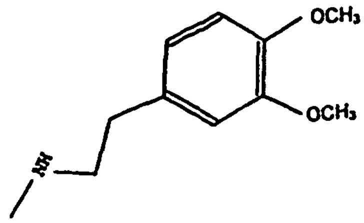 | 3 | Chlorhydrate (solide amorphe) | 93 |
| 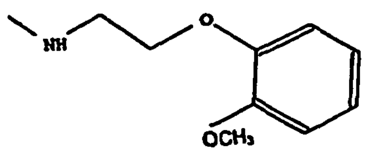 | 3 | Chlorhydrate P.F. : 116°C | 94 |
| 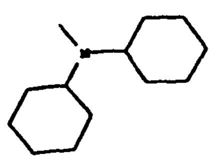 | 2 | Oxalate P.F.:71°C (poudre amorphe) | 95 |
| 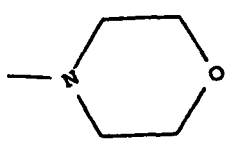 | 3 | Base P.F. 94°C | 96 |
| 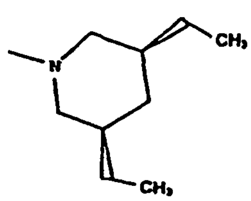 | 5 | Oxalate P.F. : 120°C | 97 |

(suite)

| Am' | n' | Caractéristiques | Ex. |
|---|---|---|---|
| 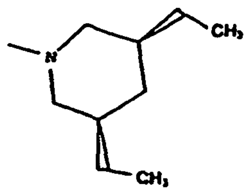 | 4 | Oxalate P.F. : 121°C | 98 |
| 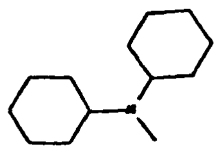 | 3 | Chlorhydrate P.F. : 129°C | 99 |
| 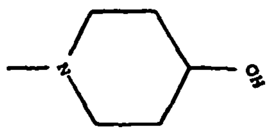 | 3 | Oxalate solide blanc amorphe) | 100 |
| 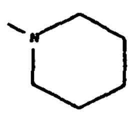 | 3 | Chlorhydrate P.F. : 158°C | 101 |
| $-NH-C_4H_9-n$ | 2 | Oxalate P.F. : 188°C | 102 |
| $-NH-C_4H_9-n$ | 3 | Oxalate P.F. : 170°C | 103 |
| $-N(C_4H_9-n)_2$ | 2 | Oxalate (solide blanc amorphe) | 104 |
| 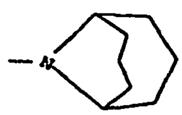 | 2 | Oxalate P.F.: 86°C | 105 |
| 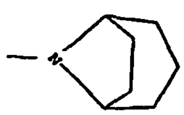 | 2 | Chlorhydrate P. F. : 89°C | 106 |
| 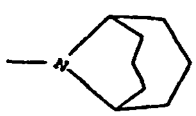 | 3 | Chlorhydrate P.F. : 179°C | 107 |
| 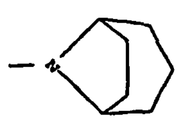 | 3 | Oxalate P.F. : 150°C | 108 |

(suite)

| Am' | n' | Caractéristiques | Ex. |
|---|---|---|---|
| 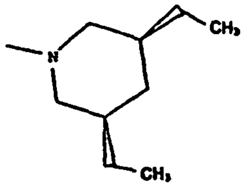 | 2 | Chlorhydrate P.F. : 156°C | 109 |
| 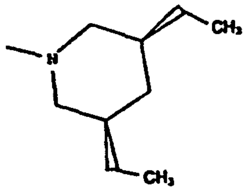 | 3 | Oxalate P.F. : 127°C | 110 |
| 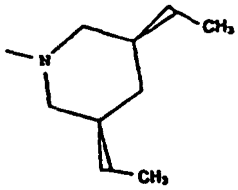 | 3 | Chlorhydrate P.F.:86°C | 111 |
| 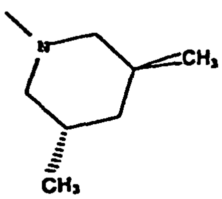 | 3 | Chlorhydrate P.F.: 161°C | 112 |
| 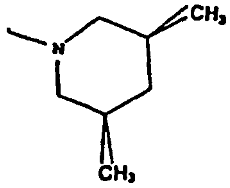 | 3 | Chlorhydrate P.F. : 172°C | 113 |
| 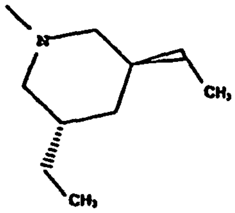 | 3 | Chlorhydrate P. F. : 146°C | 114 |
| 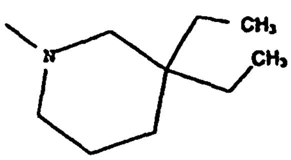 | 3 | Chlorhydrate P.F.: 154°C | 115 |

(suite)

| Am' | n' | Caractéristiques | Ex. |
|---|---|---|---|
| 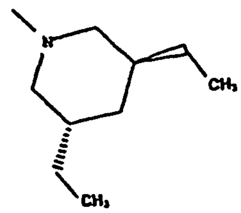 | 3 | Chlorhydrate P.F. : 117°C | 116 |
| 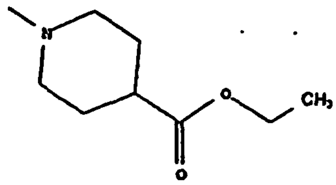 | 3 | Chlorhydrate (solide) | 117 |

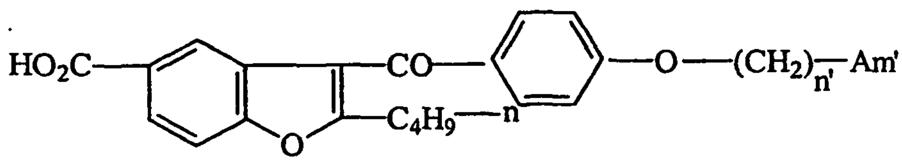

| **Am'** | **n'** | **Caractéristiques** | **Ex.** |
|---|---|---|---|
| 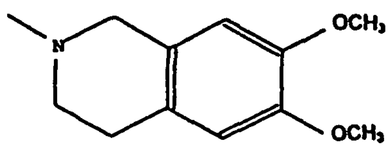 | 3 | Base (solide amorphe) | 118 |
| 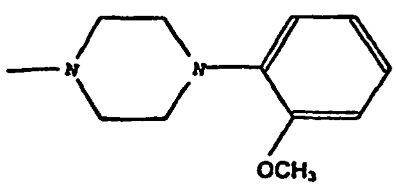 | 3 | Base P.F. : 108°C | 119 |
| 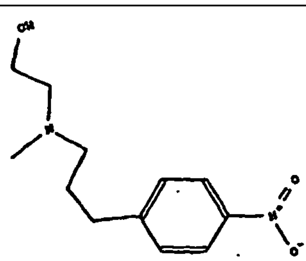 | 2 | Base P.F. : 80°C | 120 |

(suite)

| Am' | n' | Caractéristiques | Ex. |
|---|---|---|---|
| 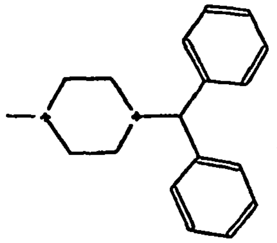 | 2 | Base (solide blanc amorphe) | 121 |
| 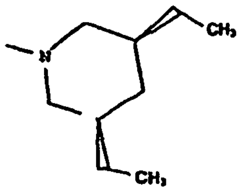 | 2 | Base (solide blanc amorphe) | 122 |
| 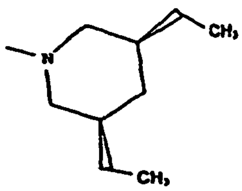 | 3 | Base (solide blanc amorphe) | 123 |
| 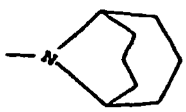 | 2 | Chlorhydrate (solide) | 124 |
| 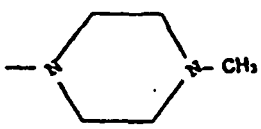 | 3 | Base P.F. : 95°C | 125 |
| 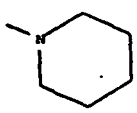 | 2 | Base (solide amorphe) | 126 |
| 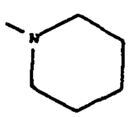 | 3 | Base (solide amorphe) | 127 |
| $-N(C_2H_5)_2$ | 2 | Base P.F. : 73°C | 128 |
| 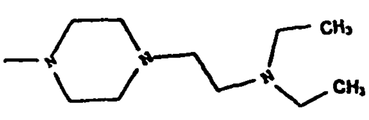 | 3 | Trichlorhydrate P.F. : >200°C (dégradation) | 129 |

(suite)

| Am' | n' | Caractéristiques | Ex. |
|---|---|---|---|
| 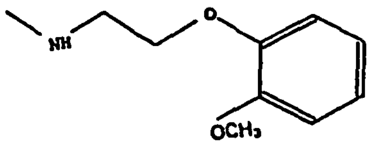 | 3 | Chlorhydrate P.F. : 156°C | 130 |
| 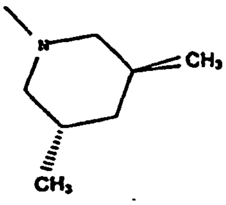 | 3 | Base (poudre amorphe) | 131 |
| 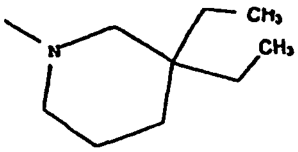 | 3 | Chlorhydrate P.F. : 106°C | 132 |
| 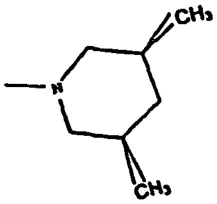 | 3 | Chlorhydrate P.F. : 116°C | 133 |
| 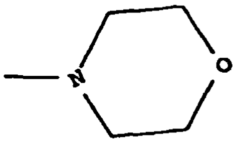 | 3 | Base P.F. : 85°C | 134 |
| 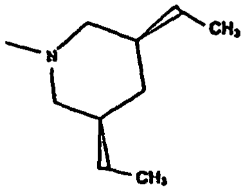 | 5 | Base P.F.: 167°C | 135 |
| 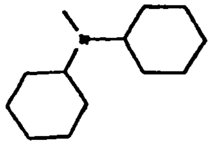 | 2 | Chlorhydrate P.F. : 173°C | 136 |

(suite)

| Am' | n' | Caractéristiques | Ex. |
|---|---|---|---|
| 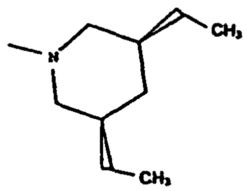 | 4 | Base P.F. : 100°C | 137 |
| 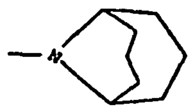 | 3 | Chlorhydrate (solide) | 138 |
| 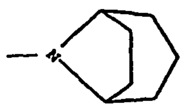 | 3 | Chlorhydrate P.F. : 191°C | 139 |
| 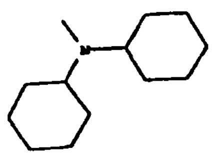 | 3 | Chlorhydrate (poudre amorphe) | 140 |
| 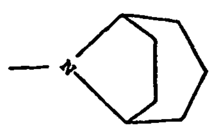 | 2 | Chlorhydrate (solide) | 141 |
| $-N(C_4H_9)_2$ | 2 | Base (solide amorphe) | 142 |
| $-N(C_4H_9)_2$ | 3 | Chlorhydrate (poudre amorphe) | 143 |
| 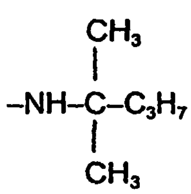 | 3 | Chlorhydrate (poudre amorphe) | 144 |
| $-N(C_2H_5)_2$ | 3 | Base P.F. : 83°C | 145 |
| 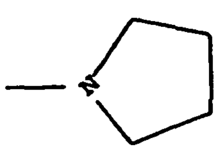 | 2 | Chlorhydrate P.F. : 188-190°C | 146 |
| $-NH-C_5H_9$-neo | 3 | Base P.F. :188°C | 147 |

(suite)

| Am' | n' | Caractéristiques | Ex. |
|---|---|---|---|
| 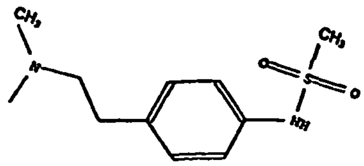 | 2 | Base (solide amorphe) | 148 |

On a également préparé les composés listés ci-après.

| Composé | Caractéristiques | Exemple |
|---|---|---|
| 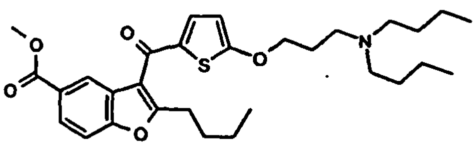 | Oxalate Solide P.F. : 130°C | 152 |
| 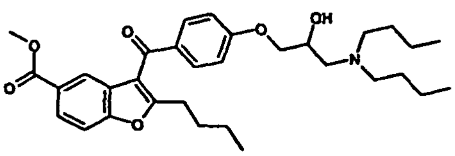 | Huile | 153 |
| 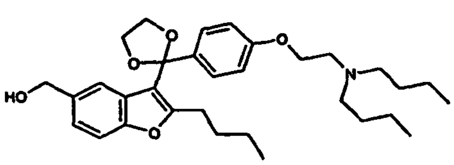 | Oxalate Glace (spectre RMN ci-dessous) | 154 |
| 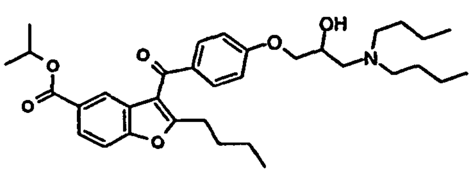 | Oxalate Poudre P.F. : 87-88°C | 155 |

**EXEMPLE 149**

Selon des techniques pharmaceutiques connues, on a préparé une capsule contenant les ingrédients suivants :

| Ingrédient | Mg |
|---|---|
| Composé de l'invention | 100,0 |
| Amidon | 99,5 |
| Silice colloïdale | 0,5 |

**Spectres R.M.N. $^1$H à 200 MHz**

Exemple 153

Solvant : DMSO
δ (ppm) : 6.9-8.2 (massif, 7H) ; 4.85 (singulet large, 1 H) ; 3.7-4.3 (massif, 6H) ; 2.2-3.0 (massif, 8H) ; 0.6-1.8 (massif, 21 H)

Exemple 154

Solvant : DMSO
δ (ppm) : 6.7-7.5 (massif, 7H) ; 4.45 (singulet, 2H) ; 3.7-4.3 (massif, 6H) ; 3.2-3.5 (massif, 2H) ; 2.95 (doublet dédoublé large, 4H) ; 2.80 (triplet, 2H) ; 0.7-3.3 (massif, 21H)

Exemple 158

Solvant : DMSO
δ (ppm) : 6.9-8.2 (massif, 7H) ; 2.6-4.8 (massif, 16H) ; 0.9-2.3 (massif, 18H) ; 0.78 (triplet, 3H)

Exemple 162

Solvant : DMSO
δ (ppm) : 6.9-8.2 (massif, 7H) ; 3.0-4.4 (massif, 8H) ; 2.80 (triplet, 2H) ; 1.0-2.3 (massif, 10H) ; 0.78 (triplet, 3H)

## Revendications

**1.** Dérivés de benzofurane ou de benzothiophène de formate générale :

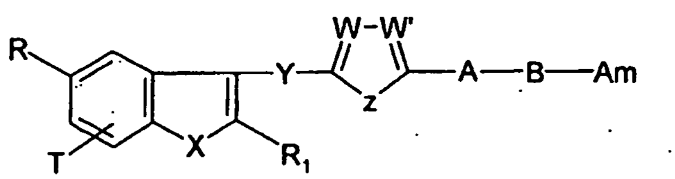

(1)

ainsi que leurs sels pharmaceutiquement acceptables, dans laquelle :

A représente -O- ou -S-
B représente un groupement alkylène, linéaire ou ramifié, en $C_1$-$C_5$, éventuellement substitué par un groupement hydroxyle,
T représente l'hydrogène
R représente :

- le groupement cyano ou hydroxyméthyle
- un groupement oxime de formule :

$R_4$-O-N=CH-

dans laquelle $R_4$ représente un groupement alkyle en $C_1$-$C_4$
- un groupement carboxyle de formule générale :

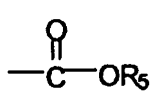

(a)

dans laquelle $R_5$ représente l'hydrogène ou un atome de métal alcalin, un groupement alkyle en $C_1$-$C_{10}$ ,linéaire ou ramifié, un groupement cycloalkyle en $C_3$-$C_6$, ou $R_5$ représente le groupement de formule générale :

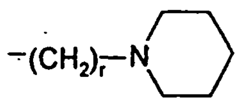

(a-1)

dans laquelle r représente 1 à 4

• un groupement carboxyle de formule générale :

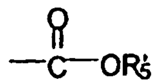

(b)

dans laquelle $R'_5$ représente un groupement pipéridinyle éventuellement N-substitué par un groupement alkyle en $C_1$-$C_4$ ou l'un des groupements de formule générale :

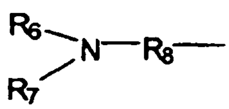

ou

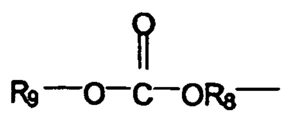

(c) (d)

dans lequel $R_6$ et $R_7$, identiques ou différents, représentent un groupement alkyle en $C_1$-$C_4$, $R_8$ représente un groupement alkylène, linéaire ou ramifié, en $C_1$-$C_6$, et $R_9$ représente l'hydrogène, un atome de métal alcalin, un groupement alkyle en $C_1$-$C_4$.

• un groupement aminocarbonyle de formule:

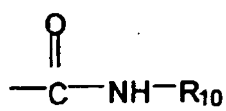

(e)

dans lequel $R_{10}$ représente l'hydrogène, un groupement alkyle en $C_1$-$C_4$, hydroxyle ou amino, un groupement (c) ci-dessus ou l'un des groupements :

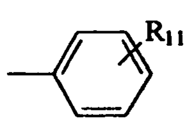

ou

$-R_{12}-R_{11}$

(f) (g)

dans lequel $R_{11}$ représente un groupement (a) et $R_{12}$ représente un radical alkylène en $C_1$-$C_8$.,

• un groupement de formule:

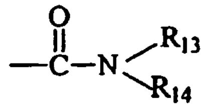

(h)

dans laquelle $R_{13}$ et $R_{14}$, identiques ou différents, représentent un radical alkyle en $C_1$-$C_4$, ou un groupement hydroxyalkyle en $C_1$-$C_4$

• l'un des groupements de formule ci-dessous :

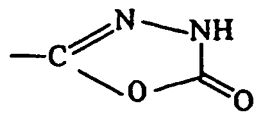

ou

(j)

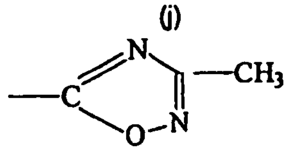

ou

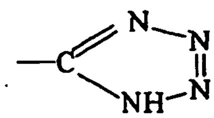

(k) (l)

$R_1$ représente un groupement alkyle en $C_1$-$C_6$ ou un groupement de formule :

$- (CH_2)_p-R_{11}$ (m)

dans laquelle $R_{11}$ a la même signification que précédemment et p représente 1à4
Am représente un groupement azoté de formule :

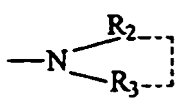

ou

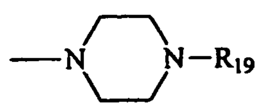

ou

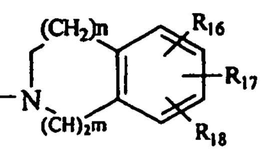

($Am_1$) ($Am_2$) ($Am_3$)

dans laquelle :

$R_2$ représente l'hydrogène, un groupement alkyle en $C_1$-$C_6$, linéaire ou ramifié. éventuellement substitué par un groupement hydroxyle, un groupement (m), un groupement cycloalkyle en $C_3$-$C_6$ ou un groupement benzyle, $R_3$ représente un groupement alkyle en $C_1$-$C_6$, linéaire ou ramifié. éventuellement substitué par un groupement hydroxyle, un groupement cycloalkyle en $C_3$-$C_6$. un groupement (m), un groupement benzyle ou un groupement phényle de formule:

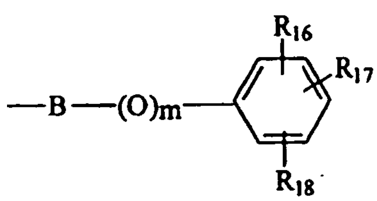

(n)

$R_{16}$, $R_{17}$ et $R_{18}$, identiques ou différents, représentent l'hydrogène, un groupement hydroxyle, nitro, amino, alkoxy en $C_1$-$C_4$ ou alkylsulfonamido en $C_1$-$C_4$.,
$R_{18}$ représente l'hydrogène, un groupement alkyle en $C_1$-$C_5$, le groupement diphénylméthyle, un groupement mono-, di- ou triméthylphényle, un groupement mono-, di- ou triméthoxyphényle, un groupement (a), un groupement (b) ou un groupement (c),
m et n représentent chacun 0 ou 1,
$R_2$ et $R_3$, lorsqu'ils sont pris ensemble, représentent un groupement alkylène en $C_3$-$C_{10}$, linéaire ou ramifié, éventuellement substitué par le groupement hydroxyle, par un groupement (a) ou par un groupement (m) et éventuellement interrompu par -O-,
W, W' et Z sont tels que :

- lorsque W et W', identiques représentent CH, Z représente -O- ou -S-
- lorsque W représente CH et W' représente C-$R_{20}$, Z représente

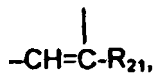

$R_{20}$ et $R_{21}$, étant identiques ou différents et représentant l'hydrogène, un atome d'halogène, un radical alkyle en $C_1$-$C_4$ ou un radical alkoxy en $C_1$-$C_4$
X représente -O- ou -S-
Y représente un radical - CO -, - $CH_2$-, un radical de formule

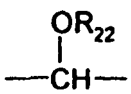 (73)

dans laquelle $R_{22}$ représente l'hydrogène, un radical alkyle en $C_1$-$C_4$ ou acyle de formule:

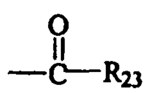 (74)

dans laquelle $R_{23}$ représente un radical alkyle en $C_1$-$C_4$,
ou Y représente

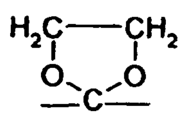

étant entendu que l'ensemble formé par les groupements R, $R_1$ et Am contient 0, 1 ou 2 groupements (a),
ces dérivés de benzofuranne ou de benzothiophène étant sous forme d'isomères individuels ou de mélange de ceux-ci.

**2.** Dérivés de benzofurane ou de benzothiophène selon la Revendication 1 dans laquelle Y représente un radical - CO - ou Y représente

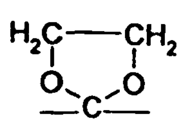

**3.** Dérivés de benzofurane ou de benzothiophène selon la Revendication 1 ou 2 dans laquelle R représente le groupement isopropoxy carbonyle.

**4.** Dérivés de benzofurane ou de benzothiophène selon une des Revendications 1 à 3 dans laquelle Am représente un groupement diéthylpipéridinyle.

**5.** Dérivés de benzofurane ou de benzothiophène selon un des Revendications 1 à 4, dans laquelle Y représente le radical - CO - .

**6.** Dérivés de benzofurane ou de benzothiophène selon une des Revendications 1 à 5, dans laquelle

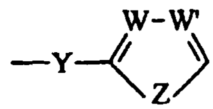

représente le radical benzoyle.

**7.** Dérivés de benzofurane ou de benzothiophène selon une des Revendications 1 à 6, dans laquelle :

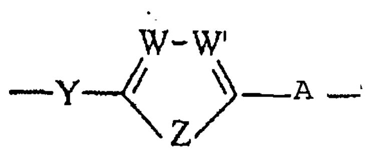

représente le radical 4- oxybenzoyle.

**8.** Dérivés de benzofurane ou de benzothiophène selon une des Revendications 1 à 7 dans laquelle la chaîne :

A-B-Am

se trouve en position 4.

**9.** Dérivés de benzofurane ou de benzothiophène selon une des Revendications 1 à 8 dans laquelle $R_1$ représente n-butyle, B représente le groupement propylène et Am représente un groupement diéthylpipéridinyle.

**10.** Dérivés de benzofurane ou de benzothiophène selon la Revendication 9 dans laquelle le groupement diéthylpipéridinyle est le groupement 3,5-diéthylpipéridinyle.

**11.** Dérivés de benzofurane ou de benzothiophène selon une des Revendications 1 à 10 dans lesquels le sel pharmaceutiquement acceptable est choisi parmi les oxalate, maléate, fumarate, méthanesulfonate, benzoate, ascorbate, pamoate, succinate, hexamate, bisméthylènesalicylate, éthanedisulfonate, acétate, propionate, tartrate, salicylate, citrate, gluconate, lactate, malate, cinnamate, mandélate, citraconate, aspartate, palmitate, stéarate, itaconate, glycolate, p-aminobenzoate, glutamate, benzènesulfonate, p-toluènesulfonate et théophilline acétate ainsi que les sels formés à partir d'un acide aminé tel que le sel de lysine ou l'histidine.

**12.** Dérivés de benzofurane ou de benzothiophène selon l'une quelconque des revendications 1 à 10, dans lesquels le sel pharmaceutiquement acceptable est choisi parmi les chlorhydrate, bromhydrate, sulfate, sulfamate, phosphate et nitrate.

**13.** Procédé de préparation de dérivés de benzofurane ou de benzothiophène selon la Revendication 1 dans laquelle Y représente le groupement -CO-, $R_1$, ($Am_1$) et ($Am_2$) ne comportent pas de groupement carboxylique ou carboxylate de métal alcalin, $R_{16}$ et / ou $R_{17}$ et / ou $R_{18}$ sont différents du groupement amino ou d'un groupement alkylsulfonamido en $C_1$-$C_4$ et R représente un groupement cyano ou hydroxyméthyle, le groupement (k) ou un groupement (a) dans lequel $R_5$ représente un groupement alkyle en $C_1$-$C_{10}$ ou cycloalkyle en $C_3$-$C_8$, **caractérisé en ce que** l'on fait

réagir, en présence d'un agent basique, un dérivé cétonique de formule générale :

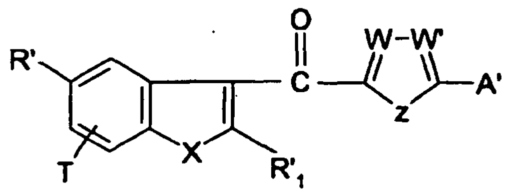

(2)

dans laquelle $R'_1$ représente un groupement alkyle en $C_1$-$C_6$ ou un groupement (m) ne comportant pas de groupement carboxylique ou carboxylate de métal alcalin, T, W, W', X et Z ont la même signification que dans la Revendication 1, A' représente OH, SH ou $NH_2$ et R' représente un groupement cyano ou hydroxyméthyle, le groupement (k) ou un groupement $-CO_2\ R''_5$ dans lequel $R''_5$ représente un radical alkyle en $C_1$-$C_{10}$ ou cycloalkyle en $C_3$-$C_6$, avec un composé de formule générale :

$R_{24}$-B-Am' (3)

dans laquelle Am' représente un groupement ($Am_1$) ou ($Am_2$) tel que défini dans la Revendication 1, ce groupement ne comportant ne comportant pas de groupement carboxylique ou carboxylate de métal alcalin ou encore Am' représente un groupement ($Am_3$) tel que défini dans la Revendication 1 dans lequel $R_{16}$ et/ou $R_{17}$ et/ou $R_{18}$ sont autres qu'un groupement amino ou un groupement alkylsulfonamido en $C_1$-$C_4$. B a la même signification que dans la Revendication 1 et $R_{24}$ représente :

- un atome d'halogène, un radical alkylsulfonyloxy en $C_1$-$C_4$ ou arylsulfonyloxy en $C_6$-$C_{10}$, ce qui permet d'obtenir, sous forme de base libre, les composés désirés dans lesquelles A représente -0- ou -S-, les composés sous forme de base libre ainsi obtenus pouvant, si nécessaire, être traités par un acide organique ou inorganique approprié pour former un sel pharmaceutiquement acceptable.

**14.** Procédé de préparation de dérivés de benzofurane ou de benzothiophène selon la Revendication 1 dans laquelle Y représente le groupement -CO-, $R_1$, ($Am_1$) et ($Am_2$) ne comportent pas le groupement carboxylique ou carboxylate de métal alcalin, $R_{16}$ et/ou $R_{17}$ et/ou $R_{18}$ sont différents du groupement amino ou d'un groupement alkylsulfonamido en $C_1$-$C_4$ et R représente le groupement cyano, le groupement (k), un groupement $R_4$-O-N=CH-ou un groupement (a) dans lequel $R_5$ représente un groupement alkyle en $C_1$-$C_{10}$ ou cycloalkyle en $C_3$-$C_6$. **caractérisé en ce que** l'on fait réagir un composé de formule générale :

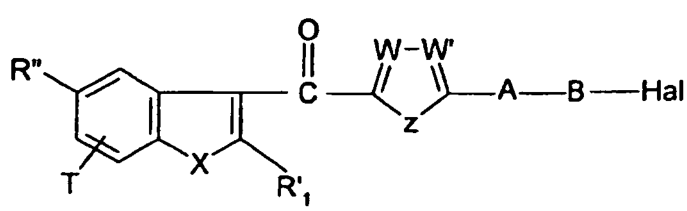

(4)

dans laquelle A, B, T, X, W, W' et Z ont la même signification que dans la Revendication 1, $R'_1$ représente un groupement alkyle en $C_1$-$C_6$ ou un groupement (m) ne comportant pas de groupement carboxylique ou carboxylate de métal alcalin et R" représente le groupement cyano, le groupement (k), un groupement $R_4$-O-N=CH- ou un groupement $-CO_2R''_5$ dans lequel $R''_5$ représente un groupement alkyle en $C_1$-$C_{10}$ ou cycloalkyle en $C_3$-$C_6$ et Hal représente un atome d'halogène, avec un composé de formule générale :

H-Am' (5)

éventuellement sous forme de sel, dans laquelle Am' représente un groupement ($Am_1$) ou ($Am_2$) tel que défini dans la Revendication 1, ce groupement ne comportant pas de groupement carboxylique ou carboxylate de métal alcalin ou encore Am' représente un groupement ($Am_3$) tel que défini dans la Revendication 1 dans lequel $R_{16}$ et/ou $R_{17}$ et/ou $R_{18}$ sont autres qu'un groupement amino ou un groupement alkylsulfonamido en $C_1$-$C_4$, la réaction ayant lieu en présence d'un agent basique ou un excès d'amine de formule (5) sous forme basique, ce qui fournit les composés

désirés sous forme de base libre que l'on peut faire réagir, si nécessaire, avec un acide organique ou inorganique approprié pour former un sel pharmaceutiquement acceptable.

**15.** Procédé de préparation de dérivés de benzofurane ou de benzothiophène selon la Revendication 1 dans laquelle Y représente le groupement -CO-, $R_1$, ($Am_1$) et ($Am_2$) ne comportent pas de groupement carboxylique ou carboxylate de métal alcalin, $R_{16}$ et/ou $R_{17}$ et/ou $R_{18}$ sont différents du groupement amino ou d'un groupement alkylsulfonamido en $C_1$-$C_4$ et R représente le groupement cyano, un groupement $R_4$-O-N=CH-, un groupement (a) dans lequel $R_5$ représente un groupement alkyle en $C_1$-$C_{10}$ ou cycloalkyle en $C_3$-$C_6$ ou encore le groupement (k), **caractérisé en ce que** l'on fait réagir un composé de formule générale :

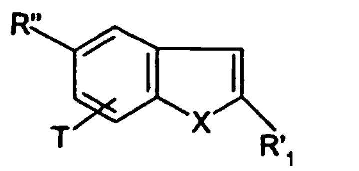

(6)

dans laquelle T et X ont la même signification que dans la Revendication 1, R'' représente le groupement cyano, le groupement (k) un groupement $R_4$-O-N=CHou un groupement -$CO_2R''_5$ dans lequel $R''_5$ représente un groupement alkyle en $C_1$-$C_{10}$ ou cycloalkyle en $C_3$-$C_6$ et $R'_1$ représente un groupement alkyle en $C_1$-$C_8$ ou un groupement (m) ne comportant pas de groupement carboxylique ou carboxylate de métal alcalin, avec un halogénure de formule générale :

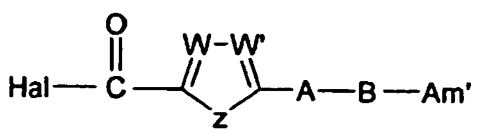

(7)

dans laquelle A, B, W, W' et Z ont la même signification que dans la Revendication 1, Hal représente un atome d'halogène et Am' représente un groupement ($Am_1$) ou ($Am_2$) tel que défini dans la Revendication 1, ce groupement ne comportant pas de groupement carboxylique ou carboxylate de métal alcalin ou encore Am' représente un groupement ($Am_3$) tel que défini dans la Revendication 1 dans lequel $R_{18}$ et/ou $R_{17}$ et/ou $R_{18}$ sont autres qu'un groupement amino ou un groupement alkylsulfonamido en $C_1$-$C_4$, la réaction ayant lieu éventuellement en présence d'un acide de Lewis, ce qui fournit les composés désirés sous forme de base libre que l'on peut faire réagir, si nécessaire, avec un acide organique ou inorganique pour former un sel pharmaceutiquement acceptable.

**16.** Procédé de préparation de dérivés de benzofurane ou de benzothiophène selon la Revendication 1 dans laquelle Y représente le groupement -CO-, $R_1$, ($Am_1$) et ($Am_2$) ne comportent pas de groupement carboxylique ou carboxylate de métal alcalin, $R_{16}$ et/ou $R_{17}$ et/ou $R_{18}$ sont différents du groupement amino ou d'un groupement alkylsulfonamido en $C_1$-$C_4$ et R représente le groupement (j), **caractérisé en ce que** l'on fait réagir avec le phosgène un composé de formule:

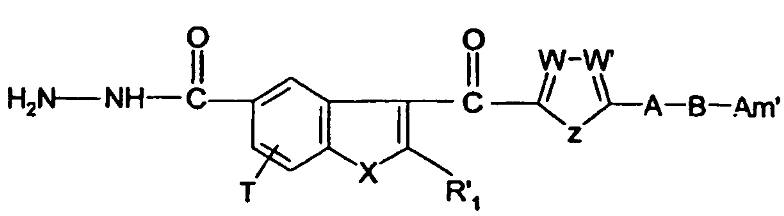

(9)

dans laquelle A, B, T, W, W', X et Z ont la même signification que dans la Revendication 1, $R'_1$ représente un groupement alkyle en $C_1$-$C_6$ ou un groupement (m) ne comportant pas de groupement carboxylique ou carboxylate de métal alcalin et Am' représente un groupement ($Am_1$) ou ($Am_2$) tel que défini dans la Revendication 1, ce groupement ne comportant pas de groupement carboxylique ou carboxylate de métal alcalin ou encore Am' repré-

sente un groupement ($Am_3$) tel que défini dans la Revendication 1 dans lequel $R_{16}$ et/ou $R_{17}$ et/ou $R_{18}$ sont autres qu'un groupement amino ou un groupement alkylsulfonamido en $C_1$-$C_4$, ce qui fournit les composés désirés sous forme de chlorhydrate que l'on peut traiter, si nécessaire, avec un agent basique tel qu'un hydroxyde de métal alcalin ou un carbonate de métal alcalin, ce qui fournit les composés désirés sous forme de base libre que l'on peut faire réagir, si nécessaire, avec un acide organique ou inorganique pour former un sel pharmaceutiquement acceptable.

**17.** Procédé de préparation de dérivés de benzofurane ou de benzothiophène selon la Revendication 1 dans laquelle Y représente le groupement -CO-, $R_1$, ($Am_1$) et ($Am_2$) ne comportent pas de groupement carboxylique ou carboxylate de métal alcalin, $R_{16}$ et/ou $R_{17}$ et/ou $R_{18}$ sont différents du groupement amino ou d'un groupement alkylsulfonamido en $C_1$-$C_4$ et R représente un groupement (b) dans lequel $R'_5$ représente un groupement (c) du type dialkylaminoalkyle primaire, **caractérisé en ce que** l'on fait réagir un composé de formule :

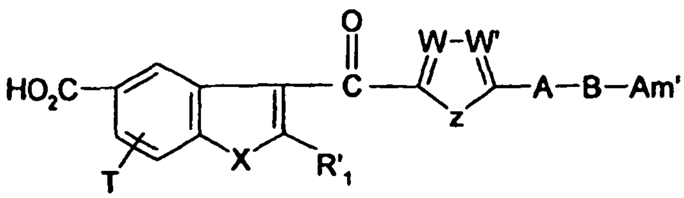

**(10)**

dans laquelle A, B, T, X, W, W', X et Z ont la même signification que dans la Revendication 1, $R'_1$ représente un groupement alkyle en $C_1$-$C_6$ ou un groupement (m) ne comportant pas de groupement carboxylique ou carboxylate de métal alcalin et Am' représente un groupement ($Am_1$) ou ($Am_2$) tel que défini dans la Revendication 1, ce groupement ne comportant pas de groupement carboxylique ou carboxylate de métal alcalin ou encore Am' représente un groupement ($Am_3$) tel que défini dans la Revendication 1 dans lequel $R_{16}$ et/ou $R_{17}$ et/ou $R_{18}$ sont autres qu'un groupement amino ou un groupement alkylsulfonamido en $C_1$-$C_4$, et après protection de la fonction amine lorsque Am' représente un groupement ($Am_1$) dans lequel $R_2$ représente l'hydrogène et ce, avec un alcool de formule générale:

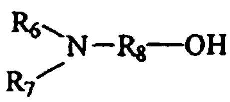

**(11)**

dans laquelle $R_6$ et $R_7$ ont la même signification que dans la Revendication 1 et $R_8$ représente un groupement alkylène linéaire en $C_1$-$C_8$, la réaction ayant lieu en présence de carbonyl diimidazole et de 1,8-diazabicyclo[5.4.0.] undec-7-ene, puis on déprotège, si nécessaire, le composé formé, ce qui fournit les composés désirés sous forme de base libre, que l'on peut traiter, si nécessaire, avec un acide organique ou inorganique approprié pour obtenir un sel pharmaceutiquement acceptable.

**18.** Procédé de préparation de dérivés de benzofurane ou de benzothiophène selon la Revendication 1 dans laquelle Y représente le groupement -CO-, $R_1$, ($Am_1$) et ($Am_2$) ne comportent pas de groupement carboxylique ou carboxylate de métal alcalin, $R_{16}$ et/ou $R_{17}$ et/ou $R_{18}$ sont différents du groupement amino ou d'un groupement alkylsulfonamido en $C_1$-$C_4$ et R représente un groupement (b) dans lequel $R'_5$ représente un groupement (c) du type dialkylaminoalkyle secondaire ou tertiaire, **caractérisé en ce que** l'on fait réagir un composé de formule :

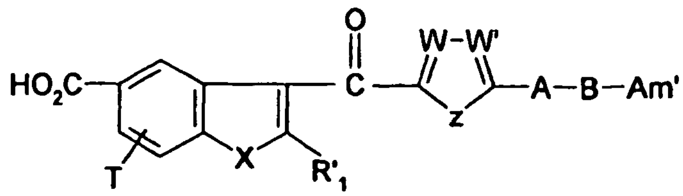

(10)

dans laquelle A, B, T, X, W, W' et Z ont la même signification que dans la Revendication 1, $R'_1$ représente un groupement alkyle en $C_1$-$C_6$ ou un groupement (m) ne comportant pas de groupement carboxylique ou carboxylate de métal alcalin et Am' représente un groupement ($Am_1$) ou ($Am_2$) tel que défini dans la Revendication 1, ce groupement ne comportant pas de groupement carboxylique ou carboxylate de métal alcalin ou encore Am' représente un groupement ($Am_3$) tel que défini dans la Revendication 1 dans lequel $R_{18}$ et/ou $R_{17}$ et/ou $R_{18}$ sont autres qu'un groupement amino ou un groupement alkylsulfonamido en $C_1$-$C_4$, et après protection de la fonction amine lorsque Am' représente un groupement ($Am_1$) dans lequel $R_2$ représente l'hydrogène et ce, avec un agent halogénant pour obtenir un halogénure d'acyle que l'on traite ensuite avec un alcool de formule :

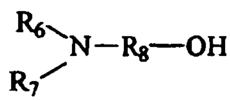

(11)

dans laquelle $R_6$ et $R_7$ ont la même signification que dans la Revendication 1 et $R_8$ représente un groupement alkylène secondaire ou tertiaire en $C_2$-$C_6$, puis on déprotège, si nécessaire, le composé formé, ce qui fournit les composés désirés sous forme d'halohydrate ou sous forme de base libre lorsque le composé de formule (10) est en excès, halohydrate que l'on peut traiter, si nécessaire, avec un agent basique tel qu'un hydroxyde de métal alcalin ou un carbonate de métal alcalin, pour obtenir les composés désirés sous forme de base libre, la base libre ainsi formée pouvant, si nécessaire, être traitée avec un acide organique ou inorganique approprié pour obtenir un sel pharmaceutiquement acceptable.

**19.** Procédé de préparation de dérivés de benzofurane ou de benzothiophène selon la Revendication 1 dans laquelle Y représente le groupement -CO-, $R_1$, ($Am_1$) et ($Am_2$) ne comportent pas de groupement carboxylique ou carboxylate de métal alcalin, $R_{16}$ et/ou $R_{17}$ et/ou $R_{18}$ sont différents du groupement amino ou d'un groupement alkylsulfonamido en $C_1$-$C_4$ et R représente soit un groupement (a) dans lequel $R_5$ représente un groupement alkyle en $C_1$-$C_{10}$ ou cycloalkyle en $C_3$-$C_6$, soit un groupement (b) dans lequel $R'_5$ représente un groupement pipéridinyle éventuellement N-substitué par un groupement alkyle en $C_1$-$C_4$ ou dans lequel $R'_5$ représente un groupement (d) ne comportant pas de groupement carboxylique ou carboxylate de métal alcalin, **caractérisé en ce que** l'on fait réagir un composé de formule :

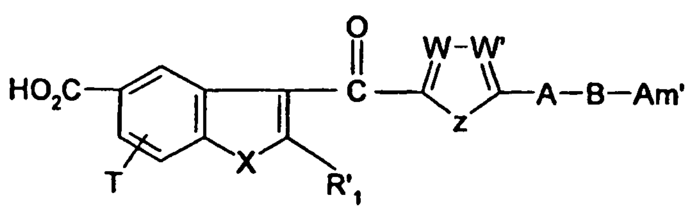

(10)

dans laquelle A, B, T, X, W, W' et Z ont la même signification que dans la Revendication 1, $R'_1$ représente un groupement alkyle en $C_1$-$C_6$ ou un groupement (m) ne comportant pas de groupement carboxylique ou carboxylate de métal alcalin et Am' représente un groupement ($Am_1$) ou ($Am_2$) tel que défini dans la Revendication 1, ce groupement ne comportant pas de groupement carboxylique ou carboxylate de métal alcalin ou encore Am' repré-

sente un groupement ($Am_3$) tel que défini dans la Revendication 1 dans lequel $R_{16}$ et/ou $R_{17}$ et/ou $R_{18}$ sont autres qu'un groupement amino ou un groupement alkylsulfonamido en $C_1$-$C_4$, et après protection de la fonction amine lorsque Am' représente un groupement ($Am_1$) dans lequel $R_2$ représente l'hydrogène et ce, avec un agent halogénant pour obtenir un halogénure d'acyle que l'on traite ensuite avec un alcool de formule générale :

$R'''_5$-OH (12)

dans laquelle $R'''_5$ représente un groupement alkyle en $C_1$-$C_{10}$, cycloalkyle en $C_3$-$C_6$ ou un groupement (b) dans lequel $R'_5$ représente un groupement pipéridinyle éventuellement N-substitué par un groupement alkyle en $C_1$-$C_4$ ou $R'_5$ représente un groupement (d) ne comportant pas de groupement carboxylique ou carboxylate de métal alcalin puis on déprotège, si nécessaire, le composé formé, ce qui fournit les composés désirés sous forme d'halohydrate ou sous forme de base fibre lorsque le composé de formule (10) est en excès, halohydrate que l'on peut traiter, si on le désire, avec un agent basique tel qu'un hydroxyde de métal alcalin ou un carbonate de métal alcalin, pour obtenir les composés désirés sous forme de base libre, la base libre ainsi formée pouvant, si nécessaire, être traitée avec un acide organique ou inorganique approprié pour obtenir un sel pharmaceutiquement acceptable.

**20.** Procédé de préparation de dérivés de benzofurane ou de benzothiophène selon la Revendication 1 dans laquelle Y représente le groupement -CO- , $R_1$, ($Am_1$) et ($Am_2$) ne comportent pas de groupement carboxylique ou carboxylate de métal alcalin, $R_{16}$ et/ou $R_{17}$ et/ou $R_{18}$ sont différents du groupement amino ou d'un groupement alkylsulfonamido en $C_1$-$C_4$ et R représente un groupement (e) dans lequel $R_{10}$ représente un groupement (f) ne comportant pas de groupement carboxylique ou carboxylate de métal alcalin, **caractérisé en ce que** l'on fait réagir un composé de formule :

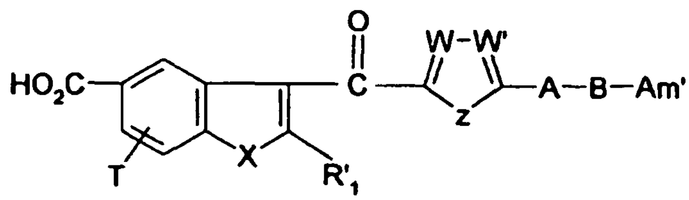

(10)

dans laquelle A, B, T, X, W, W' et Z ont la même signification que dans la Revendication 1, $R'_1$ représente un groupement alkyle en $C_1$-$C_8$ ou un groupement (m) ne comportant pas de groupement carboxylique ou carboxylate de métal alcalin et Am' représente un groupement ($Am_1$) ou ($Am_2$) tel que défini dans la Revendication 1, ce groupement ne comportant pas de groupement carboxylique ou carboxylate de métal alcalin ou encore Am' représente un groupement ($Am_3$) tel que défini dans la Revendication 1 dans lequel $R_{16}$ et/ou $R_{17}$ et/ou $R_{18}$ sont autres qu'un groupement amino ou un groupement alkylsulfonamido en $C_1$-$C_4$, et après protection de la fonction amine lorsque Am' représente un groupement ($Am_1$) dans lequel $R_2$ représente l'hydrogène et ce, avec un agent halogénant pour obtenir un chlorure d'acyle que l'on traite ensuite, avec un composé de formule :

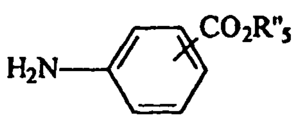

(13)

dans laquelle $R''_5$ représente un radical alkyle en $C_1$-$C_{10}$ ou cycloalkyle en $C_3$-$C_6$, puis on déprotège, si nécessaire, le composé formé ce qui fournit, sous forme de base libre, les composés désirés dans lesquels $R_{10}$ représente un groupement (f) dont le groupement $R_{11}$ représente un groupement (a) dans lequel $R_5$ représente un groupement alkyle en $C_1$-$C_4$ ou cycloalkyle en $C_3$-$C_8$., la base libre ainsi formée pouvant si nécessaire, être traitée avec un acide organique ou inorganique approprié pour former un sel pharmaceutiquement acceptable.

**21.** Procédé de préparation de dérivés de benzofurane ou de benzothiophène selon la Revendication 1 dans laquelle Y représente le groupement -CO- , $R_1$, ($Am_1$) et ($Am_2$) ne comportent pas de groupement carboxylique ou carboxylate de métal alcalin, $R_{16}$ et/ou $R_{17}$ et/ou $R_{18}$ sont différents du groupement amino ou d'un groupement alkylsulfonamido

en $C_1$-$C_4$ et R représente un groupement (e) dans lequel $R_{10}$ représente un groupement alkyle en $C_1$-$C_4$, un groupement amino ou un groupement (c), **caractérisé en ce que** l'on fait réagir un composé de formule :

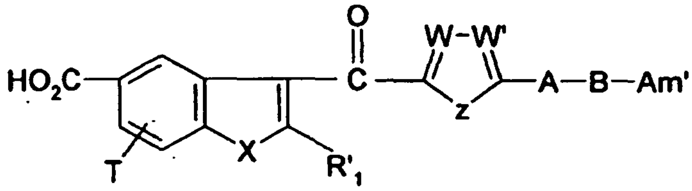

(10)

dans laquelle A, B, T, X, W, W et Z ont la même signification que dans la Revendication 1, R'$_1$ représente un groupement alkyle en $C_1$-$C_6$ ou un groupement (m) ne comportant pas de groupement carboxylique ou carboxylate de métal alcalin et Am' représente un groupement ($Am_1$) ou ($Am_2$) tel que défini dans la Revendication 1, ce groupement ne comportant pas de groupement carboxylique ou carboxylate de métal alcalin ou encore Am' représente un groupement ($Am_3$) tel que défini dans la Revendication 1 dans lequel $R_{16}$ et/ou $R_{17}$ et/ou $R_{18}$ sont autres qu'un groupement amino ou un groupement alkylsulfonamido en $C_1$-$C_4$, et après protection de la fonction amine lorsque Am' représente un groupement ($Am_1$) dans lequel $R_2$ représente l'hydrogène et ce, avec un agent halogénant pour obtenir un halogénure d'acyle que l'on traite ensuite avec une amine de formule générale :

$R'_{10}$-$NH_2$ (14)

ou

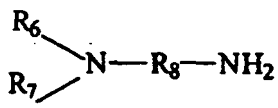

(15)

dans laquelle $R_6$, $R_7$, et $R_8$ ont la même signification que dans la Revendication 1 et $R'_{10}$ représente un radical alkyle en $C_1$-$C_4$ ou amino, puis on déprotège, si nécessaire, le composé formé, ce qui fournit éventuellement après traitement basique, le composé désiré de formule (1) sous forme d'halohydrate ou sous forme de base libre lorsque le composé de formule (10) est en excès, halohydrate que l'on peut traiter, si nécessaire, avec un agent basique tel qu'un hydroxyde de métal alcalin ou un carbonate de métal alcalin, pour obtenir les composés désirés sous forme de base libre que l'on peut, si nécessaire, faire réagir avec un acide organique ou inorganique approprié pour former un sel pharmaceutiquement acceptable.

**22.** Procédé de préparation de dérivés de benzofurane ou de benzothiophène selon la Revendication 1 dans laquelle Y représente le groupement -CO- , $R_1$, ($Am_1$) et ($Am_2$) ne comportent pas de groupement carboxylique ou carboxylate de métal alcalin, $R_{16}$ et/ou $R_{17}$ et/ou $R_{18}$ sont différents du groupement amino ou d'un groupement alkylsulfonamido en $C_1$-$C_4$ et R représente un groupement (e) dans lequel $R_{10}$ représente un groupement (g) ne comportant pas de groupement carboxylique ou carboxylate de métal alcalin, **caractérisé en ce que** l'on fait réagir un composé de formule :

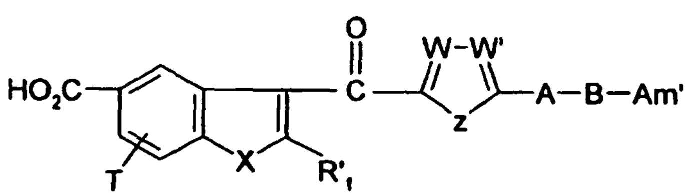

(10)

dans laquelle A, B, T, X, W, W' et Z ont la même signification que dans la Revendication 1, $R'_1$ représente un groupement alkyle en $C_1$-$C_6$ ou un groupement (m) ne comportant pas de groupement carboxylique ou carboxylate de métal alcalin et Am' représente un groupement ($Am_1$) ou ($Am_2$) tel que défini dans la Revendication 1, ce groupement ne comportant pas de groupement carboxylique ou carboxylate de métal alcalin ou encore Am' représente un groupement ($Am_3$) tel que défini dans le Revendication 1 dans lequel $R_{16}$ et/ou $R_{17}$ et/ou $R_{18}$ sont autres qu'un groupement amino ou un groupement alkylsulfonamido en $C_1$-$C_4$, et après protection de la fonction amine lorsque Am' représente un groupement ($Am_1$) dans lequel $R_2$ représente l'hydrogène et ce, avec un sel d'un composé de formule générale :

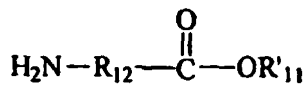

(16)

dans laquelle $R_{12}$ a la même signification que dans la Revendication 1 et $R'_{11}$ représente un radical alkyle en $C_1$-$C_4$ ou cycloalkyle en $C_3$-$C_6$, la réaction ayant lieu en présence d'un capteur d'acide, puis on déprotège, si nécessaire, le composé formé, ce qui fournit, sous forme de base libre, les composés désirés dans lesquels $R_{10}$ représente un groupement (g) dont le groupement $R_{11}$ représente un groupement (a) dans lequel $R_5$ représente un groupement alkyle en $C_1$-$C_4$ ou cycloalkyle en $C_3$-$C_6$, la base libre ainsi formée pouvant, si nécessaire, être traitée avec un acide organique ou inorganique approprié pour obtenir un sel pharmaceutiquement acceptable.

**23.** Procédé de préparation de dérivés de benzofurane ou de benzothiophène selon la Revendication 1 dans laquelle Y représente le groupement -CO-, $R_1$, ($Am_1$) et ($Am_2$) ne comportent pas de groupement carboxylique ou carboxylate de métal alcalin, $R_{16}$ et/ou $R_{17}$ et/ou $R_{18}$ sont différents du groupement amino ou d'un groupement alkylsulfonamido en $C_1$-$C_4$ et R représente un groupement (h), **caractérisé en ce que** l'on fait réagir un composé de formule :

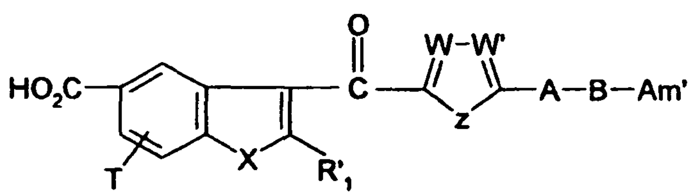

(10)

dans laquelle A, B. T, X, W, W' et Z ont la même signification que dans la Revendication 1, $R'_1$ représente un groupement alkyle en $C_1$-$C_6$ ou un groupement (m) ne comportant pas de groupement carboxylique ou carboxylate de métal alcalin et Am' représente un groupement ($Am_1$) ou ($Am_2$) tel que défini dans la Revendication 1, ce groupement ne comportant pas de groupement carboxylique ou carboxylate de métal alcalin ou encore Am' représente un groupement ($Am_3$) tel que défini dans le Revendication 1 dans lequel $R_{16}$ et/ou $R_{17}$ et/ou $R_{18}$ sont autres qu'un groupement amino ou un groupement alkylsulfonamido en $C_1$-$C_4$, et après protection de la fonction amine lorsque Am' représente un groupement ($Am_1$) dans lequel $R_2$ représente l'hydrogène et ce avec un agent halogénant, pour obtenir un halogénure d'acyle que l'on traite ensuite avec une amine de formule générale :

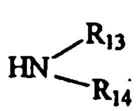

(17)

dans laquelle $R_{13}$ et $R_{14}$ ont la même signification que dans la Revendication 1, puis on déprotège, si nécessaire le composé formé, ce qui fournit un sel du composé désiré que l'on traite par un agent basique approprié, pour obtenir sous forme de base libre, les composés désirés, la base libre ainsi formée pouvant, si nécessaire, être traitée avec un acide organique ou inorganique pour obtenir un sel pharmaceutiquement acceptable.

**24.** Procédé de préparation de dérivés de benzofurane ou de benzothiophène selon la Revendication 1 dans laquelle Y représente le groupement -CO-, $R_1$, ($Am_1$) et ($Am_2$) ne comportent pas de groupement carboxylique ou carboxylate de métal alcalin, $R_{16}$ et/ou $R_{17}$ et/ou $R_{18}$ sont différents du groupement amino ou d'un groupement alkylsulfonamido en $C_1$-$C_4$ et R représente un groupement (e) dans lequel $R_{10}$ représente le groupement hydroxyle, **caractérisé en ce que** l'on fait réagir un composé de formule :

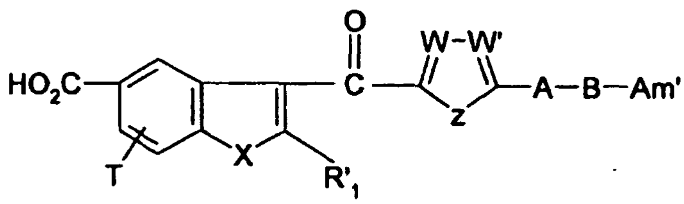

**(10)**

dans laquelle A, B, T, X, W, W' et Z ont la même signification que dans la Revendication 1, $R'_1$ représente un groupement alkyle en $C_1$-$C_6$ ou un groupement (m) ne comportant pas de groupement carboxylique ou carboxylate de métal alcalin et Am' représente un groupement ($Am_1$) ou ($Am_2$) tel que défini dans la Revendication 1, ce groupement ne comportant pas de groupement carboxylique ou carboxylate de métal alcalin ou encore Am' représente un groupement ($Am_3$) tel que défini dans la Revendication 1 dans lequel $R_{16}$ et/ou $R_{17}$ et/ou $R_{18}$ sont autres qu'un groupement amino ou un groupement alkylsulfonamido en $C_1$-$C_4$, et après protection de la fonction amine lorsque Am' représente un groupement ($Am_1$) dans lequel $R_2$ représente l'hydrogène et ce, avec un sel de benzyloxyamine, en présence d'un capteur d'acide et de benzotriazol-1-yloxy-tris(diméthytamino)phosphonium hexafluorophosphate, puis on déprotège, si nécessaire, le composé formé, ce qui fournit des dérivés benzyloxyaminocarbonyle que l'on hydrogène en présence d'un catalyseur approprié pour obtenir sous forme de base libre, les composés désirés que l'on traite, si nécessaire, avec un acide organique ou inorganique approprié pour former un sel pharmaceutiquement acceptable.

**25.** Procédé selon une des Revendications 18 à 24, **caractérisé en ce que** la protection de la fonction amine s'effectue par traitement au moyen de 9-fluorenylméthylchloroformiate et la déprotection s'opère par traitement avec une amine secondaire.

**26.** Procédé de préparation de dérivés de benzofurane ou de benzothiophène selon la Revendication 1 dans laquelle Y représente le groupement -CO-, $R_1$, ($Am_1$) et ($Am_2$) ne comportent pas de groupement carboxylique ou carboxylate de métal alcalin, $R_{16}$ et/ou $R_{17}$ et/ou $R_{18}$ sont différents du groupement amino ou d'un groupement alkylsulfonamido en $C_1$-$C_4$ et R représente un groupement (e) dans lequel $R_{10}$ représente l'hydrogène, **caractérisé en ce que** l'on hydrolyse en présence d'un acide fort, un composé de formule :

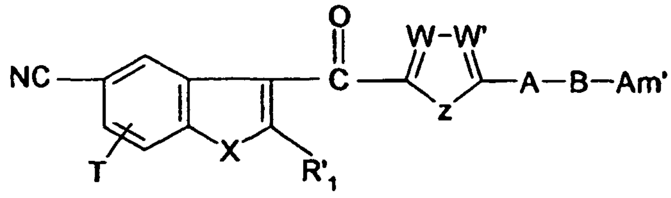

**(18)**

dans laquelle A, B, T, X, W, W' et Z ont la même signification que dans la Revendication 1, $R'_1$ représente un groupement alkyle en $C_1$-$C_6$ ou un groupement (m) ne comportant pas de groupement carboxylique ou carboxylate

de métal alcalin et Am' représente un groupement ($Am_1$) ou ($Am_2$) tel que défini dans la Revendication 1, ce groupement ne comportant pas de groupement carboxylique ou carboxylate de métal alcalin ou encore Am' représente un groupement ($Am_3$) tel que défini dans la Revendication 1 dans lequel $R_{16}$ et/ou $R_{17}$ et/ou $R_{18}$ sont autres qu'un groupement amino ou un groupement alkylsulfonamido en $C_1$-$C_4$, ce qui fournit les composés désirés sous forme de base libre que l'on peut traiter, si nécessaire, avec un acide organique ou inorganique approprié pour former un sel pharmaceutiquement acceptable.

**27.** Procédé de préparation de dérivés de benzofurane ou de benzothiophène selon la Revendication 1 dans laquelle Y représente le groupement -CO-, $R_1$, ($Am_1$) et ($Am_2$) ne comportent pas de groupement carboxylique ou carboxylate de métal alcalin, $R_{16}$ et/ou $R_{17}$ et/ou $R_{18}$ sont différents du groupement amino ou d'un groupement alkylsulfonamido en $C_1$-$C_4$ et R représente le groupement (I), **caractérisé en ce que** l'on fait réagir, avec le tributylazido étain, un composé de formule :

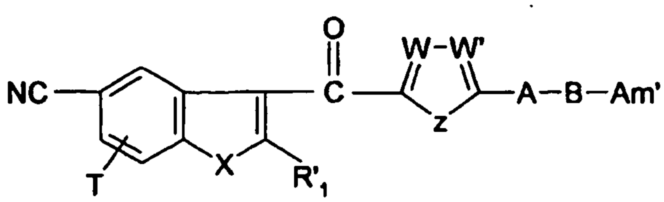

**(18)**

dans laquelle A, B, T, X, W, W' et Z ont la même signification que dans la Revendication 1, R'$_1$ représente un groupement alkyle en $C_1$-$C_6$ ou un groupement (m) ne comportant pas de groupement carboxylique ou carboxylate de métal alcalin et Am' représente un groupement ($Am_1$) ou ($Am_2$) tel que défini dans la Revendication 1, ce groupement ne comportant pas de groupement carboxylique ou carboxylate de métal alcalin ou encore un groupement ($Am_3$) tel que défini dans la Revendication 1 dans lequel $R_{16}$ et/ou $R_{17}$ et/ou $R_{18}$ sont autres qu'un groupement amino ou un groupement alkylsulfonamido en $C_1$-$C_4$, ce qui fournit les composés désirés sous forme de base libre que l'on peut traiter, si nécessaire, avec un acide organique ou inorganique approprié pour former un sel pharmaceutiquement acceptable.

**28.** Procédé de préparation de dérivés de benzofurane ou de benzothiophène selon la Revendication 1 dans laquelle Y représente le groupement -CO-, $R_1$ ne comporte pas de groupement carboxylique ou carboxylate de métal alcalin et Am représente un groupement ($Am_1$) ne comportant pas de groupement carboxylique ou carboxylate de métal alcalin et dans lequel $R_{16}$ et/ou $R_{17}$ et/ou $R_{18}$ représentent le groupement amino ou encore Am représente un groupement ($Am_3$) dans lequel $R_{16}$ et/ou $R_{17}$ et/ou $R_{18}$ représentent le groupement amino, **caractérisé en ce que** l'on hydrogène un composé nitro de formule :

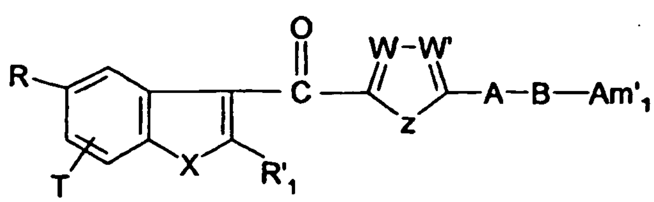

**(19)**

dans laquelle A, B, T, W, W', X et Z ont la même signification que dans la Revendication 1, R a la même signification que dans la Revendication 1 mais ne comporte pas de groupement carboxylique ou carboxylate de métal alcalin, R'$_1$ représente un groupement alkyle en $C_1$-$C_6$ ou un groupement (m) ne comportant pas de groupement carboxylique ou carboxylate de métal alcalin et Am'$_1$ représente soit un groupement ($Am_1$) ne comportant pas de groupement carboxylique ou carboxylate de métal alcalin et dans lequel $R_{16}$ et/ou $R_{17}$ et/ou $R_{18}$ représentent le groupement nitro, soit un groupement ($Am_3$) dans lequel $R_{18}$ et/ou $R_{17}$ et/ou $R_{18}$ représentent le groupement nitro et ce, en présence d'un catalyseur approprié, ce qui fournit sous forme de base libre les composés désirés que l'on peut

faire réagir, si nécessaire, avec un acide organique ou inorganique approprié pour former un sel pharmaceutiquement acceptable.

**29.** Procédé de préparation de dérivés de benzofurane ou de benzothiophène selon la Revendication 1 dans laquelle Y représente le groupement -CO-, $R_1$ ne comporte pas de groupement carboxylique ou carboxylate de métal alcalin et Am représente un groupement ($Am_1$) ne comportant pas de groupement carboxylique ou carboxylate de métal alcalin et dans lequel $R_{16}$ et/ou $R_{17}$ et/ou $R_{18}$ représentent un groupement alkylsulfonamido en $C_1$-$C_4$ ou encore Am représente un groupement ($Am_3$) dans lequel $R_{18}$ et/ou $R_{17}$ et/ou $R_{18}$ représentent un groupement alkylsulfonamido en $C_1$-$C_4$, **caractérisé en ce que** l'on fait réagir un composé amino de formule :

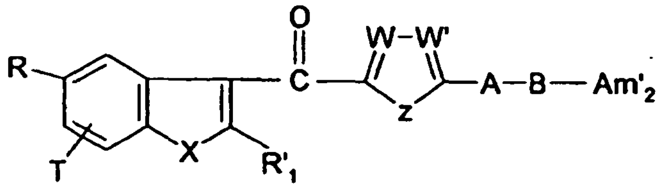

**(20)**

dans laquelle A, B, T, W, W', X et Z ont la même signification que dans la Revendication 1, R a la même signification que dans la Revendication 1, mais ne comporte pas de groupement carboxylique ou carboxylate de métal alcalin, $R'_1$ représente un groupement alkyle en $C_1$-$C_6$ ou un groupement (m) ne comportant pas de groupement carboxylique ou carboxylate de métal alcalin et $Am'_2$ représente soit un groupement ($Am_1$) ne comportant pas de groupement carboxylique ou carboxylate de métal alcalin et dans lequel $R_{16}$ et/ou $R_{17}$ et/ou $R_{18}$ représentent le groupement amino, soit un groupement ($Am_3$) dans lequel $R_{18}$ et/ou $R_{17}$ et/ou $R_{18}$ représentent le groupement amino et ce, avec un halogénure de formule générale :

$$Hal\text{-}SO_2\text{-}R'_{16} \quad (21)$$

ou un anhydride de formule générale :

$$(R'_{16}SO_2)_2O \quad (22)$$

dans laquelle $R'_{16}$ représente un radical alkyle, linéaire ou ramifié en $C_1$-$C_4$, et éventuellement en présence d'un accepteur d'acide, ce qui fournit sous forme de base libre les composés désirés que l'on peut faire réagir, si nécessaire, avec un acide organique ou inorganique approprié pour former un sel pharmaceutiquement acceptable.

**30.** Procédé de préparation de dérivés de benzofurane ou de benzothiophène selon la Revendication 1 dans laquelle Y représente le groupement -CO- et un ou deux des groupements R, $R_1$ et ($Am_1$) ou ($Am_2$) comportent un groupement $-CO_2R_5$ dans lequel $R_5$ représente l'hydrogène ou un atome de métal alcalin, l'autre ou les autres groupements étant différents d'un groupement $-CO_2R_5$ dans lequel $R_5$ représente un radical alkyle en $C_1$-$C_{10}$ ou cycloalkyle en $C_3$-$C_6$, **caractérisé en ce que** l'on saponifie, en présence d'un hydroxyde de métal alcalin, un composé de formule :

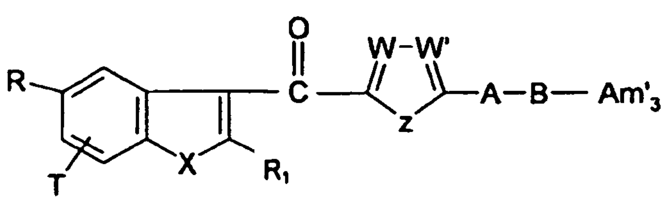

**(23)**

dans laquelle A, B, R, $R_1$, T, W, W, X et Z ont la même signification que dans la Revendication 1, $Am'_3$ représente un groupement ($Am_1$) ou ($Am_2$) tel que défini dans la Revendication 1 et R, $R_1$ et ($Am_1$) ou ($Am_2$) sont tels que l'un ou deux d'entre eux comportent un groupement $-CO_2R''_5$ dans lequel $R''_5$ représente un radical alkyle en $C_1$-$C_{10}$ ou cycloalkyle en $C_3$-$C_6$, l'autre ou les autres groupements étant différents d'un groupement $-CO_2R_5$ dans lequel

$R_5$ représente un radical alkyle en $C_1$-$C_{10}$ ou cycloalkyle en $C_3$-$C_6$. ce qui fournit, sous forme de base libre, les composés désirés de formule (1) dans laquelle un ou deux des groupements R, $R_1$ et ($Am_1$) ou ($Am_2$) comporte un groupement- $CO_2R_5$ dans lequel $R_5$ représente un atome de métal alcalin, composé que l'on traite, si nécessaire, avec un acide fort, ce qui fournit, sous forme de base libre, les composes désirés de formule (1) dans laquelle $R_5$ représente l'hydrogène, les bases libres ainsi formées pouvant, si nécessaire, être traitées avec un acide organique ou inorganique approprié pour obtenir un sel pharmaceutiquement acceptable.

**31.** Procédé de préparation de dérivés de benzofurane ou de benzothiophène selon la Revendication 1 dans laquelle Y représente le groupement -CO- et deux des groupements R, $R_1$ et ($Am_1$) ou ($Am_2$) comportent l'un un groupement -$CO_2R_5$ dans lequel $R_5$ représente l'hydrogène ou un atome de métal alcalin et l'autre un groupement -$CO_2R_5$ dans lequel $R_5$ représente un groupement alkyle en $C_1$-$C_{10}$ ou cycloalkyle en $C_3$-$C_6$, le troisième groupement étant différent d'un groupement -$CO_2R_5$ dans lequel $R_5$ représente un groupement alkyle en $C_1$-$C_{10}$ ou cycloalkyle en $C_3$-$C_6$, **caractérisé en ce que** :

- l'on hydrogène un composé de formule :

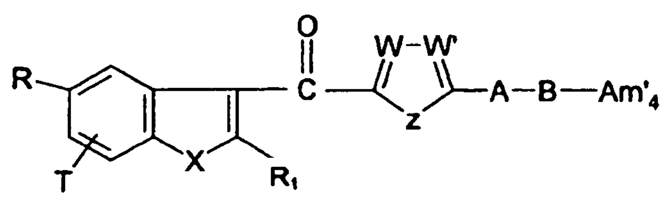

(24)

dans laquelle A, B, R, $R_1$, T, W, W', X et Z ont la même signification que dans la Revendication 1, $Am'_4$ représente un groupement ($Am_1$) ou ($Am_2$) tel que défini dans la Revendication 1 et R, $R_1$ et ($Am_1$) ou ($Am_2$) sont tels que l'un d'entre eux comporte un groupement -$CO_2R''_5$ dans lequel $R''_5$ représente un groupement alkyle en $C_1$-$C_{10}$ ou cycloalkyle en $C_3$-$C_6$ et un autre d'entre eux comporte un groupement benzyloxycarbonyle, le troisième groupement étant différent d'un groupement -$CO_2R_5$ dans lequel $R_5$ représente un groupement alkyle en $C_1$-$C_{10}$ ou cycloalkyle en $C_3$-$C_6$ et ce, en présence d'un catalyseur approprié

- l'on hydrolyse un composé de formule :

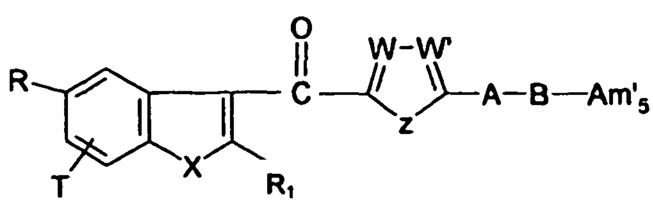

(25)

dans laquelle A, B, R, $R_1$, T, W, W', X et Z ont la même signification que dans la Revendication 1, $Am'_5$ représente un groupement ($Am_1$) ou ($Am_2$) tel que défini dans la Revendication 1 et R, $R_1$ et ($Am_1$) ou ($Am_2$) sont tels que l'un d'entre eux comporte un groupement -$CO_2R''_5$ dans lequel $R''_5$ a la même signification que précédemment et un autre d'entre eux comporte un groupement t-butoxycarbonyle, le troisième groupement étant différent d'un groupement -$CO_2R_5$ dans lequel $R_5$ représente un groupement alkyle en $C_1$-$C_{10}$ ou cycloalkyle en $C_3$-$C_8$ et ce, en présence d'acide trifluoroacétique ce qui permet d'obtenir les composés désirés de formule (1) dans laquelle deux des groupements R, $R_1$ et ($Am_1$) ou ($Am_2$) comportent l'un un groupement -$CO_2R_5$ dans lequel $R_5$ représente un groupement alkyle en $C_1$-$C_{10}$ ou cycloalkyle en $C_3$-$C_6$ et l'autre un groupement -$CO_2R_5$ dans lequel $R_5$ représente l'hydrogène, composés que l'on peut traiter, si nécessaire, avec un agent basique approprié pour obtenir, sous forme de base libre, les composés désirés de formule (1) dans laquelle deux des groupements R, $R_1$ et ($Am_1$) ou ($Am_2$) comportent l'un un groupement -$CO_2R_5$ dans lequel $R_5$ représente un atome de métal alcalin, l'autre un

groupement $-CO_2R_5$ dans lequel $R_5$ représente un groupement alkyle en $C_1$-$C_{10}$ ou cycloalkyle en $C_3$-$C_6$, composés que l'on peut eux-mêmes traiter, si nécessaire, avec un acide fort pour obtenir, sous forme de base libre, les composés désirés de formule (1), dans laquelle deux des groupements R, $R_1$ et ($Am_1$) ou ($Am_2$) comportent l'un un groupement carboxylique, l'autre un groupement $-CO_2R_5$ dans lequel $R_5$ représente un groupement alkyle en $C_1$-$C_{10}$ ou cycloalkyle en $C_3$-$C_6$, les bases libres ainsi formées pouvant, si nécessaire, être traitées avec un acide organique ou inorganique approprié pour obtenir un sel pharmaceutiquement acceptable.

**32.** Procédé de préparation de dérivés de benzofurane ou de benzothiophène selon la Revendication 1 dans laquelle Y représente -CO-, R représente le groupement cyano et l'un des groupements $R_1$, ($Am_1$) ou ($Am_2$) comporte un groupement carboxylique, **caractérisé en ce que** l'on traite au moyen d'oxyde de tributylétain, un composé de formule (1) selon la Revendication 1, dans laquelle Y représente le groupement -CO-. R représente le groupement cyano, et dans laquelle A, B, $R_1$, T, W, W', X et Z ont la même signification que dans la Revendication 1, Am représente un groupement ($Am_1$) ou ($Am_2$) tel que défini dans la Revendication 1 et $R_1$, ($Am_1$) et ($Am_2$) sont tels que l'un d'eux comporte un groupement $-CO_2R''_5$ dans lequel $R''_5$ représente un radical alkyle en $C_1$-$C_{10}$ ou cycloalkyle en $C_3$-$C_6$, pour obtenir les composés désirés sous forme de base libre que l'on peut faire réagir, si nécessaire, avec un acide organique ou inorganique pour former un sel pharmaceutiquement acceptable.

**33.** Procédé de préparation de dérivés de benzofurane ou de benzophiophéne selon la Revendication 1 dans laquelle Y représente le groupement

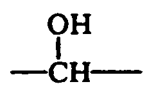

**caractérisé en ce que** l'on réduit au moyen d'un borohydrure de métal alcalin, un composé de formule (1) selon la Revendication 1 dans laquelle Y représente le groupement -CO- et dans laquelle A, B, Am, R, $R_1$, T, W, W, X et Z ont la même signification que dans la Revendication 1, ce qui fournit les composés désirés sous forme de base libre que l'on peut faire réagir, si nécessaire, avec un acide organique ou inorganique pour obtenir un sel pharmaceutiquement acceptable.

**34.** Procédé de préparation de dérivés de benzofurane ou de benzothiophène selon la Revendication 1 dans laquelle Y représente le groupement

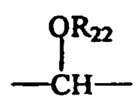

dans lequel $R_{22}$ représente un radical alkyle en $C_1$-$C_4$, **caractérisé en ce que** l'on faire réagir un composé de formule (1) selon la Revendication 1 dans laquelle Y représente le groupement -CHOH- et dans laquelle A, B, Am, R, $R_1$, T, W, W', X et Z ont la même signification que dans la Revendication 1, avec un alcoolate de métal alcalin puis avec un halogénure de formule :

$$R_{23}\text{-Hal}$$

dans laquelle Hal représente un atome d'halogène et $R_{23}$ représente un radical alkyle en $C_1$-$C_4$, ce qui fournit les composés désirés sous forme de base libre que l'on peut faire réagir, si nécessaire, avec un acide organique ou inorganique pour former un sel pharmaceutiquement acceptable.

**35.** Procédé de préparation de dérivés de benzofurane ou de benzothiophène selon la Revendication 1 dans laquelle Y représente un groupement

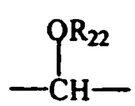

dans lequel $R_{22}$ représente un radical acyle de formule -CO-$R_{23}$ dans lequel $R_{23}$ représente un radical alkyle en $C_1$-$C_4$, **caractérisé en ce que** l'on fait réagir un composé de formule (1) selon la Revendication 1 dans laquelle Y représente le groupement -CHOH- et dans laquelle A, B, Am, R, $R_1$, T, W, W', X et Z ont la même signification que dans la Revendication 1, avec un halogénure d'acyle de formule :

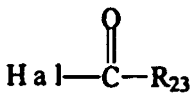

(27)

dans laquelle Hal représente un atome d'halogène et $R_{23}$ représente un radical alkyle en $C_1$-$C_4$, ce qui fournit les composés désirés sous forme de base libre que l'on peut faire réagir, si nécessaire, avec un acide organique ou inorganique pour former un sel pharmaceutiquement acceptable.

**36.** Procédé de préparation de dérivés de benzofurane ou de benzothiophène selon la Revendication 1 dans laquelle Y représente le groupement -$CH_2$-, **caractérisé en ce que** l'on réduit au moyen d'un borohydrure de métal alcalin et en présence d'acide trifluoroacétique, un composé de formule (1) selon la Revendication 1 dans laquelle Y représente le groupement -CHOH- et dans laquelle A, B, Am, R, $R_1$, T, W, W', X et Z ont la même signification que dans la Revendication 1, ce qui fournit les composés désirés sous forme de base libre que l'on peut faire réagir, si nécessaire, avec un acide organique ou inorganique approprié pour former un sel pharmaceutiquement acceptable.

**37.** Médicament, **caractérisé en ce qu'**il comprend un dérivé de benzofurane ou de benzothiophène, ou un sel pharmaceutiquement acceptable de ce dernier, selon l'une quelconque des revendications 1 à 12.

**38.** Composition pharmaceutique ou vétérinaire **caractérisée en ce qu'**elle contient comme principe actif au moins un dérivé de benzofurane ou de benzothiophène, ou un sel pharmaceutiquement acceptable de ce dernier, selon une des Revendications 1 à 12, en association avec un véhicule pharmaceutique ou excipient approprié.

**39.** Composition pharmaceutique ou vétérinaire selon la Revendication 38, pour le traitement de syndromes pathologiques du système cardio-vasculaire.

**40.** Composition pharmaceutique ou vétérinaire selon la Revendication 38 ou 39, pour le traitement de l'angine de poitrine, de l'hypertension, de l'arythmie atriale, ventriculaire ou supraventriculaire, de l'insuffisance circulatoire cérébrale, de l'insuffisance cardiaque, de l'infarctus du myocarde compliqué ou non d'insuffisance cardiaque ou pour la prévention de la mortalité post-infarctus.

**41.** Composition pharmaceutique ou vétérinaire selon une des Revendications 38 à 40, **caractérisée en ce qu'**elle contient de 50 à 500 mg de principe actif.

**42.** Utilisation d'au moins un dérivé de benzofurane ou de benzothiophène, ou d'un sel pharmaceutiquement acceptable de ce dernier, selon une des Revendications 1 à 12, pour la fabrication d'un médicament destiné au traitement de syndromes pathologiques du système cardio-vasculaire.

## Claims

**1.** Benzofuran or benzothiophene derivatives of general formula:

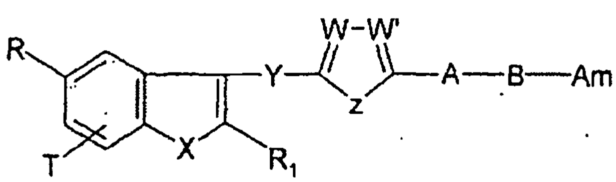

(1)

and also their pharmaceutically acceptable salts, in which formula:

A represents -O- or -S-
B represents a linear or branched $C_1$-$C_5$ alkylene group optionally substituted with a hydroxyl group,
T represents hydrogen
R represents

- the cyano or hydroxymethyl group
- an oxime group of formula:

$R_4$-O-N=CH-

in which $R_4$ represents a $C_1$-$C_4$ alkyl group
- a carboxyl group of general formula:

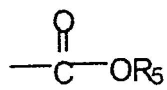 (a)

in which $R_5$ represents hydrogen or an alkali metal atom, a linear or branched $C_1$-$C_{10}$ alkyl group or a $C_3$-$C_6$ cycloalkyl group, or $R_5$ represents the group of general formula:

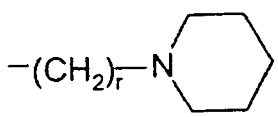 (a-1)

in which r represents 1 to 4

- a carboxyl group of general formula:

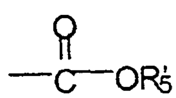 (b)

in which R'$_5$ represents a piperidinyl group optionally N-substituted with a $C_1$-$C_4$ alkyl group or one of the groups of general formula:

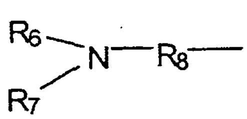 or 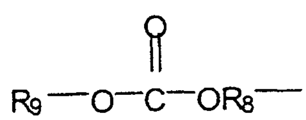

(c) (d)

in which $R_6$ and $R_7$, which may be identical or different, represent a $C_1$-$C_4$ alkyl group, $R_8$ represents a linear or branched $C_1$-$C_6$ alkylene group, and $R_9$ represents hydrogen, an alkali metal atom or a $C_1$-$C_4$ alkyl group,

- an aminocarbonyl group of formula:

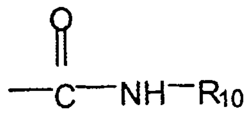

(e)

in which $R_{10}$ represents hydrogen, a $C_1$-$C_4$ alkyl, hydroxyl or amino group, a group (c) above or one of the groups:

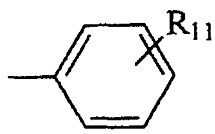

or

$-R_{12}-R_{11}$

(f) (g)

in which $R_{11}$ represents a group (a) and $R_{12}$ represents a $C_1$-$C_6$ alkylene radical,

• a group of formula:

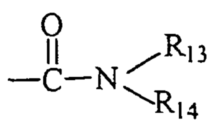

(h)

in which $R_{13}$ and $R_{14}$, which may be identical or different, represent a $C_1$-$C_4$ alkyl radical or a $C_1$-$C_4$ hydroxyalkyl group,

• one of the groups of formula below:

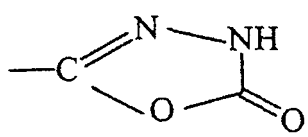

(j)

or

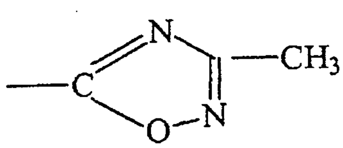

or

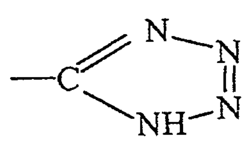

(k) (l)

$R_1$ represents a $C_1$-$C_6$ alkyl group, or a group of formula:

$-(CH_2)_p-R_{11}$ (m)

in which $R_{11}$ has the same meaning as previously and p represents 1 to 4,
Am represents a nitrogenous group of formula:

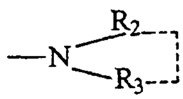 or 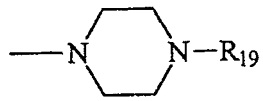 or 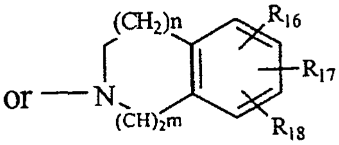

(Am$_1$) (Am$_2$) (Am$_3$)

in which:

$R_2$ represents hydrogen, a linear or branched $C_1$-$C_6$ alkyl group optionally substituted with a hydroxyl group, a group (m), a $C_3$-$C_6$ cycloalkyl group or a benzyl group,
$R_3$ represents a linear or branched $C_1$-$C_6$ alkyl group optionally substituted with a hydroxyl group, a $C_3$-$C_6$ cycloalkyl group, a group (m), a benzyl group or a phenyl group of formula:

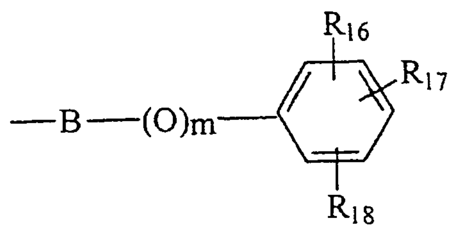

(n)

$R_{16}$, $R_{17}$ and $R_{18}$, which may be identical or different, represent hydrogen, or a hydroxyl, nitro, amino, $C_1$-$C_4$ alkoxy or $C_1$-$C_4$ alkylsulfonamido group,
$R_{19}$ represents hydrogen, a $C_1$-$C_5$ alkyl group, the diphenylmethyl group, a mono-, di- or trimethylphenyl group, a mono-, di- or trimethoxyphenyl group, a group (a), a group (b) or a group (c),
m and n each represent 0 or 1,
$R_2$ and $R_3$, when they are taken together, represent a linear or branched $C_3$-$C_{10}$ alkylene group optionally substituted with the hydroxyl group, with a group (a) or with a group (m) and optionally interrupted by -O-,
W, W' and Z are such that:

- when W and W', which are identical, represent CH, Z represents -O- or -S-,
- when W represents CH and W' represents C-$R_{20}$, Z represents

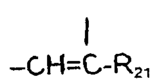,

$R_{20}$ and $R_{21}$ being identical or different and representing hydrogen, a halogen atom, a $C_1$-$C_4$ alkyl radical, or a $C_1$-$C_4$ alkoxy radical,
X represents -O- or -S-
Y represents a -CO- or -$CH_2$- radical, or a radical of formula

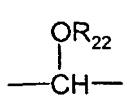 (73)

in which $R_{22}$ represents hydrogen, a $C_1$-$C_4$ alkyl radical or an acyl radical of formula:

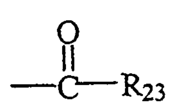 (74)

in which $R_{23}$ represents a $C_1$-$C_4$ alkyl radical, or Y represents

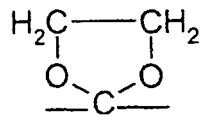

it being understood that the combination of the R, $R_1$ and Am groups contains 0, 1 or 2 groups (a), these benzofuran or benzothiophene derivatives being in the form of individual isomers or of a mixture thereof.

**2.** Benzofuran or benzothiophene derivatives according to Claim 1, in which Y represents a -CO- radical or Y represents

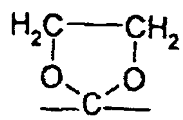

**3.** Benzofuran or benzothiophene derivatives according to Claim 1 or 2, in which R represents the isopropoxycarbonyl group.

**4.** Benzofuran or benzothiophene derivatives according to one of Claims 1 to 3, in which Am represents a diethylpiperidinyl group.

**5.** Benzofuran or benzothiophene derivatives according to one of Claims 1 to 4, in which Y represents the -CO- radical.

**6.** Benzofuran or benzothiophene derivatives according to one of Claims 1 to 5, in which

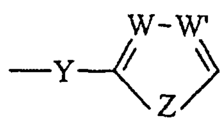

represents the benzoyl radical.

**7.** Benzofuran or benzothiophene derivatives according to one of Claims 1 to 6, in which:

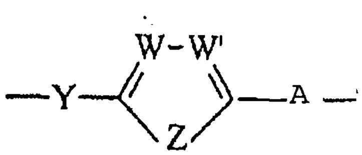

represents the 4-oxybenzoyl radical.

**8.** Benzofuran or benzothiophene derivatives according to one of Claims 1 to 7, in which the chain:

A-B-Am

is located at position 4.

**9.** Benzofuran or benzothiophene derivatives according to one of Claims 1 to 8, in which $R_1$ represents n-butyl, B represents the propylene group and Am represents a diethylpiperidinyl group.

**10.** Benzofuran or benzothiophene derivatives according to Claim 9, in which the diethylpiperidinyl group is the 3,5-diethylpiperidinyl group.

**11.** Benzofuran or benzothiophene derivatives according to one of Claims 1 to 10, in which the pharmaceutically acceptable salt is chosen from oxalate, maleate, fumarate, methanesulfonate, benzoate, ascorbate, pamoate, succinate, hexamate, bismethylenesalicylate, ethanedisulfonate, acetate, propionate, tartrate, salicylate, citrate, gluconate, lactate, malate, cinnamate, mandelate, citraconate, aspartate, palmitate, stearate, itaconate, glycolate, p-aminobenzoate, glutamate, benzenesulfonate, p-toluenesulfonate and theophyllineacetate salts and the salts formed from an amino acid, such as the lysine or histidine salt.

**12.** Benzofuran or benzothiophene derivatives according to any one of Claims 1 to 10, in which the pharmaceutically acceptable salt is chosen from the hydrochloride, hydrobromide, sulfate, sulfamate, phosphate and nitrate salts.

**13.** Process for the preparation of benzofuran or benzothiophene derivatives according to Claim 1, in which Y represents the -CO- group, $R_1$, ($Am_1$) and ($Am_2$) comprise no carboxylic or alkali metal carboxylate group, $R_{16}$ and/or $R_{17}$ and/or $R_{18}$ are different from the amino group or from a $C_1$-$C_4$ alkylsulfonamido group and R represents a cyano or hydroxymethyl group, the group (k) or a group (a) in which $R_5$ represents a $C_1$-$C_{10}$ alkyl or $C_3$-$C_6$ cycloalkyl group, **characterized in that** a ketone derivative of general formula:

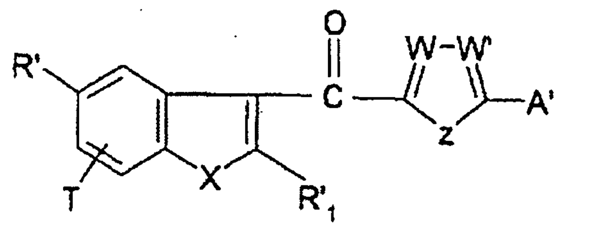

(2)

in which $R'_1$ represents a $C_1$-$C_6$ alkyl group or a group (m) comprising no carboxylic or alkali metal carboxylate group, T, W, W', X and Z have the same meaning as in Claim 1, A' represents OH, SH or $NH_2$ and R' represents a cyano or hydroxymethyl group, the group (k) or a $-CO_2R''_5$ group in which $R''_5$ represents a $C_1$-$C_{10}$ alkyl or $C_3$-$C_6$ cycloalkyl radical, is reacted, in the presence of a basic agent, with a compound of general formula:

$R_{24}$-B-Am'    (3)

in which Am' represents a group ($Am_1$) or ($Am_2$) as defined in Claim 1, this group comprising no carboxylic or alkali metal carboxylate group, or else Am' represents a group ($Am_3$) as defined in Claim 1 in which $R_{16}$ and/or $R_{17}$ and/or $R_{18}$ are different from an amino group or a $C_1$-$C_4$ alkylsulfonamido group, B has the same meaning as in Claim 1 and $R_{24}$ represents:

- a halogen atom, or a $C_1$-$C_4$ alkylsulfonyloxy or $C_6$-$C_{10}$ arylsulfonyloxy radical, which makes it possible to obtain, in the free base form, the desired compounds in which A represents -O- or -S-,
the compounds in the free base form thus obtained possibly being, if necessary, treated with a suitable organic or inorganic acid, to form a pharmaceutically acceptable salt.

**14.** Process for the preparation of benzofuran or benzothiophene derivatives according to Claim 1, in which Y represents the -CO- group, $R_1$, ($Am_1$) and ($Am_2$) comprise no carboxylic or alkali metal carboxylate group, $R_{16}$ and/or $R_{17}$ and/or $R_{18}$ are different from the amino group or from a $C_1$-$C_4$ alkylsulfonamido group and R represents the cyano group, the group (k), an $R_4$-O-N=CH-group or a group (a) in which $R_5$ represents a $C_1$-$C_{10}$ alkyl or $C_3$-$C_6$ cycloalkyl group, **characterized in that** a compound of general formula:

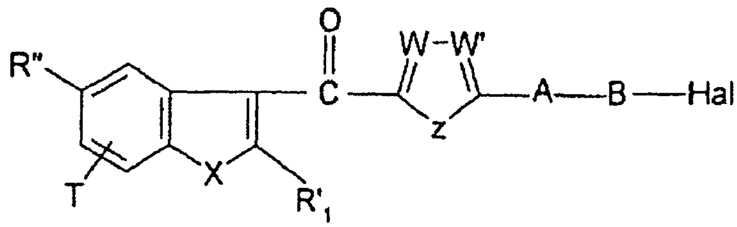

(4)

in which A, B, T, X, W, W' and Z have the same meaning as in Claim 1, $R'_1$ represents a $C_1$-$C_6$ alkyl group or a group (m) comprising no carboxylic or alkali metal carboxylate group and R" represents the cyano group, the group (k), an $R_4$-O-N=CH- group or a -$CO_2R"_5$ group in which $R"_5$ represents a $C_1$-$C_{10}$ alkyl or $C_3$-$C_6$ cycloalkyl group and Hal represents a halogen atom, is reacted with a compound of general formula:

H-Am' (5)

optionally in the form of a salt, in which Am' represents a group ($Am_1$) or ($Am_2$) as defined in Claim 1, this group comprising no carboxylic or alkali metal carboxylate group, or else Am' represents a group ($Am_3$) as defined in Claim 1 in which $R_{16}$ and/or $R_{17}$ and/or $R_{18}$ are different from an amino group or a $C_1$-$C_4$ alkylsulfonamido group, the reaction taking place in the presence of a basic agent or an excess of amine of formula (5) in the basic form, which gives the desired compounds in the free base form, which base can be reacted, if necessary, with a suitable organic or inorganic acid, to form a pharmaceutically acceptable salt.

**15.** Process for the preparation of benzofuran or benzothiophene derivatives according to Claim 1, in which Y represents the -CO- group, $R_1$, ($Am_1$) and ($Am_2$) comprise no carboxylic or alkali metal carboxylate group, $R_{16}$ and/or $R_{17}$ and/or $R_{18}$ are different from the amino group or from a $C_1$-$C_4$ alkylsulfonamido group and R represents the cyano group, an $R_4$-O-N=CH- group, a group (a) in which $R_5$ represents a $C_1$-$C_{10}$ alkyl or $C_3$-$C_6$ cycloalkyl group or else the group (k), **characterized in that** a compound of general formula:

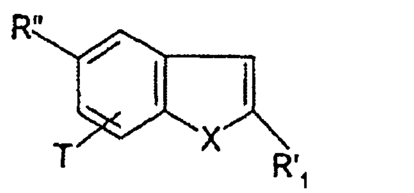

(6)

in which T and X have the same meaning as in Claim 1, R" represents the cyano group, the group (k), an $R_4$-O-N=CH- group or a -$CO_2R"_5$ group in which $R"_5$ represents a $C_1$-$C_{10}$ alkyl or $C_3$-$C_6$ cycloalkyl group and $R'_1$ represents a $C_1$-$C_6$ alkyl group or a group (m) comprising no carboxylic or alkali metal carboxylate group, is reacted with a halide of general formula:

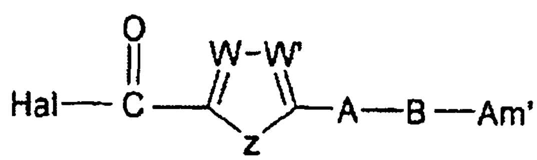

(7)

in which A, B, W, W' and Z have the same meaning as in Claim 1, Hal represents a halogen atom and Am' represents a group ($Am_1$) or ($Am_2$) as defined in Claim 1, this group comprising no carboxylic or alkali metal carboxylate group, or else Am' represents a group ($Am_3$) as defined in Claim 1 in which $R_{16}$ and/or $R_{17}$ and/or $R_{18}$ are different from an amino group or a $C_1$-$C_4$ alkylsulfonamido group, the reaction optionally taking place in the presence of a Lewis acid, which gives the desired compounds in the free base form, which base can be reacted, if necessary, with an organic or inorganic acid, to form a pharmaceutically acceptable salt.

**16.** Process for the preparation of benzofuran or benzothiophene derivatives according to Claim 1, in which Y represents the -CO- group, $R_1$, ($Am_1$) and ($Am_2$) comprise no carboxylic or alkali metal carboxylate group, $R_{16}$ and/or $R_{17}$ and/or $R_{18}$ are different from the amino group or from a $C_1$-$C_4$ alkylsulfonamido group and R represents the group (j), **characterized in that** a compound of formula:

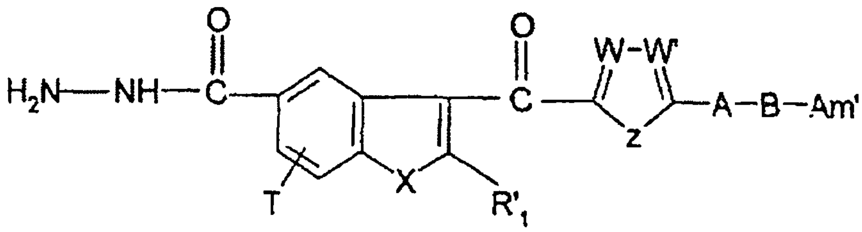

(9)

in which A, B, T, W, W', X and Z have the same meaning as in Claim 1, $R'_1$ represents a $C_1$-$C_6$ alkyl group or a group (m) comprising no carboxylic or alkali metal carboxylate group and Am' represents a group ($Am_1$) or ($Am_2$) as defined in Claim 1, this group comprising no carboxylic or alkali metal carboxylate group, or else Am' represents a group ($Am_3$) as defined in Claim 1 in which $R_{16}$ and/or $R_{17}$ and/or $R_{18}$ are different from an amino group or a $C_1$-$C_4$ alkylsulfonamido group, is reacted with phosgene, which gives the desired compounds in the hydrochloride form, which hydrochloride can be treated, if necessary, with a basic agent, such as an alkali metal hydroxide or an alkali metal carbonate, which gives the desired compounds in the free base form, which base can be reacted, if necessary, with an organic or inorganic acid, to form a pharmaceutically acceptable salt.

**17.** Process for the preparation of benzofuran or benzothiophene derivatives according to Claim 1, in which Y represents the -CO- group, $R_1$, ($Am_1$) and ($Am_2$) comprise no carboxylic or alkali metal carboxylate group, $R_{16}$ and/or $R_{17}$ and/or $R_{18}$ are different from the amino group or from a $C_1$-$C_4$ alkylsulfonamido group and R represents a group (b) in which $R'_5$ represents a group (c) of the primary dialkylaminoalkyl type, **characterized in that** a compound of formula:

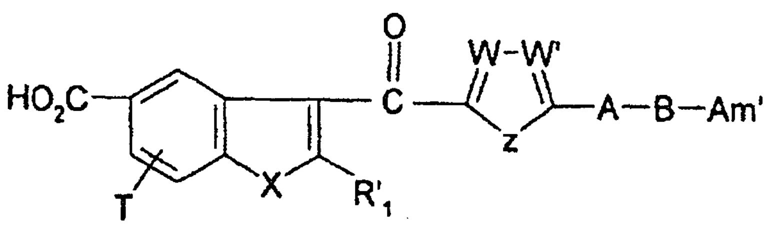

(10)

in which A, B, T, X, W, W', X and Z have the same meaning as in Claim 1, $R'_1$ represents a $C_1$-$C_6$ alkyl group or a group (m) comprising no carboxylic or alkali metal carboxylate group and Am' represents a group ($Am_1$) or ($Am_2$) as defined in Claim 1, this group comprising no carboxylic or alkali metal carboxylate group, or else Am' represents a group ($Am_3$) as defined in Claim 1 in which $R_{16}$ and/or $R_{17}$ and/or $R_{18}$ are different from an amino group or a $C_1$-$C_4$ alkylsulfonamido group, after protection of the amino functional group when Am' represents a group ($Am_1$) in which $R_2$ represents hydrogen, is reacted with an alcohol of general formula:

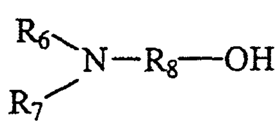

(11)

in which $R_6$ and $R_7$ have the same meaning as in Claim 1 and $R_8$ represents a linear $C_1$-$C_8$ alkylene group, the reaction taking place in the presence of carbonyldiimidazole and 1,8-diazabicyclo[5.4.0]undec-7-ene, and then, if necessary, the compound formed is deprotected, which gives the desired compounds in the free base form, which

base can be treated, if necessary, with a suitable organic or inorganic acid, to produce a pharmaceutically acceptable salt.

**18.** Process for the preparation of benzofuran or benzothiophene derivatives according to Claim 1, in which Y represents the -CO- group, $R_1$, ($Am_1$) and ($Am_2$) comprise no carboxylic or alkali metal carboxylate group, $R_{16}$ and/or $R_{17}$ and/or $R_{18}$ are different from the amino group or from a $C_1$-$C_4$ alkylsulfonamido group and R represents a group (b) in which R'$_5$ represents a group (c) of the secondary or tertiary dialkylaminoalkyl type, **characterized in that** a compound of formula:

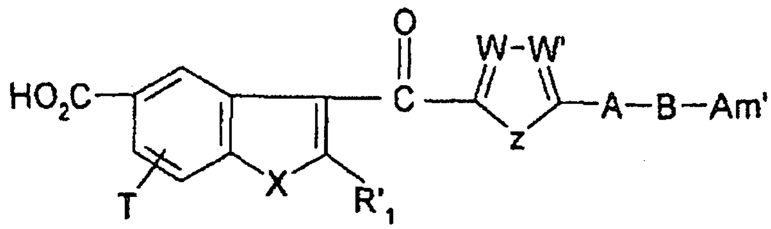

(10)

in which A, B, T, X, W, W' and Z have the same meaning as in Claim 1, R'$_1$ represents a $C_1$-$C_6$ alkyl group or a group (m) comprising no carboxylic or alkali metal carboxylate group and Am' represents a group ($Am_1$) or ($Am_2$) as defined in Claim 1, this group comprising no carboxylic or alkali metal carboxylate group, or else Am' represents a group ($Am_3$) as defined in Claim 1 in which $R_{16}$ and/or $R_{17}$ and/or $R_{18}$ are different from an amino group or a $C_1$-$C_4$ alkylsulfonamido group, after protection of the amino functional group when Am' represents a group ($Am_1$) in which $R_2$ represents hydrogen, is reacted with a halogenating agent, to produce an acyl halide, which is subsequently treated with an alcohol of formula:

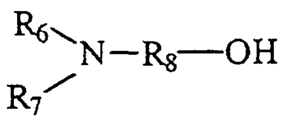

(11)

in which $R_6$ and $R_7$ have the same meaning as in Claim 1 and $R_8$ represents a secondary or tertiary $C_2$-$C_6$ alkylene group, and then, if necessary, the compound formed is deprotected, which gives the desired compounds in the hydrohalide form or in the free base form when the compound of formula (10) is in excess, which hydrohalide can be treated, if necessary, with a basic agent, such as an alkali metal hydroxide or an alkali metal carbonate, to produce the desired compounds in the free base form, the free base thus formed possibly being, if necessary, treated with a suitable organic or inorganic acid, to produce a pharmaceutically acceptable salt.

**19.** Process for the preparation of benzofuran or benzothiophene derivatives according to Claim 1, in which Y represents the -CO- group, $R_1$, ($Am_1$) and ($Am_2$) comprise no carboxylic or alkali metal carboxylate group, $R_{16}$ and/or $R_{17}$ and/or $R_{18}$ are different from the amino group or from a $C_1$-$C_4$ alkylsulfonamido group and R represents either a group (a) in which $R_5$ represents a $C_1$-$C_{10}$ alkyl or $C_3$-$C_6$ cycloalkyl group, or a group (b) in which R'$_5$ represents a piperidinyl group optionally N-substituted with a $C_1$-$C_4$ alkyl group or in which R'$_5$ represents a group (d) comprising no carboxylic or alkali metal carboxylate group, **characterized in that** a compound of formula:

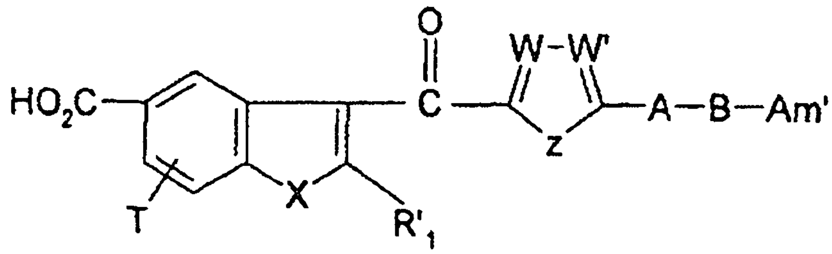

(10)

in which A, B, T, X, W, W' and Z have the same meaning as in Claim 1, $R'_1$ represents a $C_1$-$C_6$ alkyl group or a group (m) comprising no carboxylic or alkali metal carboxylate group and Am' represents a group ($Am_1$) or ($Am_2$) as defined in Claim 1, this group comprising no carboxylic or alkali metal carboxylate group, or else Am' represents a group ($Am_3$) as defined in Claim 1 in which $R_{16}$ and/or $R_{17}$ and/or $R_{18}$ are different from an amino group or a $C_1$-$C_4$ alkylsulfonamido group, after protection of the amino functional group when Am' represents a group ($Am_1$) in which $R_2$ represents hydrogen, is reacted with a halogenating agent, to produce an acyl halide, which is subsequently treated with an alcohol of general formula:

$R'''_5$-OH (12)

in which $R'''_5$ represents a $C_1$-$C_{10}$ alkyl or $C_3$-$C_6$ cycloalkyl group or a group (b) in which $R'_5$ represents a piperidinyl group optionally N-substituted with a $C_1$-$C_4$ alkyl group or $R'_5$ represents a group (d) comprising no carboxylic or alkali metal carboxylate group, and then, if necessary, the compound formed is deprotected, which gives the desired compounds in the hydrohalide form or in the free base form when the compound of formula (10) is in excess, which hydrohalide can be treated, if desired, with a basic agent, such as an alkali metal hydroxide or an alkali metal carbonate, to produce the desired compounds in the free base form, the free base thus formed possibly being, if necessary, treated with a suitable organic or inorganic acid, to produce a pharmaceutically acceptable salt.

**20.** Process for the preparation of benzofuran or benzothiophene derivatives according to Claim 1, in which Y represents the -CO- group, $R_1$, ($Am_1$) and ($Am_2$) comprise no carboxylic or alkali metal carboxylate group, $R_{16}$ and/or $R_{17}$ and/or $R_{18}$ are different from the amino group or from a $C_1$-$C_4$ alkylsulfonamido group and R represents a group (e) in which $R_{10}$ represents a group (f) comprising no carboxylic or alkali metal carboxylate group, **characterized in that** a compound of formula:

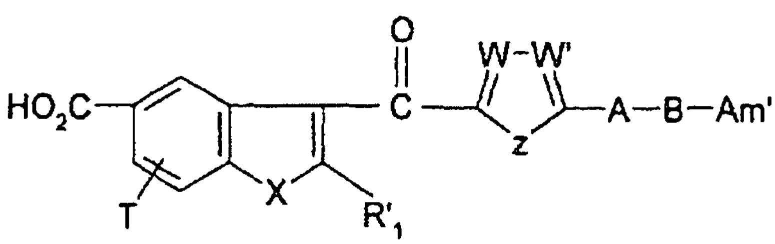

(10)

in which A, B, T, X, W, W' and Z have the same meaning as in Claim 1, $R'_1$ represents a $C_1$-$C_6$ alkyl group or a group (m) comprising no carboxylic or alkali metal carboxylate group and Am' represents a group ($Am_1$) or ($Am_2$) as defined in Claim 1, this group comprising no carboxylic or alkali metal carboxylate group, or else Am' represents a group ($Am_3$) as defined in Claim 1 in which $R_{16}$ and/or $R_{17}$ and/or $R_{18}$ are different from an amino group or a $C_1$-$C_4$ alkylsulfonamido group, after protection of the amine functional group when Am' represents a group ($Am_1$) in which $R_2$ represents hydrogen, is reacted with a halogenating agent, to produce an acyl chloride, which is subsequently treated with a compound of formula:

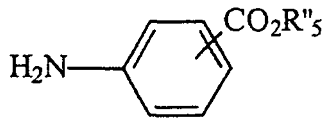

(13)

in which $R''_5$ represents a $C_1$-$C_{10}$ alkyl or $C_3$-$C_6$ cycloalkyl radical, and then, if necessary, the compound formed is deprotected, which gives, in the free base form, the desired compounds in which $R_{10}$ represents a group (f) in which the $R_{11}$ group represents a group (a) in which $R_5$ represents a $C_1$-$C_4$ alkyl or $C_3$-$C_6$ cycloalkyl group, the free base thus formed possibly being, if necessary, treated with a suitable organic or inorganic acid, to form a pharmaceutically acceptable salt.

**21.** Process for the preparation of benzofuran or benzothiophene derivatives according to Claim 1, in which Y represents the -CO- group, $R_1$, ($Am_1$) and ($Am_2$) comprise no carboxylic or alkali metal carboxylate group, $R_{16}$ and/or $R_{17}$ and/or $R_{18}$ are different from the amino group or from a $C_1$-$C_4$ alkylsulfonamido group and R represents a group (e) in which $R_{10}$ represents a $C_1$-$C_4$ alkyl group, an amino group or a group (c), **characterized in that** a compound of formula:

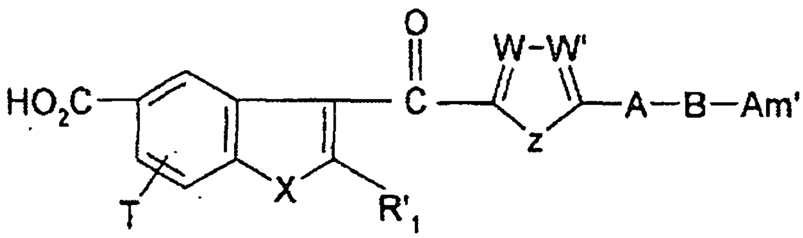

(10)

in which A, B, T, X, W, W' and Z have the same meaning as in Claim 1, $R'_1$ represents a $C_1$-$C_6$ alkyl group or a group (m) comprising no carboxylic or alkali metal carboxylate group and Am' represents a group ($Am_1$) or ($Am_2$) as defined in Claim 1, this group comprising no carboxylic or alkali metal carboxylate group, or else Am' represents a group ($Am_3$) as defined in Claim 1 in which $R_{16}$ and/or $R_{17}$ and/or $R_{18}$ are different from an amino group or a $C_1$-$C_4$ alkylsulfonamido group, after protection of the amine functional group when Am' represents a group ($Am_1$) in which $R_2$ represents hydrogen, is reacted with a halogenating agent, to produce an acyl halide, which is subsequently treated with an amine of general formula:

$$R'_{10}\text{-}NH_2 \qquad (14)$$

or

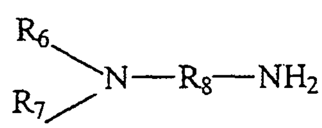

(15)

in which $R_6$, $R_7$ and $R_8$ have the same meaning as in Claim 1 and $R'_{10}$ represents a $C_1$-$C_4$ alkyl or amino radical, and then, if necessary, the compound formed is deprotected, which gives, optionally after basic treatment, the desired compound of formula (1) in the hydrohalide form or in the free base form when the compound of formula (10) is in excess, which hydrohalide can be treated, if necessary, with a basic agent, such as an alkali metal hydroxide or an alkali metal carbonate, to produce the desired compounds in the free base form, which base can, if necessary, be reacted with a suitable organic or inorganic acid, to form a pharmaceutically acceptable salt.

**22.** Process for the preparation of benzofuran or benzothiophene derivatives according to Claim 1, in which Y represents the -CO- group, $R_1$, ($Am_1$) and ($Am_2$) comprise no carboxylic or alkali metal carboxylate group, $R_{16}$ and/or $R_{17}$ and/or $R_{18}$ are different from the amino group or from a $C_1$-$C_4$ alkylsulfonamido group and R represents a group (e) in which $R_{10}$ represents a group (g) comprising no carboxylic or alkali metal carboxylate group, **characterized in that** a compound of formula:

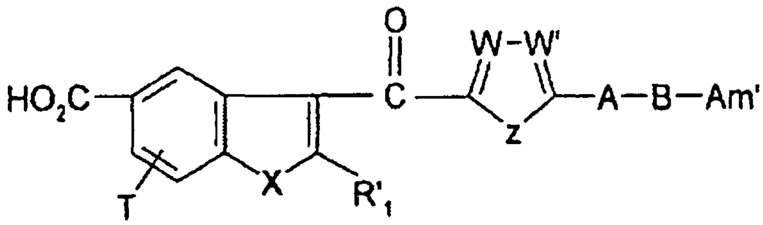

(10)

in which A, B, T, X, W, W' and Z have the same meaning as in Claim 1, R'$_1$ represents a $C_1$-$C_6$ alkyl group or a group (m) comprising no carboxylic or alkali metal carboxylate group and Am' represents a group ($Am_1$) or ($Am_2$) as defined in Claim 1, this group comprising no carboxylic or alkali metal carboxylate group, or else Am' represents a group ($Am_3$) as defined in Claim 1 in which $R_{16}$ and/or $R_{17}$ and/or $R_{18}$ are different from an amino group or a $C_1$-$C_4$ alkylsulfonamido group, after protection of the amine functional group when Am' represents a group ($Am_1$) in which $R_2$ represents hydrogen, is reacted with a salt of a compound of general formula:

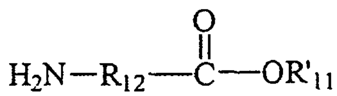

(16)

in which $R_{12}$ has the same meaning as in Claim 1 and R'$_{11}$ represents a $C_1$-$C_4$ alkyl or $C_3$-$C_6$ cycloalkyl radical, the reaction taking place in the presence of an acid scavenger, and then, if necessary, the compound formed is deprotected, which gives, in the free base form, the desired compounds in which $R_{10}$ represents a group (g) in which the $R_{11}$ group represents a group (a) in which $R_5$ represents a $C_1$-$C_4$ alkyl or $C_3$-$C_6$ cycloalkyl group, the free base thus formed possibly being, if necessary, treated with a suitable organic or inorganic acid, to produce a pharmaceutically acceptable salt.

**23.** Process for the preparation of benzofuran or benzothiophene derivatives according to Claim 1, in which Y represents the -CO- group, $R_1$, ($Am_1$) and ($Am_2$) comprise no carboxylic or alkali metal carboxylate group, $R_{16}$ and/or $R_{17}$ and/or $R_{18}$ are different from the amino group or from a $C_1$-$C_4$ alkylsulfonamido group and R represents a group (h), **characterized in that** a compound of formula:

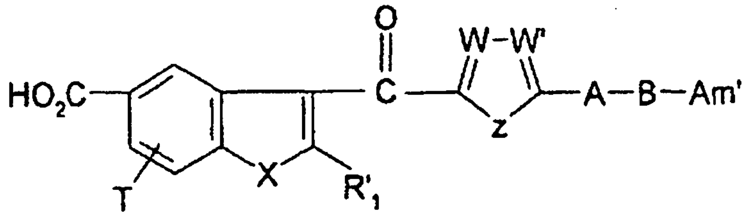

(10)

in which A, B, T, X, W, W' and Z have the same meaning as in Claim 1, R'$_1$ represents a $C_1$-$C_6$ alkyl group or a group (m) comprising no carboxylic or alkali metal carboxylate group and Am' represents a group ($Am_1$) or ($Am_2$)

as defined in Claim 1, this group comprising no carboxylic or alkali metal carboxylate group, or else Am' represents a group ($Am_3$) as defined in Claim 1 in which $R_{16}$ and/or $R_{17}$ and/or $R_{18}$ are different from an amino group or a $C_1$-$C_4$ alkylsulfonamido group, after protection of the amine functional group when Am' represents a group ($Am_1$) in which $R_2$ represents hydrogen, is reacted with a halogenating agent, to produce an acyl halide, which is subsequently treated with an amine of general formula:

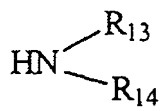

(17)

in which $R_{13}$ and $R_{14}$ have the same meaning as in Claim 1, and then, if necessary, the compound formed is deprotected, which gives a salt of the desired compound, which is treated with a suitable basic agent, to produce, in the free base form, the desired compounds, the free base thus formed possibly being, if necessary, treated with an organic or inorganic acid, to produce a pharmaceutically acceptable salt.

**24.** Process for the preparation of benzofuran or benzothiophene derivatives according to Claim 1, in which Y represents the -CO- group, $R_1$, ($Am_1$) and ($Am_2$) comprise no carboxylic or alkali metal carboxylate group, $R_{16}$ and/or $R_{17}$ and/or $R_{18}$ are different from the amino group or from a $C_1$-$C_4$ alkylsulfonamido group and R represents a group (e) in which $R_{10}$ represents the hydroxyl group, **characterized in that** a compound of formula:

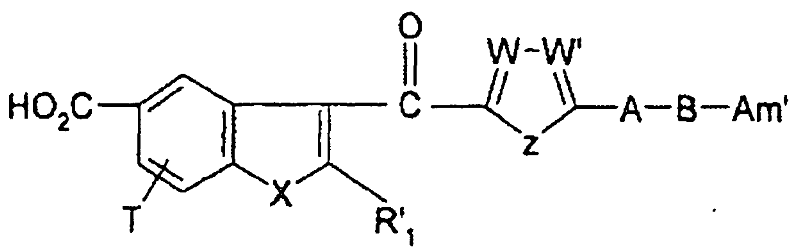

(10)

in which A, B, T, X, W, W' and Z have the same meaning as in Claim 1, R'$_1$ represents a $C_1$-$C_6$ alkyl group or a group (m) comprising no carboxylic or alkali metal carboxylate group and Am' represents a group ($Am_1$) or ($Am_2$) as defined in Claim 1, this group comprising no carboxylic or alkali metal carboxylate group, or else Am' represents a group ($Am_3$) as defined in Claim 1 in which $R_{16}$ and/or $R_{17}$ and/or $R_{18}$ are different from an amino group or a $C_1$-$C_4$ alkylsulfonamido group, after protection of the amine functional group when Am' represents a group ($Am_1$) in which $R_2$ represents hydrogen, is reacted with a benzyloxyamine salt, in the presence of an acid scavenger and of benzotriazol-1-yloxytris(dimethylamino)phosphonium hexafluorophosphate, and then, if necessary, the compound formed is deprotected, which gives benzyloxyaminocarbonyl derivatives, which are hydrogenated in the presence of a suitable catalyst, to produce, in the free base form, the desired compounds, which are treated, if necessary, with a suitable organic or inorganic acid, to form a pharmaceutically acceptable salt.

**25.** Process according to one of Claims 18 to 24, **characterized in that** the protection of the amine functional group is carried out by treatment by means of 9-fluorenylmethyl chloroformate and the deprotection is carried out by treatment with a secondary amine.

**26.** Process for the preparation of benzofuran or benzothiophene derivatives according to Claim 1, in which Y represents the -CO- group, $R_1$, ($Am_1$) and ($Am_2$) comprise no carboxylic or alkali metal carboxylate group, $R_{16}$ and/or $R_{17}$ and/or $R_{18}$ are different from the amino group or from a $C_1$-$C_4$ alkylsulfonamido group and R represents a group (e) in which $R_{10}$ represents hydrogen, **characterized in that** a compound of formula:

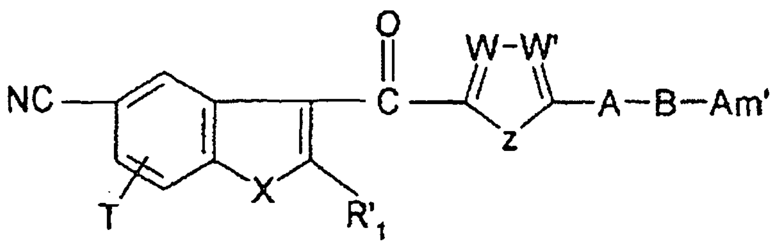

(18)

in which A, B, T, X, W, W' and Z have the same meaning as in Claim 1, $R'_1$ represents a $C_1$-$C_6$ alkyl group or a group (m) comprising no carboxylic or alkali metal carboxylate group and Am' represents a group ($Am_1$) or ($Am_2$) as defined in Claim 1, this group comprising no carboxylic or alkali metal carboxylate group, or else Am' represents a group ($Am_3$) as defined in Claim 1 in which $R_{16}$ and/or $R_{17}$ and/or $R_{18}$ are different from an amino group or a $C_1$-$C_4$ alkylsulfonamido group, is hydrolyzed in the presence of a strong acid, which gives the desired compounds in the free base form,
which base can be treated, if necessary, with a suitable organic or inorganic acid, to form a pharmaceutically acceptable salt.

**27.** Process for the preparation of benzofuran or benzothiophene derivatives according to Claim 1, in which Y represents the -CO- group, $R_1$, ($Am_1$) and ($Am_2$) comprise no carboxylic or alkali metal carboxylate group, $R_{16}$ and/or $R_{17}$ and/or $R_{18}$ are different from the amino group or from a $C_1$-$C_4$ alkylsulfonamido group and R represents the group (1), **characterized in that** a compound of formula:

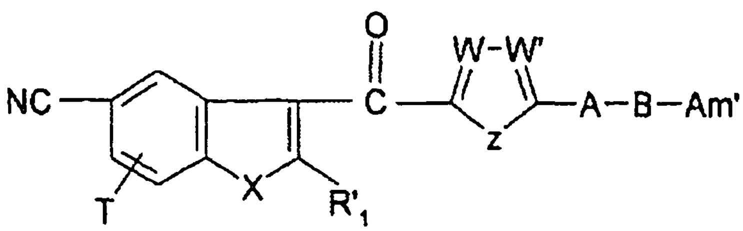

(18)

in which A, B, T, X, W, W' and Z have the same meaning as in Claim 1, $R'_1$ represents a $C_1$-$C_6$ alkyl group or a group (m) comprising no carboxylic or alkali metal carboxylate group and Am' represents a group ($Am_1$) or ($Am_2$) as defined in Claim 1, this group comprising no carboxylic or alkali metal carboxylate group, or else a group ($Am_3$) as defined in Claim 1 in which $R_{16}$ and/or $R_{17}$ and/or $R_{18}$ are different from an amino group or a $C_1$-$C_4$ alkylsulfonamido group, is reacted with tributylazidotin, which gives the desired compounds in the free base form, which base can be treated, if necessary, with a suitable organic or inorganic acid, to form a pharmaceutically acceptable salt.

**28.** Process for the preparation of benzofuran or benzothiophene derivatives according to Claim 1, in which Y represents the -CO- group, $R_1$ comprises no carboxylic or alkali metal carboxylate group and Am represents a group ($Am_1$) comprising no carboxylic or alkali metal carboxylate group and in which $R_{16}$ and/or $R_{17}$ and/or $R_{18}$ represent the amino group, or else Am represents a group ($Am_3$) in which $R_{16}$ and/or $R_{17}$ and/or $R_{18}$ represent the amino group, **characterized in that** a nitro compound of formula:

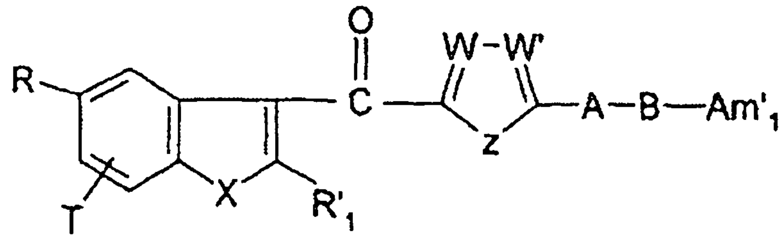

(19)

in which A, B, T, W, W', X and Z have the same meaning as in Claim 1, R has the same meaning as in Claim 1 but comprises no carboxylic or alkali metal carboxylate group, $R'_1$ represents a $C_1$-$C_6$ alkyl group or a group (m) comprising no carboxylic or alkali metal carboxylate group and $Am'_1$ represents either a group ($Am_1$) comprising no carboxylic or alkali metal carboxylate group and in which $R_{16}$ and/or $R_{17}$ and/or $R_{18}$ represent the nitro group, or a group ($Am_3$) in which $R_{16}$ and/or $R_{17}$ and/or $R_{18}$ represent the nitro group, is hydrogenated in the presence of a suitable catalyst, which gives, in the free base form, the desired compounds, which can be reacted, if necessary, with a suitable organic or inorganic acid, to form a pharmaceutically acceptable salt.

**29.** Process for the preparation of benzofuran or benzothiophene derivatives according to Claim 1, in which Y represents the -CO- group, $R_1$ comprises no carboxylic or alkali metal carboxylate group and Am represents a group ($Am_1$) comprising no carboxylic or alkali metal carboxylate group and in which $R_{16}$ and/or $R_{17}$ and/or $R_{18}$ represent a $C_1$-$C_4$ alkylsulfonamido group, or else Am represents a group ($Am_3$) in which $R_{16}$ and/or $R_{17}$ and/or $R_{18}$ represent a $C_1$-$C_4$ alkylsulfonamido group, **characterized in that** an amino compound of formula:

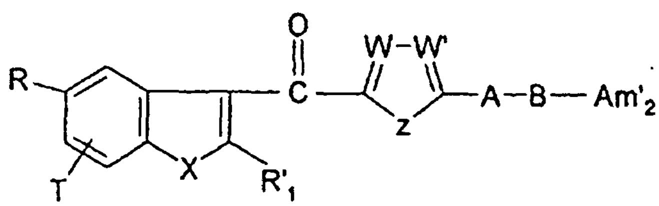

(20)

in which A, B, T, W, W', X and Z have the same meaning as in Claim 1, R has the same meaning as in Claim 1 but comprises no carboxylic or alkali metal carboxylate group, $R'_1$ represents a $C_1$-$C_6$ alkyl group or a group (m) comprising no carboxylic or alkali metal carboxylate group and $Am'_2$ represents either a group ($Am_1$) comprising no carboxylic or alkali metal carboxylate group and in which $R_{16}$ and/or $R_{17}$ and/or $R_{18}$ represent the amino group, or a group ($Am_3$) in which $R_{16}$ and/or $R_{17}$ and/or $R_{18}$ represent the amino group, is reacted with a halide of general formula:

$Hal-SO_2-R'_{16}$ (21)

or an anhydride of general formula:

$(R'_{16}SO_2)_2O$ (22)

in which $R'_{16}$ represents a linear or branched $C_1$-$C_4$ alkyl radical, optionally in the presence of an acid acceptor, which gives, in the free base form, the desired compounds, which can be reacted, if necessary, with a suitable organic or inorganic acid, to form a pharmaceutically acceptable salt.

**30.** Process for the preparation of benzofuran or benzothiophene derivatives according to Claim 1, in which Y represents the -CO- group and one or two of the groups R, $R_1$, and ($Am_1$) or ($Am_2$) comprise a $-CO_2R_5$ group in which $R_5$ represents hydrogen or an alkali metal atom, the other group (s) being different from a $-CO_2R_5$ group in which $R_5$

represents a $C_1$-$C_{10}$ alkyl or $C_3$-$C_6$ cycloalkyl radical, **characterized in that** a compound of formula:

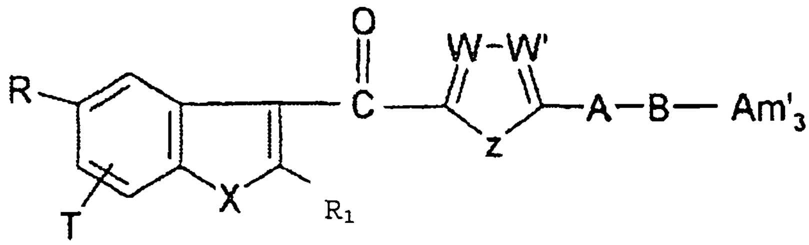

(23)

in which A, B, R, $R_1$, T, W, W', X and Z have the same meaning as in Claim 1, $Am'_3$ represents a group ($Am_1$) or ($Am_2$) as defined in Claim 1, and R, $R_1$, and ($Am_1$) or ($Am_2$) are such that one or two of them comprise (s) a $-CO_2R''_5$ group in which $R''_5$ represents a $C_1$-$C_{10}$ alkyl or $C_3$-$C_6$ cycloalkyl radical, the other group(s) being different from a $-CO_2R_5$ group in which $R_5$ represents a $C_1$-$C_{10}$ alkyl or $C_3$-$C_6$ cycloalkyl radical, is saponified in the presence of an alkali metal hydroxide, which gives, in the free base form, the desired compounds of formula (1) in which one or two of the groups R, $R_1$, and ($Am_1$) or ($Am_2$) comprise (s) a $-CO_2R_5$ group in which $R_5$ represents an alkali metal atom, which compound can be treated, if necessary, with a strong acid, which gives, in the free base form, the desired compounds of formula (1) in which $R_5$ represents hydrogen, the free bases thus formed possibly being, if necessary, treated with a suitable organic or inorganic acid, to produce a pharmaceutically acceptable salt.

**31.** Process for the preparation of benzofuran or benzothiophene derivatives according to Claim 1, in which Y represents the -CO- group and one of two of the groups R, $R_1$, and ($Am_1$) or ($Am_2$) comprises a $-CO_2R_5$ group in which $R_5$ represents hydrogen or an alkali metal atom, and the other comprises a $-CO_2R_5$ group in which $R_5$ represents a $C_1$-$C_{10}$ alkyl or $C_3$-$C_6$ cycloalkyl group, the third group being different from a $-CO_2R_5$ group in which $R_5$ represents a $C_1$-$C_{10}$ alkyl or $C_3$-$C_6$ cycloalkyl group,
**characterized in that**:

- a compound of formula:

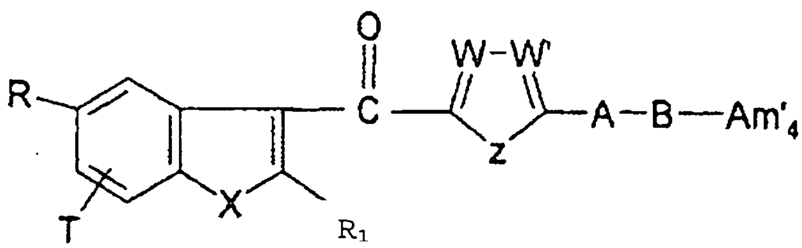

(24)

in which A, B, R, $R_1$, T, W, W', X and Z have the same meaning as in Claim 1, $Am'_4$ represents a group ($Am_1$) or ($Am_2$) as defined in Claim 1, and R, $R_1$, and ($Am_1$) or ($Am_2$) are such that one of them comprises a $-CO_2R''_5$ group in which $R''_5$ represents a $C_1$-$C_{10}$ alkyl or $C_3$-$C_6$ cycloalkyl group and another of them comprises a benzyloxycarbonyl group, the third group being different from a $-CO_2R_5$ group in which $R_5$ represents a $C_1$-$C_{10}$ alkyl or $C_3$-$C_6$ cycloalkyl group, is hydrogenated in the presence of a suitable catalyst;

- a compound of formula:

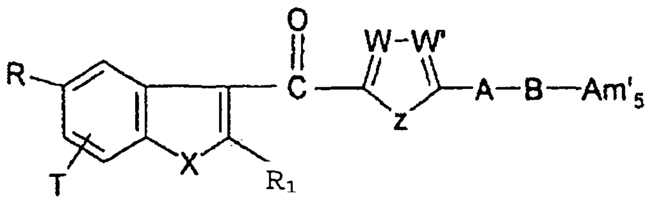

(25)

in which A, B, R, $R_1$, T, W, W', X and Z have the same meaning as in Claim 1, $Am'_5$ represents a group ($Am_1$) or ($Am_2$) as defined in Claim 1, and R, $R_1$, and ($Am_1$) or ($Am_2$) are such that one of them comprises a $-CO_2R''_5$ group in which $R''_5$ has the same meaning as above and another of them comprises a t-butoxycarbonyl group, the third group being different from a $-CO_2R_5$ group in which $R_5$ represents a $C_1$-$C_{10}$ alkyl or $C_3$-$C_6$ cycloalkyl group, is hydrolyzed in the presence of trifluoroacetic acid,
which makes it possible to produce the desired compounds of formula (1) in which one of two of the groups R, $R_1$, and ($Am_1$) or ($Am_2$) comprises a $-CO_2R_5$ group in which $R_5$ represents a $C_1$-$C_{10}$ alkyl or $C_3$-$C_6$ cycloalkyl group, and the other comprises a $-CO_2R_5$ group in which $R_5$ represents hydrogen, which compounds can be treated, if necessary, with a suitable basic agent, to produce, in the free base form, the desired compounds of formula (1) in which one of two of the groups R, $R_1$, and ($Am_1$) or ($Am_2$) comprises a $-CO_2R_5$ group in which $R_5$ represents an alkali metal atom, the other a $-CO_2R_5$ group in which $R_5$ represents a $C_1$-$C_{10}$ alkyl or $C_3$-$C_6$ cycloalkyl group, which compounds can themselves be treated, if necessary, with a strong acid, to produce, in the free base form, the desired compounds of formula (1) in which one of two of the groups R, $R_1$, and ($Am_1$) or ($Am_2$) comprises a carboxylic group, the other a $-CO_2R_5$ group in which $R_5$ represents a $C_1$-$C_{10}$ alkyl or $C_3$-$C_6$ cycloalkyl group, the free bases thus formed possibly being, if necessary, treated with a suitable organic or inorganic acid, to produce a pharmaceutically acceptable salt.

**32.** Process for the preparation of benzofuran or benzothiophene derivatives according to Claim 1, in which Y represents -CO-, R represents the cyano group and one of the groups $R_1$, ($Am_1$) or ($Am_2$) comprises a carboxylic group, **characterized in that** a compound of formula (1) according to Claim 1, in which Y represents the -CO- group, R represents the cyano group, and in which A, B, $R_1$, T, W, W' , X and Z have the same meaning as in Claim 1, Am represents a group ($Am_1$) or ($Am_2$) as defined in Claim 1 and $R_1$, ($Am_1$) and ($Am_2$) are such that one of them comprises a $-CO_2R''_5$ group in which $R''_5$ represents a $C_1$-$C_{10}$ alkyl or $C_3$-$C_6$ cycloalkyl radical, is treated by means of tributyltin oxide, to produce the desired compounds in the free base form, which base can be reacted, if necessary, with an organic or inorganic acid, to form a pharmaceutically acceptable salt.

**33.** Process for the preparation of benzofuran or benzothiophene derivatives according to Claim 1, in which Y represents the group

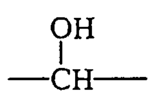

**characterized in that** a compound of formula (1) according to Claim 1, in which Y represents the -CO-group and in which A, B, Am, R, $R_1$, T, W, W', X and Z have the same meaning as in Claim 1, is reduced by means of an alkali metal borohydride, which gives the desired compounds in the free base form, which base can be reacted, if necessary, with an organic or inorganic acid, to produce a pharmaceutically acceptable salt.

**34.** Process for the preparation of benzofuran or benzothiophene derivatives according to Claim 1, in which Y represents the group

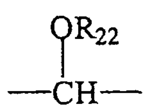

in which $R_{22}$ represents a $C_1$-$C_4$ alkyl radical, **characterized in that** a compound of formula (1) according to Claim 1, in which Y represents the -CHOH-group and in which A, B, Am, R, $R_1$, T, W, W', X and Z have the same meaning as in Claim 1, is reacted with an alkali metal alkoxide and then with a halide of formula:

$R_{23}$-Hal

in which Hal represents a halogen atom and $R_{23}$ represents a $C_1$-$C_4$ alkyl radical, which gives the desired compounds in the free base form, which base can be reacted, if necessary, with an organic or inorganic acid, to produce a pharmaceutically acceptable salt.

**35.** Process for the preparation of benzofuran or benzothiophene derivatives according to Claim 1, in which Y represents a group

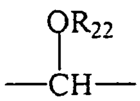

in which $R_{22}$ represents an acyl radical of formula -CO-$R_{23}$ in which $R_{23}$ represents a $C_1$-$C_4$ alkyl radical , **characterized in that** a compound of formula (1) according to Claim 1, in which Y represents the -CHOH-group and in which A, B, Am, R, $R_1$, T, W, W', X and Z have the same meaning as in Claim 1, is reacted with an acyl halide of formula:

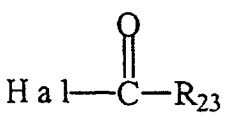

(27)

in which Hal represents a halogen atom and $R_{23}$ represents a $C_1$-$C_4$ alkyl radical, which gives the desired compounds in the free base form, which base can be reacted, if necessary, with an organic or inorganic acid, to form a pharmaceutically acceptable salt.

**36.** Process for the preparation of benzofuran or benzothiophene derivatives according to Claim 1, in which Y represents the -$CH_2$- group, **characterized in that** a compound of formula (1) according to Claim 1, in which Y represents the -CHOH- group and in which A, B, Am, R, $R_1$, T, W, W', X and Z have the same meaning as in Claim 1, is reduced by means of an alkali metal borohydride, in the presence of trifluoroacetic acid, which gives the desired compounds in the free base form, which base can be reacted, if necessary, with a suitable organic or inorganic acid, to form a pharmaceutically acceptable salt.

**37.** Medicinal product, **characterized in that** it comprises a benzofuran or benzothiophene derivative, or a pharmaceutically acceptable salt of the latter, according to any one of Claims 1 to 12.

**38.** Pharmaceutical or veterinary composition, **characterized in that** it comprises, as active principle, at least one benzofuran or benzothiophene derivative, or a pharmaceutically acceptable salt of the latter, according to one of Claims 1 to 12, in combination with a suitable excipient or pharmaceutical vehicle.

**39.** Pharmaceutical or veterinary composition according to Claim 38, for the treatment of pathological syndromes of the cardiovascular system.

**40.** Pharmaceutical or veterinary composition according to Claim 38 or 39, for the treatment of angina pectoris, hypertension, atrial arrhythmia, ventricular or supraventricular arrhythmia, cerebral circulatory insufficiency, heart failure, myocardial infarction, possibly complicated by heart failure, or for the prevention of post infarction mortality.

**41.** Pharmaceutical or veterinary composition according to one of Claims 38 to 40, **characterized in that** it comprises from 50 to 500 mg of active principle.

**42.** Use of at least one benzofuran or benzothiophene derivative, or of a pharmaceutically acceptable salt of the latter, according to one of Claims 1 to 12, for the production of a medicinal product intended for the treatment of pathological

syndromes of the cardiovascular system.

**Patentansprüche**

**1.** Benzofuran- oder Benzothiophenderivate der allgemeinen Formel:

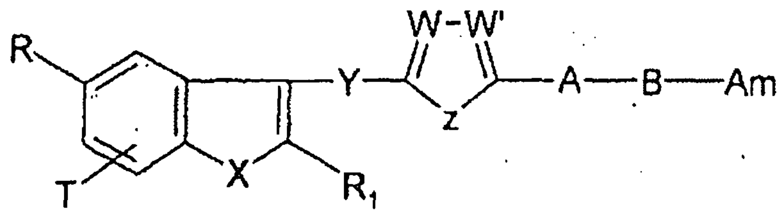

(1)

sowie pharmazeutisch unbedenkliche Salze davon,
wobei:

A für -O- oder -S- steht,
B für eine lineare oder verzweigte $C_1$-$C_5$-Alkylengruppe, die gegebenenfalls durch eine Hydroxylgruppe substituiert ist, steht,
T für Wasserstoff steht,
R für :

• eine Cyano- oder Hydroxymethylgruppe
• eine Oximgruppe der Formel:

$R_4$-O-N=CH-

worin $R_4$ eine $C_1$-$C_4$-Alkylgruppe bedeutet,
• eine Carboxylgruppe der allgemeinen Formel:

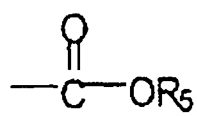

(a)

worin $R_5$ für Wasserstoff oder ein Alkalimetallatom, eine lineare oder verzweigte $C_1$-$C_{10}$-Alkylgruppe, eine $C_3$-$C_8$-Cycloalkylgruppe oder eine Gruppe der allgemeinen Formel:

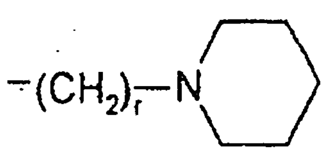

(a-1)

worin r 1 bis 4 bedeutet, steht,

• eine Carboxylgruppe der allgemeinen Formel:

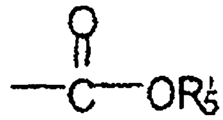

(b)

worin $R'_5$ für eine Piperidinylgruppe steht, die gegebenenfalls durch eine $C_1$-$C_4$-Gruppe oder eine der Gruppen der allgemeinen Formel:

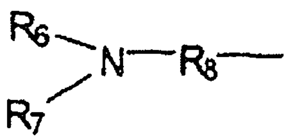

oder

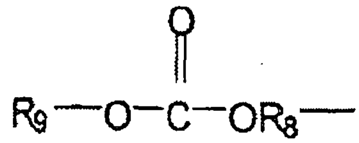

(c)

(d)

worin $R_6$ und $R_7$ gleich oder verschieden sind und für eine $C_1$-$C_4$-Alkylgruppe stehen, $R_8$ für eine lineare oder verzweigte $C_1$-$C_6$-Alkylengruppe steht und $R_9$ für Wasserstoff, ein Alkalimetall oder eine $C_1$-$C_4$-Alkylgruppe steht, N-substituiert ist,

- eine Aminocarbonylgruppe der Formel:

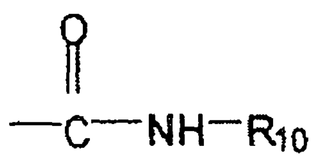

(e)

worin $R_{10}$ für Wasserstoff, eine $C_1$-$C_4$-Alkyl-, Hydroxyl- oder Aminogruppe, eine obige Gruppe (c) oder eine der Gruppen:

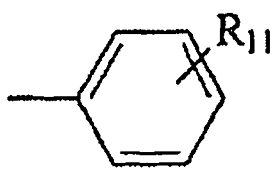

oder

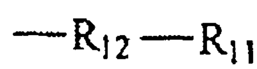

(f)

(g)

worin $R_{11}$ für eine Gruppe (a) steht und $R_{12}$ für einen $C_1$-$C_6$-Alkylenrest steht,

- eine Gruppe der Formel:

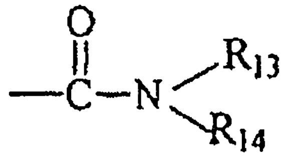

(h)

worin $R_{13}$ und $R_{14}$ gleich oder verschieden sind und für einen $C_1$-$C_4$-Alkylrest oder eine eine $C_1$-$C_4$-Hydroxyalkylgruppe stehen,

- eine der Gruppen der folgenden Formel:

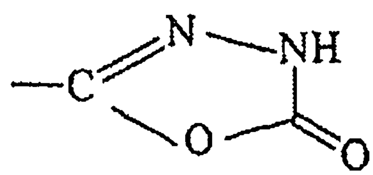

(j)

oder

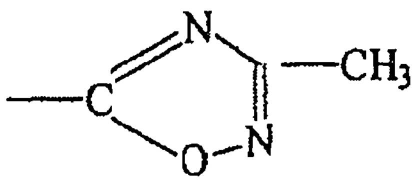

oder

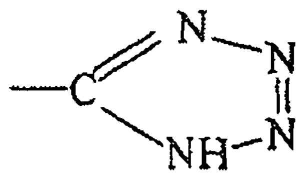

(k) (l)

steht,

$R_1$ für eine $C_1$-$C_6$-Alkylgruppe oder eine Gruppe der Formel:

$$-(CH_2)_p-R_{11} \quad \text{(m)}$$

worin $R_{11}$ die gleiche Bedeutung wie oben besitzt und p für 1 bis 4 steht, steht,
Am für eine stickstoffhaltige Gruppe der Formel:

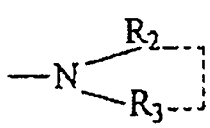

oder

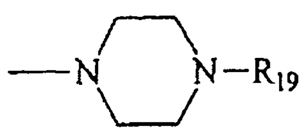

oder

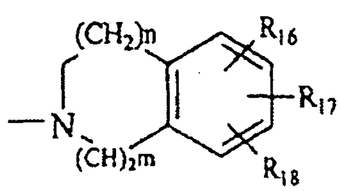

($Am_1$) ($Am_2$) ($Am_3$)

worin:

$R_2$ für Wasserstoff, eine lineare oder verzweigte $C_1$-$C_6$-Alkylgruppe, die gegebenenfalls durch eine Hydroxylgruppe substituiert ist, eine Gruppe (m), eine $C_3$-$C_6$-Cycloalkylgruppe oder eine Benzylgruppe steht, $R_3$ für eine lineare oder verzweigte $C_1$-$C_6$-Alkylgruppe, die gegebenenfalls durch eine Hydroxylgruppe substituiert ist, eine $C_3$-$C_6$-Cycloalkylgruppe, eine Gruppe (m), eine Benzylgruppe oder eine Phenylgruppe der Formel:

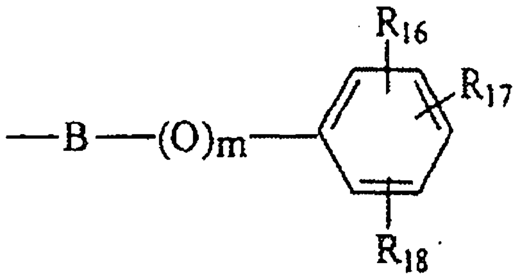

(n)

worin $R_{16}$, $R_{17}$ und $R_{18}$ gleich oder verschieden sind und für Wasserstoff oder eine Hydroxyl-, Nitro-, Amino-, $C_1$-$C_4$-Alkoxy- oder $C_1$-$C_4$-Alkylsulfonamidogruppe stehen, steht,
$R_{19}$ für Wasserstoff, eine $C_1$-$C_5$-Alkylgruppe, eine Diphenylmethylgruppe, eine Mono-, Di- oder Trimethylphenylgruppe, eine Mono-, Di- oder Trimethoxyphenylgruppe, eine Gruppe (a), eine Gruppe (b) oder eine Gruppe (c) steht,
m und n jeweils für 0 oder 1 stehen,
$R_2$ und $R_3$ zusammengenommen für eine lineare oder verzweigte $C_3$-$C_{10}$-Alkylengruppe, die gegebenenfalls durch eine Hydroxylgruppe, eine Gruppe (a) oder eine Gruppe (m) substituiert und gegebenenfalls durch -O- unterbrochen ist, stehen, steht,
W, W' und Z so beschaffen sind, daß:

- Z für -O- oder -S- steht, wenn W und W' gleich sind und für CH stehen,
- Z für $-CH=\overset{|}{C}-R_{21}$ steht, wenn W für CH und W' für C-$R_{20}$ steht, wobei $R_{20}$ und $R_{21}$ gleich oder verschieden sind und für Wasserstoff, ein Halogenatom, einen $C_1$-$C_4$-Alkylrest oder einen $C_1$-$C_4$-Alkoxyrest stehen,

X für -O- oder -S- steht,
Y für einen -CO- oder -$CH_2$-Rest, einen Rest der Formel:

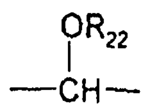

(73)

worin $R_{23}$ Wasserstoff, einen $C_1$-$C_4$-Alkylrest oder einen Acylrest der Formel:

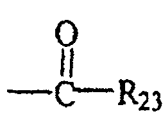

(74)

worin $R_{23}$ für einen $C_1$-$C_4$-Alkylrest steht, bedeutet, oder

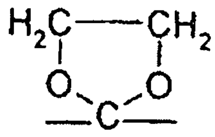

steht,
mit der Maßgabe, daß die Gruppen R, $R_1$ und Am insgesamt 0, 1 oder 2 Gruppen (a) enthalten,
wobei diese Benzofuran- oder Benzothiophenderivate in Form von einzelnen Isomeren oder Isomerengemischen vorliegen.

**2.** Benzofuran- oder Benzothiophenderivate nach Anspruch 1, worin Y für einen -CO-Rest oder

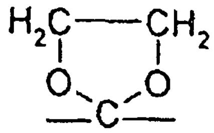

steht.

**3.** Benzofuran- oder Benzothiophenderivate nach Anspruch 1 oder 2, worin R für eine Isopropoxycarbonylgruppe steht.

**4.** Benzofuran- oder Benzothiophenderivate nach einem der Ansprüche 1 bis 3, worin Am für eine Diethylpiperidinylgruppe steht.

**5.** Benzofuran- oder Benzothiophenderivate nach einem der Ansprüche 1 bis 4, worin Y für einen -CO-Rest steht.

**6.** Benzofuran- oder Benzothiophenderivate nach einem der Ansprüche 1 bis 5, worin

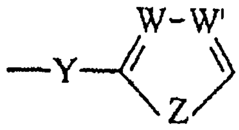

für einen Benzoylrest steht.

**7.** Benzofuran- oder Benzothiophenderivate nach einem der Ansprüche 1 bis 6, worin

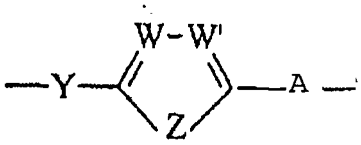

für einen 4-Oxybenzoylrest steht.

**8.** Benzofuran- oder Benzothiophenderivate nach einem der Ansprüche 1 bis 7, worin die Kette:

A-B-Am

sich in 4-Position befindet.

**9.** Benzofuran- oder Benzothiophenderivate nach einem der Ansprüche 1 bis 8, worin $R_1$ für n-Butyl steht, B für eine Propylengruppe steht und Am für eine Diethylpiperidinylgruppe steht.

**10.** Benzofuran- oder Benzothiophenderivate nach Anspruch 9, worin es sich bei der Diethylpiperidinylgruppe um eine 3,5-Diethylpiperidinylgruppe handelt.

**11.** Benzofuran- oder Benzothiophenderivate nach einem der Ansprüche 1 bis 10, worin das pharmazeutisch unbedenkliche Salz unter Oxalat-, Maleat-, Fumarat-, Methanesulfonat-, Benzoat-, Ascorbat-, Pamoat-, Succinat-, Hexamat-, Bismethylensalicylat-, Ethandisulfonat-, Acetat-, Propionat-, Tartrat-, Salicylat-, Citrat-, Gluconat-, Lactat-, Malat-, Cinnamat-, Mandelat-, Citraconat-, Aspartat-, Palmitat-, Stearat-, Itaconat-, Glykolat-, p-Aminobenzoat-, Glutamat-, Benzolsulfonat-, p-Toluolsulfonat- und Theophyllinacetatsalzen und Aminosäuresalzen, wie einem Lysin- oder Histidinsalz, ausgewählt sind.

**12.** Benzofuran- oder Benzothiophenderivate nach einem der Ansprüche 1 bis 10, worin das pharmazeutisch unbedenkliche Salz unter Hydrochlorid-, Hydrobromid-, Sulfat-, Sulfamat-, Phosphat- und Nitratsalzen ausgewählt ist.

**13.** Verfahren zur Herstellung von Benzofuran- oder Benzothiophenderivaten nach Anspruch 1, worin Y für eine -CO-Gruppe steht, $R_1$, ($Am_1$) und ($Am_2$) keine Carboxyl- oder Alkalimetallcarboxylatgruppe enthalten, $R_{16}$ und/oder $R_{17}$ und/oder $R_{18}$ von einer Amino- oder $C_1$-$C_4$-Alkylsulfonamidogruppe verschieden sind und R für eine Cyano- oder Hydroxymethylgruppe, die Gruppe (k) oder eine Gruppe (a), worin $R_5$ eine $C_1$-$C_{10}$-Alkyl- oder $C_3$-$C_6$-Cycloalkylgruppe bedeutet, steht, **dadurch gekennzeichnet, daß** man ein Ketonderivat der allgemeinen Formel:

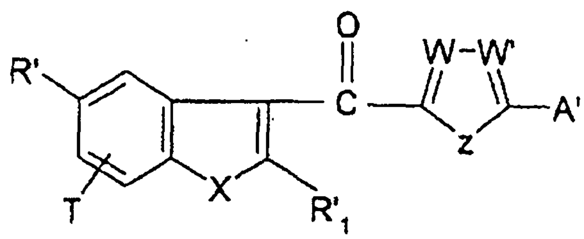

(2)

worin $R'_1$ für eine $C_1$-$C_6$-Alkylgruppe oder eine Gruppe (m), die keine Carboxyl- oder Alkalimetallcarboxylatgruppe enthält, steht, T, W, W', X und Z die gleiche Bedeutung wie in Anspruch 1 besitzen, A' für OH, SH oder $NH_2$ steht und R' für eine Cyano- oder Hydroxymethylgruppe, die Gruppe (k) oder eine Gruppe $-CO_2R''_5$, worin $R''_5$ einen $C_1$-$C_{10}$-Alkyl- oder $C_3$-$C_6$-Cycloalkylrest bedeutet, steht, in Gegenwart eines basischen Agens mit einer Verbindung der allgemeinen Formel:

$R_{24}$-B-Am' (3)

worin Am' für eine Gruppe ($Am_1$) oder ($Am_2$) gemäß der in Anspruch 1 angegebenen Definition, wobei diese Gruppe keine Carboxyl- oder Alkalimetallcarboxylatgruppe enthält, oder auch für eine Gruppe ($Am_3$) gemäß der in Anspruch 1 angegebenen Definition, worin $R_{16}$ und/oder $R_{17}$ und/oder $R_{18}$ von einer Amino- oder $C_1$-$C_4$-Alkylsulfonamidogruppe verschieden sind, steht, B die gleiche Bedeutung wie in Anspruch 1 besitzt und $R_{24}$ für:

- ein Halogenatom, einen $C_1$-$C_4$-Alkylsulfonyloxy- oder $C_6$-$C_{10}$-Arylsulfonyloxyrest steht, umsetzt, wodurch man die gewünschten Verbindungen, worin A für -O- oder -S- steht, in Form der freien Base erhält,

wobei man die so erhaltenen Verbindungen in Form der freien Base gegebenenfalls zur Bildung eines pharmazeutisch unbedenklichen Salzes mit einer geeigneten organischen oder anorganischen Säure behandelt.

**14.** Verfahren zur Herstellung von Benzofuran- oder Benzothiophenderivaten nach Anspruch 1, worin Y für eine -CO-Gruppe steht, $R_1$, ($Am_1$) und ($Am_2$) keine Carboxyl- oder Alkalimetallcarboxylatgruppe enthalten, $R_{16}$ und/oder $R_{17}$ und/oder $R_{18}$ von einer Amino- oder $C_1$-$C_4$-Alkylsulfonamidogruppe verschieden sind und R für eine Cyanogruppe, die Gruppe (k), eine $R_4$-O-N=CH-Gruppe oder eine Gruppe (a), worin $R_5$ eine $C_1$-$C_{10}$-Alkyl- oder $C_3$-$C_6$-Cycloalkylgruppe bedeutet, steht, **dadurch gekennzeichnet, daß** man eine Verbindung der allgemeinen Formel:

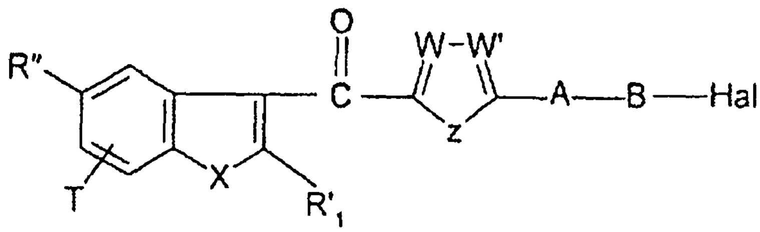

(4)

worin A, B, T, X, W, W', X und Z die gleiche Bedeutung wie in Anspruch 1 besitzen, R'$_1$ für eine $C_1$-$C_6$-Alkylgruppe oder eine Gruppe (m), die keine Carboxyl- oder Alkalimetallcarboxylatgruppe enthält, steht, R" für eine Cyanogruppe, die Gruppe (k), eine $R_4$-O-N=CH-Gruppe oder eine Gruppe -$CO_2$R"$_5$, worin R"$_5$ einen $C_1$-$C_{10}$-Alkyl- oder $C_3$-$C_6$-Cycloalkylrest bedeutet, steht und Hal für ein Halogenatom steht, mit einer gegebenenfalls in Salzform vorliegenden Verbindung der allgemeinen Formel:

H-Am' (5)

worin Am' für eine Gruppe ($Am_1$) oder ($Am_2$) gemäß der in Anspruch 1 angegebenen Definition, wobei diese Gruppe keine Carboxyl- oder Alkalimetallcarboxylatgruppe enthält, oder auch für eine Gruppe ($Am_3$) gemäß der in Anspruch 1 angegebenen Definition, worin $R_{16}$ und/oder $R_{17}$ und/oder $R_{18}$ von einer Amino- oder $C_1$-$C_4$-Alkylsulfonamidogruppe verschieden sind, steht, umsetzt, wobei man die Umsetzung in Gegenwart eines basischen Agens oder eines Überschusses des Amins der Formel (5) in Basenform durchführt, wodurch man die gewünschten Verbindungen in Form der freien Base erhält, welche man dann gegebenenfalls zur Bildung eines pharmazeutisch unbedenklichen Salzes mit einer geeigneten organischen oder anorganischen Säure umsetzen kann.

**15.** Verfahren zur Herstellung von Benzofuran- oder Benzothiophenderivaten nach Anspruch 1, worin Y für eine -CO-Gruppe steht, $R_1$, ($Am_1$) und ($Am_2$) keine Carboxyl- oder Alkalimetallcarboxylatgruppe enthalten, $R_{16}$ und/oder $R_{17}$ und/oder $R_{18}$ von einer Amino- oder $C_1$-$C_4$-Alkylsulfonamidogruppe verschieden sind und R für eine Cyanogruppe, eine $R_4$-O-N=CH-Gruppe oder eine Gruppe (a), worin $R_5$ eine $C_1$-$C_{10}$-Alkyl- oder $C_3$-$C_6$-Cycloalkylgruppe bedeutet, oder auch die Gruppe (k) steht, **dadurch gekennzeichnet, daß** man eine Verbindung der allgemeinen Formel:

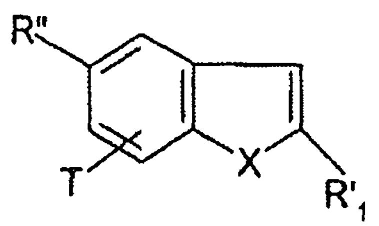

(6)

worin T und X die gleiche Bedeutung wie in Anspruch 1 besitzen, R" für eine Cyanogruppe, die Gruppe (k), eine $R_4$-O-N=CH-Gruppe oder eine Gruppe -$CO_2$R"$_5$, worin R"$_5$ einen $C_1$-$C_{10}$-Alkyl- oder $C_3$-$C_6$-Cycloalkylrest bedeutet, steht und R'$_1$ für eine $C_1$-$C_6$-Alkylgruppe oder eine Gruppe (m), die keine Carboxyl- oder Alkalimetallcarboxylatgruppe enthält, steht, mit einem Halogenid der allgemeinen Formel:

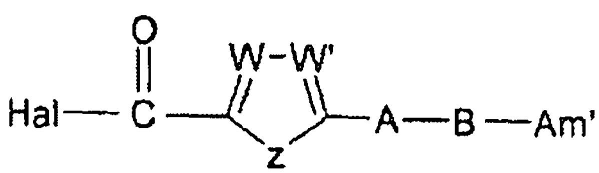

(7)

worin A, B, W, W' und Z die in Anspruch 1 angegebene Bedeutung besitzen, Hal für ein Halogenatom steht und

Am' für eine Gruppe ($Am_1$) oder ($Am_2$) gemäß der in Anspruch 1 angegebenen Definition, wobei diese Gruppe keine Carboxyl- oder Alkalimetallcarboxylatgruppe enthält, oder auch für eine Gruppe ($Am_3$) gemäß der in Anspruch 1 angegebenen Definition, worin $R_{16}$ und/oder $R_{17}$ und/oder $R_{18}$ von einer Amino- oder $C_1$-$C_4$-Alkylsulfonamidogruppe verschieden sind, steht, umsetzt, wobei man die Umsetzung in Gegenwart einer Lewis-Säure durchführt, wodurch man die gewünschten Verbindungen in Form der freien Base erhält, welche man dann gegebenenfalls zur Bildung eines pharmazeutisch unbedenklichen Salzes mit einer organischen oder anorganischen Säure umsetzen kann.

**16.** Verfahren zur Herstellung von Benzofuran- oder Benzothiophenderivaten nach Anspruch 1, worin Y für eine -CO-Gruppe steht, $R_1$, ($Am_1$) und ($Am_2$) keine Carboxyl- oder Alkalimetallcarboxylatgruppe enthalten, $R_{16}$ und/oder $R_{17}$ und/oder $R_{18}$ von einer Amino- oder $C_1$-$C_4$-Alkylsulfonamidogruppe verschieden sind und R für die Gruppe (j) steht, **dadurch gekennzeichnet, daß** man eine Verbindung der Formel:

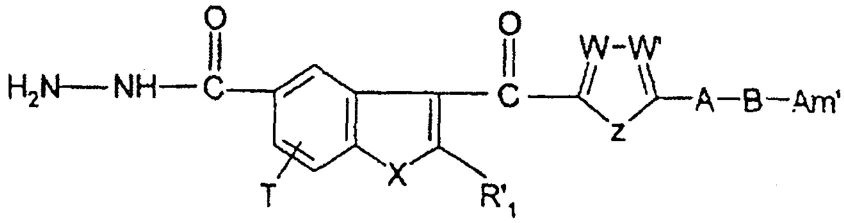

(9)

worin A, B, T, W, W', X und Z die gleiche Bedeutung wie in Anspruch 1 besitzen, $R'_1$ für eine $C_1$-$C_6$-Alkylgruppe oder eine Gruppe (m), die keine Carboxyl- oder Alkalimetallcarboxylatgruppe enthält, steht, und Am' für eine Gruppe ($Am_1$) oder ($Am_2$) gemäß der in Anspruch 1 angegebenen Definition, wobei diese Gruppe keine Carboxyl- oder Alkalimetallcarboxylatgruppe enthält, oder auch für eine Gruppe ($Am_3$) gemäß der in Anspruch 1 angegebenen Definition, worin $R_{16}$ und/oder $R_{17}$ und/oder $R_{18}$ von einer Amino- oder $C_1$-$C_4$-Alkylsulfonamidogruppe verschieden sind, steht, mit Phosgen umsetzt, wodurch man die gewünschten Verbindungen in Hydrochloridform erhält, welche man dann gegebenenfalls mit einem basischen Agens wie einem Alkalimetallhydroxid oder einem Alkalimetallcarbonat behandeln kann, wodurch man die gewünschten Verbindungen in Form der freien Base erhält, welche man gegebenenfalls zur Bildung eines pharmazeutisch unbedenklichen Salzes mit einer organischen oder anorganischen Säure umsetzen kann.

**17.** Verfahren zur Herstellung von Benzofuran- oder Benzothiophenderivaten nach Anspruch 1, worin Y für eine -CO-Gruppe steht, $R_1$, ($Am_1$) und ($Am_2$) keine Carboxyl- oder Alkalimetallcarboxylatgruppe enthalten, $R_{16}$ und/oder $R_{17}$ und/oder $R_{18}$ von einer Amino- oder $C_1$-$C_4$-Alkylsulfonamidogruppe verschieden sind und R für eine Gruppe (b), in der $R'_5$ eine Gruppe (c) vom Typ primäres Dialkylaminoalkyl bedeutet, steht, **dadurch gekennzeichnet, daß** man eine Verbindung der Formel:

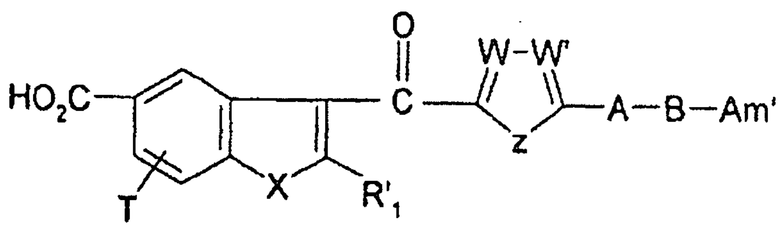

(10)

worin A, B, T, X, W, W', X und Z die gleiche Bedeutung wie in Anspruch 1 besitzen, $R'_1$ für eine $C_1$-$C_6$-Alkylgruppe oder eine Gruppe (m), die keine Carboxyl- oder Alkalimetallcarboxylatgruppe enthält, steht und Am' für eine Gruppe ($Am_1$) oder ($Am_2$) gemäß der in Anspruch 1 angegebenen Definition, wobei diese Gruppe keine Carboxyl- oder Alkalimetallcarboxylatgruppe enthält, oder auch für eine Gruppe ($Am_3$) gemäß der in Anspruch 1 angegebenen Definition, worin $R_{16}$ und/oder $R_{17}$ und/oder $R_{18}$ von einer Amino- oder $C_1$-$C_4$-Alkylsulfonamidogruppe verschieden sind, steht, und nach Schützung der Aminfunktion, wenn Am' für eine Gruppe ($Am_1$), worin $R_2$ Wasserstoff bedeutet, steht, mit einem Alkohol der allgemeinen Formel:

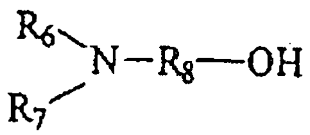

(11)

worin $R_6$ und $R_7$ die gleiche Bedeutung wie in Anspruch 1 besitzen und $R_8$ für eine lineare $C_1$-$C_8$-Alkylengruppe steht, umsetzt, wobei man die Umsetzung in Gegenwart von Carbonyldiimidazol und 1,8-Diazabicyclo[5.4.0]undec-7-en durchführt, und dann gegebenenfalls die gebildete Verbindung entschützt, wodurch man die gewünschten Verbindungen in Form der freien Base erhält, welche man gegebenenfalls zur Bildung eines pharmazeutisch unbedenklichen Salzes mit einer geeigneten organischen oder anorganischen Säure umsetzen kann.

**18.** Verfahren zur Herstellung von Benzofuran- oder Benzothiophenderivaten nach Anspruch 1, worin Y für eine -CO-Gruppe steht, $R_1$, ($Am_1$) und ($Am_2$) keine Carboxyl- oder Alkalimetallcarboxylatgruppe enthalten, $R_{16}$ und/oder $R_{17}$ und/oder $R_{18}$ von einer Amino- oder $C_1$-$C_4$-Alkylsulfonamidogruppe verschieden sind und R für eine Gruppe (b), in der $R'_5$ eine Gruppe (c) vom Typ sekundäres oder tertiäres Dialkylaminoalkyl bedeutet, steht, **dadurch gekennzeichnet, daß** man eine Verbindung der Formel:

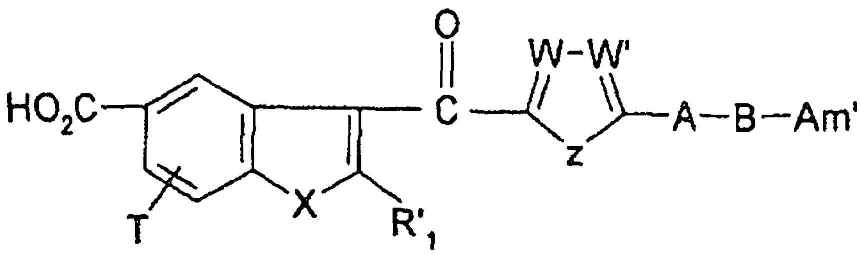

(10)

worin A, B, T, X, W, W' und Z die gleiche Bedeutung wie in Anspruch 1 besitzen, $R'_1$ für eine $C_1$-$C_6$-Alkylgruppe oder eine Gruppe (m), die keine Carboxyl- oder Alkalimetallcarboxylatgruppe enthält, steht und Am' für eine Gruppe ($Am_1$) oder ($Am_2$) gemäß der in Anspruch 1 angegebenen Definition, wobei diese Gruppe keine Carboxyl- oder Alkalimetallcarboxylatgruppe enthält, oder auch für eine Gruppe ($Am_3$) gemäß der in Anspruch 1 angegebenen Definition, worin $R_{16}$ und/oder $R_{17}$ und/oder $R_{18}$ von einer Amino- oder $C_1$-$C_4$-Alkylsulfonamidogruppe verschieden sind, steht, und nach Schützung der Aminfunktion, wenn Am' für eine Gruppe ($Am_1$), worin $R_2$ Wasserstoff bedeutet, steht, mit einem Halogenierungsmittel zu einem Acylhalogenid umsetzt, welches man dann mit einem Alkohol der Formel:

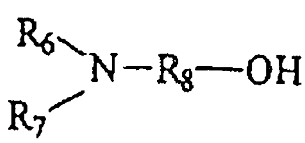

(11)

worin $R_6$ und $R_7$ die gleiche Bedeutung wie in Anspruch 1 besitzen und $R_8$ für eine sekundäre oder tertiäre $C_2$-$C_6$-Alkylengruppe steht, behandelt, und dann gegebenenfalls die gebildete Verbindung entschützt, wodurch man die gewünschten Verbindungen in Hydrohalogenidform bzw. bei Vorliegen der Verbindung der Formel (10) im Überschuß in Form der freien Base erhält, wobei man das Hydrohalogenid gegebenenfalls mit einem basischen Agens wie einem Alkalimetallhydroxid oder einem Alkalimetallcarbonat behandeln kann, wodurch man die gewünschten Verbindungen in Form der freien Base erhält, und die so gebildete freie Base gegebenenfalls zur Bildung eines pharmazeutisch unbedenklichen Salzes mit einer geeigneten organischen oder anorganischen Säure umsetzen kann.

**19.** Verfahren zur Herstellung von Benzofuran- oder Benzothiophenderivaten nach Anspruch 1, worin Y für eine -CO-Gruppe steht, $R_1$, ($Am_1$) und ($Am_2$) keine Carboxyl- oder Alkalimetallcarboxylatgruppe enthalten, $R_{16}$ und/oder $R_{17}$ und/oder $R_{18}$ von einer Amino- oder $C_1$-$C_4$-Alkylsulfonamidogruppe verschieden sind und R für eine Gruppe (a),

in der $R_5$ eine $C_1$-$C_{10}$-Alkyl- oder $C_3$-$C_6$-Cycloalkylgruppe bedeutet, oder eine Gruppe (b), in der $R'_5$ eine gegebenenfalls durch eine $C_1$-$C_4$-Alkylgruppe N-substituierte Piperidinylgruppe oder eine Gruppe (d), die keine Carboxyl- oder Alkalimetallcarboxylatgruppe enthält, bedeutet, steht, **dadurch gekennzeichnet, daß** man eine Verbindung der Formel:

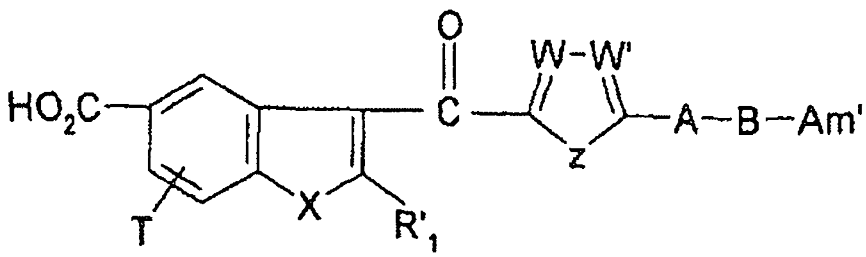

(10)

worin A, B, T, X, W, W' und Z die gleiche Bedeutung wie in Anspruch 1 besitzen, $R'_1$ für eine $C_1$-$C_6$-Alkylgruppe oder eine Gruppe (m), die keine Carboxyl- oder Alkalimetallcarboxylatgruppe enthält, steht und Am' für eine Gruppe ($Am_1$) oder ($Am_2$) gemäß der in Anspruch 1 angegebenen Definition, wobei diese Gruppe keine Carboxyl- oder Alkalimetallcarboxylatgruppe enthält, oder auch für eine Gruppe ($Am_3$) gemäß der in Anspruch 1 angegebenen Definition, worin $R_{16}$ und/oder $R_{17}$ und/oder $R_{18}$ von einer Amino- oder $C_1$-$C_4$-Alkylsulfonamidogruppe verschieden sind, steht, und nach Schützung der Aminfunktion, wenn Am' für eine Gruppe ($Am_1$), worin $R_2$ Wasserstoff bedeutet, steht, mit einem Halogenierungsmittel zu einem Acylhalogenid umsetzt, welches man dann mit einem Alkohol der Formel:

$R'''_5$-OH (12)

worin $R'''_5$ für eine $C_1$-$C_{10}$-Alkyl- oder $C_3$-$C_6$-Cycloalkylgruppe oder eine Gruppe (b), in der $R'_5$ eine gegebenenfalls durch eine $C_1$-$C_4$-Alkylgruppe N-substituierte Piperidinylgruppe oder eine Gruppe (d), die keine Carboxyl- oder Alkalimetallcarboxylatgruppe enthält, steht, behandelt, und dann gegebenenfalls die gebildete Verbindung entschützt, wodurch man die gewünschten Verbindungen in Hydrohalogenidform bzw. bei Vorliegen der Verbindung der Formel (10) im Überschuß in Form der freien Base erhält, wobei man das Hydrohalogenid gegebenenfalls mit einem basischen Agens wie einem Alkalimetallhydroxid oder einem Alkalimetallcarbonat behandeln kann, wodurch man die gewünschten Verbindungen in Form der freien Base erhält, und die so gebildete freie Base gegebenenfalls zur Bildung eines pharmazeutisch unbedenklichen Salzes mit einer geeigneten organischen oder anorganischen Säure umsetzen kann.

**20.** Verfahren zur Herstellung von Benzofuran- oder Benzothiophenderivaten nach Anspruch 1, worin Y für eine -CO-Gruppe steht, $R_1$, ($Am_1$) und ($Am_2$) keine Carboxyl- oder Alkalimetallcarboxylatgruppe enthalten, $R_{16}$ und/oder $R_{17}$ und/oder $R_{18}$ von einer Amino- oder $C_1$-$C_4$-Alkylsulfonamidogruppe verschieden sind und R für eine Gruppe (e), in der $R_{10}$ eine Gruppe (f) die keine Carboxyl- oder Alkalimetallcarboxylatgruppe enthält, bedeutet, steht, **dadurch gekennzeichnet, daß** man eine Verbindung der Formel:

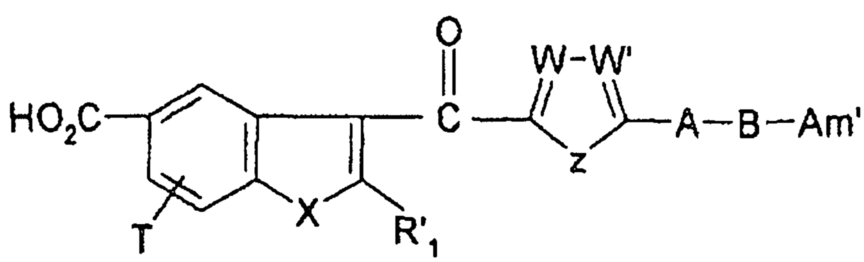

(10)

worin A, B, T, X, W, W' und Z die gleiche Bedeutung wie in Anspruch 1 besitzen, R'$_1$ für eine $C_1$-$C_6$-Alkylgruppe oder eine Gruppe (m), die keine Carboxyl- oder Alkalimetallcarboxylatgruppe enthält, steht und Am' für eine Gruppe ($Am_1$) oder ($Am_2$) gemäß der in Anspruch 1 angegebenen Definition, wobei diese Gruppe keine Carboxyl- oder Alkalimetallcarboxylatgruppe enthält, oder auch für eine Gruppe ($Am_3$) gemäß der in Anspruch 1 angegebenen Definition, worin $R_{16}$ und/oder $R_{17}$ und/oder $R_{18}$ von einer Amino- oder $C_1$-$C_4$-Alkylsulfonamidogruppe verschieden sind, steht, und nach Schützung der Aminfunktion, wenn Am' für eine Gruppe ($Am_1$), worin $R_2$ Wasserstoff bedeutet, steht, mit einem Halogenierungsmittel zu einem Acylhalogenid umsetzt, welches man dann mit einer Verbindung der Formel:

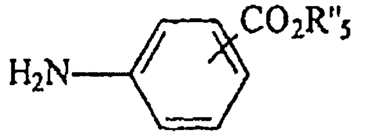

(13)

worin R''$_5$ für einen $C_1$-$C_{10}$-Alkyl- oder $C_3$-$C_6$-Cycloalkylrest steht, behandelt, und dann gegebenenfalls die gebildete Verbindung entschützt, wodurch man die gewünschten Verbindungen, worin $R_{10}$ für eine Gruppe (f) steht, deren Gruppe $R_{11}$ für eine Gruppe (a), worin $R_5$ eine $C_1$-$C_4$-Alkyl- oder $C_3$-$C_6$-Cycloalkylgruppe bedeutet, steht, in Form der freien Base erhält, wobei man die so gebildete freie Base gegebenenfalls zur Bildung eines pharmazeutisch unbedenklichen Salzes mit einer geeigneten organischen oder anorganischen Säure umsetzen kann.

**21.** Verfahren zur Herstellung von Benzofuran- oder Benzothiophenderivaten nach Anspruch 1, worin Y für eine -CO-Gruppe steht, $R_1$, ($Am_1$) und ($Am_2$) keine Carboxyl- oder Alkalimetallcarboxylatgruppe enthalten, $R_{16}$ und/oder $R_{17}$ und/oder $R_{18}$ von einer Amino- oder $C_1$-$C_4$-Alkylsulfonamidogruppe verschieden sind und R für eine Gruppe (e), in der $R_{10}$ eine $C_1$-$C_4$-Alkylgruppe, eine Aminogruppe oder eine Gruppe (c) bedeutet, steht, **dadurch gekennzeichnet, daß** man eine Verbindung der Formel:

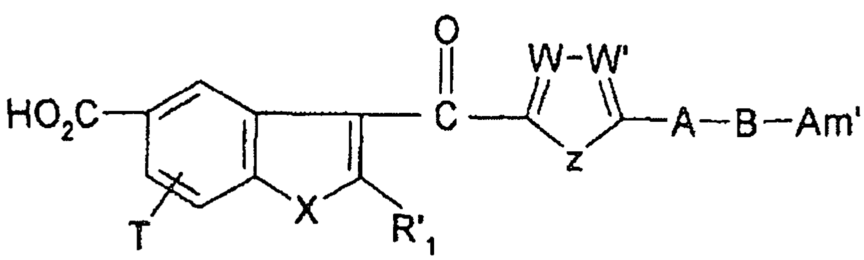

(10)

worin A, B, T, X, W, W' und Z die gleiche Bedeutung wie in Anspruch 1 besitzen, R'$_1$ für eine $C_1$-$C_6$-Alkylgruppe oder eine Gruppe (m), die keine Carboxyl- oder Alkalimetallcarboxylatgruppe enthält, steht und Am' für eine Gruppe ($Am_1$) oder ($Am_2$) gemäß der in Anspruch 1 angegebenen Definition, wobei diese Gruppe keine Carboxyl- oder Alkalimetallcarboxylatgruppe enthält, oder auch für eine Gruppe ($Am_3$) gemäß der in Anspruch 1 angegebenen Definition, worin $R_{16}$ und/oder $R_{17}$ und/oder $R_{18}$ von einer Amino- oder $C_1$-$C_4$-Alkylsulfonamidogruppe verschieden sind, steht, und nach Schützung der Aminfunktion, wenn Am' für eine Gruppe ($Am_1$), worin $R_2$ Wasserstoff bedeutet, steht, mit einem Halogenierungsmittel zu einem Acylhalogenid umsetzt, welches man dann mit einem Amin der allgemeinen Formel:

$R'_{10}$-$NH_2$ (14)

oder

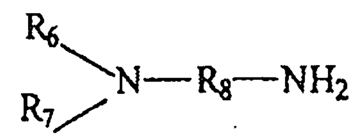

(15)

worin $R_6$, $R_7$ und $R_8$ die gleiche Bedeutung wie in Anspruch 1 besitzen und $R'_{10}$ für einen $C_1$-$C_5$-Alkyl- oder Aminorest steht, behandelt, und dann gegebenenfalls die gebildete Verbindung entschützt, wodurch man gegebenenfalls nach Basenbehandlung die gewünschte Verbindung der Formel (1), in Form eines Hydrohalogenids bzw. bei Vorliegen der Verbindung der Formel (10) im Überschuß in Form der freien Base erhält, wobei man das Hydrohalogenid gegebenenfalls mit einem basischen Agens wie einem Alkalimetallhydroxid oder einem Alkalimetallcarbonat behandeln kann, wodurch man die gewünschten Verbindungen in Form der freien Base erhält, und die so gebildete freie Base gegebenenfalls zur Bildung eines pharmazeutisch unbedenklichen Salzes mit einer geeigneten organischen oder anorganischen Säure umsetzen kann.

**22.** Verfahren zur Herstellung von Benzofuran- oder Benzothiophenderivaten nach Anspruch 1, worin Y für eine -CO-Gruppe steht, $R_1$, ($Am_1$) und ($Am_2$) keine Carboxyl- oder Alkalimetallcarboxylatgruppe enthalten, $R_{16}$ und/oder $R_{17}$ und/oder $R_{18}$ von einer Amino- oder $C_1$-$C_4$-Alkylsulfonamidogruppe verschieden sind und R für eine Gruppe (e), in der $R_{10}$ eine Gruppe (g), die keine Carboxyl- oder Alkalimetallcarboxylatgruppe enthält, bedeutet, steht, **dadurch gekennzeichnet, daß** man eine Verbindung der Formel:

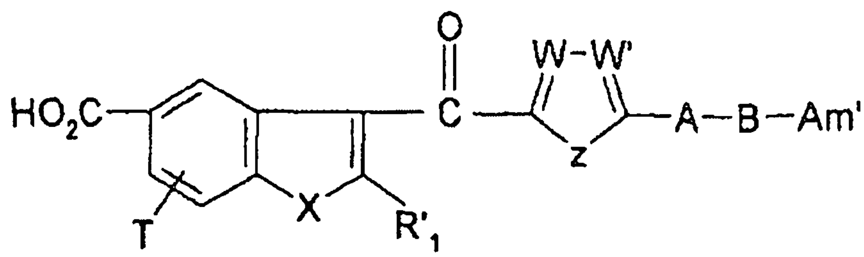

(10)

worin A, B, T, X, W, W' und Z die gleiche Bedeutung wie in Anspruch 1 besitzen, $R'_1$ für eine $C_1$-$C_6$-Alkylgruppe oder eine Gruppe (m), die keine Carboxyl- oder Alkalimetallcarboxylatgruppe enthält, steht und Am' für eine Gruppe ($Am_1$) oder ($Am_2$) gemäß der in Anspruch 1 angegebenen Definition, wobei diese Gruppe keine Carboxyl- oder Alkalimetallcarboxylatgruppe enthält, oder auch für eine Gruppe ($Am_3$) gemäß der in Anspruch 1 angegebenen Definition, worin $R_{16}$ und/oder $R_{17}$ und/oder $R_{18}$ von einer Amino- oder $C_1$-$C_4$-Alkylsulfonamidogruppe verschieden sind, steht, und nach Schützung der Aminfunktion, wenn Am' für eine Gruppe ($Am_1$), worin $R_2$ Wasserstoff bedeutet, steht, mit einem Salz der allgemeinen Formel:

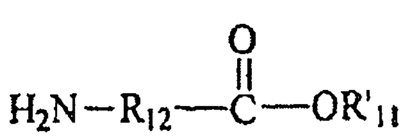

(16)

worin $R_{12}$ die gleiche Bedeutung wie in Anspruch 1 besitzt und $R'_{11}$ für einen $C_1$-$C_4$-Alkyl- oder $C_3$-$C_6$-Cycloalkylrest steht, umsetzt, wobei man die Umsetzung in Gegenwart eines Säurefängers durchführt, und dann gegebenenfalls die gebildete Verbindung entschützt, wodurch man die gewünschten Verbindungen, in denen $R_{10}$ für eine Gruppe (g) steht, deren Gruppe $R_{11}$ für eine Gruppe (a), worin $R_5$ eine $C_1$-$C_4$-Alkyl- oder $C_3$-$C_6$-Cycloalkylgruppe bedeutet,

steht, in Form der freien Base erhält, welche man gegebenenfalls zur Bildung eines pharmazeutisch unbedenklichen Salzes mit einer geeigneten organischen oder anorganischen Säure umsetzen kann.

**23.** Verfahren zur Herstellung von Benzofuran- oder Benzothiophenderivaten nach Anspruch 1, worin Y für eine -CO-Gruppe steht, $R_1$, $(Am_1)$ und $(Am_2)$ keine Carboxyl- oder Alkalimetallcarboxylatgruppe enthalten, $R_{16}$ und/oder $R_{17}$ und/oder $R_{18}$ von einer Amino- oder $C_1$-$C_4$-Alkylsulfonamidogruppe verschieden sind und R für eine Gruppe (h) steht, **dadurch gekennzeichnet, daß** man eine Verbindung der Formel:

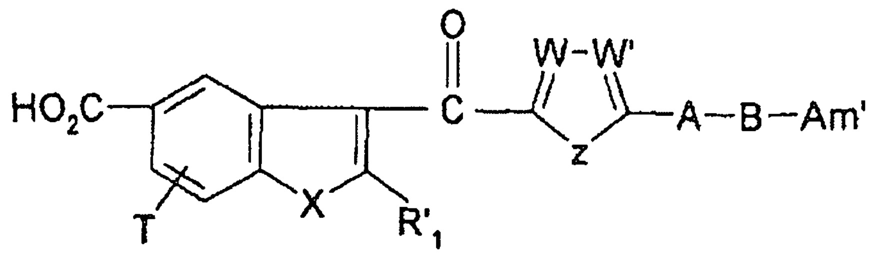

(10)

worin A, B, T, X, W, W' und Z die gleiche Bedeutung wie in Anspruch 1 besitzen, $R'_1$ für eine $C_1$-$C_6$-Alkylgruppe oder eine Gruppe (m), die keine Carboxyl- oder Alkalimetallcarboxylatgruppe enthält, steht und Am' für eine Gruppe $(Am_1)$ oder $(Am_2)$ gemäß der in Anspruch 1 angegebenen Definition, wobei diese Gruppe keine Carboxyl- oder Alkalimetallcarboxylatgruppe enthält, oder auch für eine Gruppe $(Am_3)$ gemäß der in Anspruch 1 angegebenen Definition, worin $R_{16}$ und/oder $R_{17}$ und/oder $R_{18}$ von einer Amino- oder $C_1$-$C_4$-Alkylsulfonamidogruppe verschieden sind, steht, und nach Schützung der Aminfunktion, wenn Am' für eine Gruppe $(Am_1)$, worin $R_2$ Wasserstoff bedeutet, steht, mit einem Halogenierungsmittel zu einem Acylhalogenid umsetzt, welches man dann mit einem Amin der allgemeinen Formel:

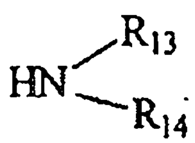

(17)

worin $R_{13}$ und $R_{14}$ die gleiche Bedeutung wie in Anspruch 1 besitzen, behandelt, und dann gegebenenfalls die gebildete Verbindung entschützt, wodurch man ein Salz der gewünschten Verbindung erhält, welches man mit einem geeigneten basischen Agens behandelt, wodurch man die gewünschten Verbindungen in Form der freien Base erhält, wobei man die so gebildete freie Base gegebenenfalls zur Bildung eines pharmazeutisch unbedenklichen Salzes mit einer organischen oder anorganischen Säure umsetzen kann.

**24.** Verfahren zur Herstellung von Benzofuran- oder Benzothiophenderivaten nach Anspruch 1, worin Y für eine -CO-Gruppe steht, $R_1$, $(Am_1)$ und $(Am_2)$ keine Carboxyl- oder Alkalimetallcarboxylatgruppe enthalten, $R_{16}$ und/oder $R_{17}$ und/oder $R_{18}$ von einer Amino- oder $C_1$-$C_4$-Alkylsulfonamidogruppe verschieden sind und R für eine Gruppe (e), in der $R_{10}$ eine Hydroxylgruppe bedeutet, steht, **dadurch gekennzeichnet, daß** man eine Verbindung der Formel:

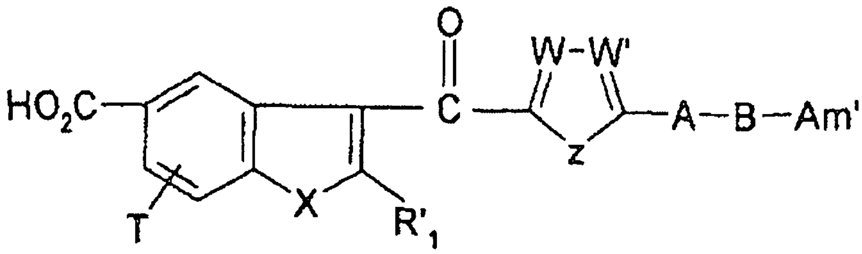

(10)

worin A, B, T, X, W, W' und Z die gleiche Bedeutung wie in Anspruch 1 besitzen, $R'_1$ für eine $C_1$-$C_6$-Alkylgruppe oder eine Gruppe (m), die keine Carboxyl- oder Alkalimetallcarboxylatgruppe enthält, steht und Am' für eine Gruppe ($Am_1$) oder ($Am_2$) gemäß der in Anspruch 1 angegebenen Definition, wobei diese Gruppe keine Carboxyl- oder Alkalimetallcarboxylatgruppe enthält, oder auch für eine Gruppe ($Am_3$) gemäß der in Anspruch 1 angegebenen Definition, worin $R_{16}$ und/oder $R_{17}$ und/oder $R_{18}$ von einer Amino- oder $C_1$-$C_4$-Alkylsulfonamidogruppe verschieden sind, steht, und nach Schützung der Aminfunktion, wenn Am' für eine Gruppe ($Am_1$), worin $R_2$ Wasserstoff bedeutet, steht, in Gegenwart eines Säurefängers und von Benzotriazol-1-yloxy-tris(dimethylamino)-phosphoniumhexafluorophosphat mit einem Salz von Benzyloxyamin umsetzt und dann gegebenenfalls die gebildete Verbindung entschützt, wodurch man Benzyloxyaminocarbonylderivate erhält, welche man in Gegenwart eines geeigneten Katalysators hydriert, wobei man die gewünschten Verbindungen in Form der freien Base erhält, welche man gegebenenfalls zur Bildung eines pharmazeutisch unbedenklichen Salzes mit einer geeigneten organischen oder anorganischen Säure umsetzen kann.

**25.** Verfahren nach einem der Ansprüche 18 bis 24, **dadurch gekennzeichnet, daß** man die Schützung der Aminfunktion durch Behandlung mit Chlorameisensäure-9-fluorenylmethylester und die Entschützung durch Behandlung mit einem sekundären Amin durchführt.

**26.** Verfahren zur Herstellung von Benzofuran- oder Benzothiophenderivaten nach Anspruch 1, worin Y für eine -CO-Gruppe steht, $R_1$, ($Am_1$) und ($Am_2$) keine Carboxyl- oder Alkalimetallcarboxylatgruppe enthalten, $R_{16}$ und/oder $R_{17}$ und/oder $R_{18}$ von einer Amino- oder $C_1$-$C_4$-Alkylsulfonamidogruppe verschieden sind und R für eine Gruppe (e), in der $R_{10}$ Wasserstoff bedeutet, steht, **dadurch gekennzeichnet, daß** man eine Verbindung der Formel:

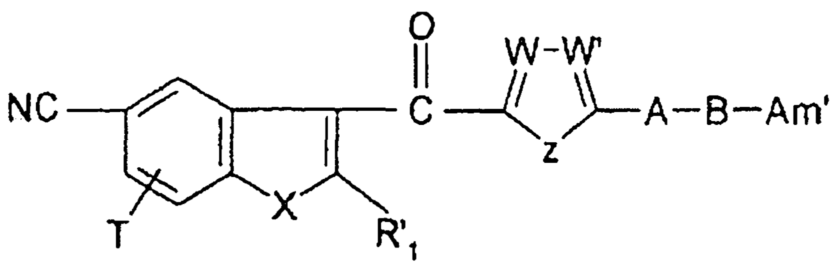

(18)

worin A, B, T, X, W, W' und Z die gleiche Bedeutung wie in Anspruch 1 besitzen, $R'_1$ für eine $C_1$-$C_6$-Alkylgruppe oder eine Gruppe (m), die keine Carboxyl- oder Alkalimetallcarboxylatgruppe enthält, steht und Am' für eine Gruppe ($Am_1$) oder ($Am_2$) gemäß der in Anspruch 1 angegebenen Definition, wobei diese Gruppe keine Carboxyl- oder Alkalimetallcarboxylatgruppe enthält, oder auch für eine Gruppe ($Am_3$) gemäß der in Anspruch 1 angegebenen Definition, worin $R_{16}$ und/oder $R_{17}$ und/oder $R_{18}$ von einer Amino- oder $C_1$-$C_4$-Alkylsulfonamidogruppe verschieden sind, steht, in Gegenwart einer starken Säure hydrolysiert, wobei man die gewünschten Verbindungen in Form der freien Base erhält, welche man gegebenenfalls zur Bildung eines pharmazeutisch unbedenklichen Salzes mit einer geeigneten organischen oder anorganischen Säure umsetzen kann.

**27.** Verfahren zur Herstellung von Benzofuran- oder Benzothiophenderivaten nach Anspruch 1, worin Y für eine -CO-

Gruppe steht, $R_1$, ($Am_1$) und ($Am_2$) keine Carboxyl- oder Alkalimetallcarboxylatgruppe enthalten, $R_{16}$ und/oder $R_{17}$ und/oder $R_{18}$ von einer Amino- oder $C_1$-$C_4$-Alkylsulfonamidogruppe verschieden sind und R für die Gruppe (1) steht, **dadurch gekennzeichnet, daß** man eine Verbindung der Formel:

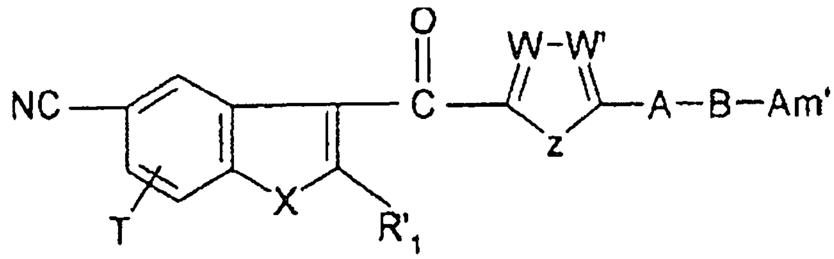

(18)

worin A, B, T, X, W, W' und Z die gleiche Bedeutung wie in Anspruch 1 besitzen, $R'_1$ für eine $C_1$-$C_6$-Alkylgruppe oder eine Gruppe (m), die keine Carboxyl- oder Alkalimetallcarboxylatgruppe enthält, steht und Am' für eine Gruppe ($Am_1$) oder ($Am_2$) gemäß der in Anspruch 1 angegebenen Definition, wobei diese Gruppe keine Carboxyl- oder Alkalimetallcarboxylatgruppe enthält, oder auch für eine Gruppe ($Am_3$) gemäß der in Anspruch 1 angegebenen Definition, worin $R_{16}$ und/oder $R_{17}$ und/oder $R_{18}$ von einer Amino- oder $C_1$-$C_4$-Alkylsulfonamidogruppe verschieden sind, steht, mit Tributylazidozinn umsetzt, wobei man die gewünschten Verbindungen in Form der freien Base erhält, welche man gegebenenfalls zur Bildung eines pharmazeutisch unbedenklichen Salzes mit einer geeigneten organischen oder anorganischen Säure umsetzen kann.

**28.** Verfahren zur Herstellung von Benzofuran- oder Benzothiophenderivaten nach Anspruch 1, worin Y für eine -CO-Gruppe steht, $R_1$ keine Carboxyl- oder Alkalimetallcarboxylatgruppe enthält und Am für eine Gruppe ($Am_1$), die keine Carboxyl- oder Alkalimetallcarboxylatgruppe enthält und in der $R_{16}$ und/oder $R_{17}$ und/oder $R_{18}$ für eine Aminogruppe stehen, oder eine Gruppe ($Am_3$), worin $R_{16}$ und/oder $R_{17}$ und/oder $R_{18}$ für eine Aminogruppe stehen, steht **dadurch gekennzeichnet, daß** man eine Nitroverbindung der Formel:

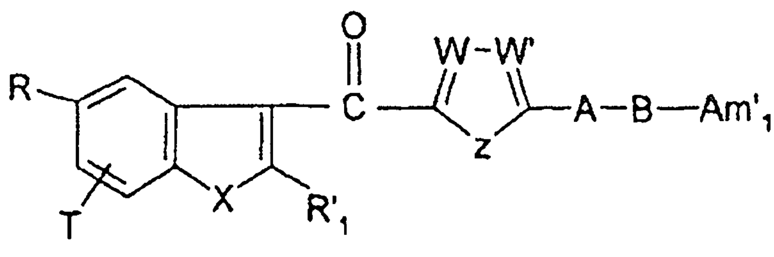

(19)

worin A, B, T, W, W', X und Z die gleiche Bedeutung wie in Anspruch 1 besitzen, R die gleiche Bedeutung wie in Anspruch 1 besitzt, aber keine Carboxyl- oder Alkalimetallcarboxylatgruppe enthält, $R'_1$ für eine $C_1$-$C_6$-Alkylgruppe oder eine Gruppe (m), die keine Carboxyl- oder Alkalimetallcarboxylatgruppe enthält, steht und $Am'_1$ für eine Gruppe ($Am_1$), die keine Carboxyl- oder Alkalimetallcarboxylatgruppe enthält und in der $R_{16}$ und/oder $R_{17}$ und/oder $R_{18}$ für eine Nitrogruppe stehen, oder eine Gruppe ($Am_3$), worin $R_{16}$ und/oder $R_{17}$ und/oder $R_{18}$ für eine Nitrogruppe stehen, in Gegenwart eines geeigneten Katalysators hydriert, wobei man die gewünschten Verbindungen in Form der freien Base erhält, welche man gegebenenfalls zur Bildung eines pharmazeutisch unbedenklichen Salzes mit einer geeigneten organischen oder anorganischen Säure umsetzen kann.

**29.** Verfahren zur Herstellung von Benzofuran- oder Benzothiophenderivaten nach Anspruch 1, worin Y für eine -CO-Gruppe steht, $R_1$ keine Carboxyl- oder Alkalimetallcarboxylatgruppe enthält und Am für eine Gruppe ($Am_1$), die keine Carboxyl- oder Alkalimetallcarboxylatgruppe enthält und in der $R_{16}$ und/oder $R_{17}$ und/oder $R_{18}$ für eine $C_1$-$C_4$-Al-

kylsulfonamidogruppe stehen, oder eine Gruppe ($Am_3$), worin $R_{16}$ und/oder $R_{17}$ und/oder $R_{18}$ für eine $C_1$-$C_4$-Alkylsulfonamidogruppe stehen, steht **dadurch gekennzeichnet, daß** man eine Aminoverbindung der Formel:

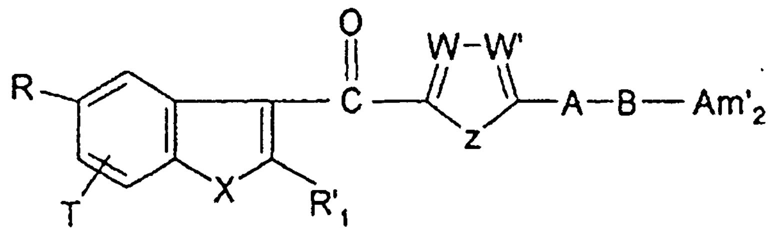

(20)

worin A, B, T, W, W', X und Z die gleiche Bedeutung wie in Anspruch 1 besitzen, R die gleiche Bedeutung wie in Anspruch 1 besitzt, aber keine Carboxyl- oder Alkalimetallcarboxylatgruppe enthält, $R'_1$ für eine $C_1$-$C_6$-Alkylgruppe oder eine Gruppe (m), die keine Carboxyl- oder Alkalimetallcarboxylatgruppe enthält, steht und $Am'_2$ für eine Gruppe ($Am_1$), die keine Carboxyl- oder Alkalimetallcarboxylatgruppe enthält und in der $R_{16}$ und/oder $R_{17}$ und/oder $R_{18}$ für eine Aminogruppe stehen, oder eine Gruppe ($Am_3$), worin $R_{16}$ und/oder $R_{17}$ und/oder $R_{18}$ für eine Aminogruppe stehen, gegebenenfalls in Gegenwart eines Säureakzeptors mit einem Halogenid der allgemeinen Formel:

$Hal\text{-}SO_2\text{-}R'_{16}$ (21)

oder einem Anhydrid der allgemeinen Formel:

$(R'_{16}SO_2)_2O$ (22)

worin $R'_{16}$ für einen linearen oder verzweigten $C_1$-$C_4$-Alkylrest steht, umsetzt, wobei man die gewünschten Verbindungen in Form der freien Base erhält, welche man gegebenenfalls zur Bildung eines pharmazeutisch unbedenklichen Salzes mit einer geeigneten organischen oder anorganischen Säure umsetzen kann.

**30.** Verfahren zur Herstellung von Benzofuran- oder Benzothiophenderivaten nach Anspruch 1, worin Y für eine -CO-Gruppe steht und eine oder zwei der Gruppen R, $R_1$ und ($Am_1$) bzw. ($Am_2$) eine Gruppe $-CO_2R_5$, in der $R_5$ für Wasserstoff oder ein Alkalimetallatom steht, enthalten, wobei die andere Gruppe bzw. die anderen Gruppen von einer Gruppe- $CO_2R_5$, in der $R_5$ für einen $C_1$-$C_{10}$-Alkyl- oder $C_3$-$C_6$-Cycloalkylrest steht, verschieden sind, **dadurch gekennzeichnet, daß** man eine Verbindung der Formel:

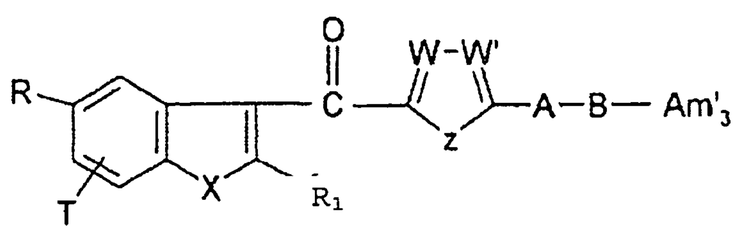

(23)

worin A, B, R, $R_1$, T, W, W', X und Z die gleiche Bedeutung wie in Anspruch 1 besitzen, $Am'_3$ für eine Gruppe ($Am_1$) oder ($Am_2$) gemäß der in Anspruch 1 angegebenen Definition steht und R, $R_1$ und ($Am_1$) bzw. ($Am_2$) so beschaffen sind, daß eine oder zwei davon eine Gruppe $-CO_2R''_5$, in der $R''_5$ für einen $C_1$-$C_{10}$-Alkyl- oder $C_3$-$C_6$-Cycloalkylrest steht, enthalten, wobei die andere Gruppe bzw. die anderen Gruppen von einer Gruppe $-CO_2R_5$, in der $R_5$ für einen $C_1$-$C_{10}$-Alkyl- oder $C_3$-$C_6$-Cycloalkylrest steht, verschieden sind, in Gegenwart eines Alkalimetallhydroxids verseift, wobei man die gewünschten Verbindungen der Formel (1), in der eine oder zwei der Gruppen R, $R_1$ und ($Am_1$) bzw. ($Am_2$) eine Gruppe $-CO_2R_5$, in der $R_5$ für ein Alkalimetallatom steht, enthalten, in Form der freien Base erhält, welche man gegebenenfalls mit einer starken Säure behandelt, wobei man die gewünschten Verbindungen der Formel (1), in der $R_5$ für Wasserstoff steht, in Form der freien Base erhält, welche man gegebenenfalls zur Bildung

eines pharmazeutisch unbedenklichen Salzes mit einer geeigneten organischen oder anorganischen Säure behandeln kann.

**31.** Verfahren zur Herstellung von Benzofuran- oder Benzothiophenderivaten nach Anspruch 1, worin Y für eine -CO-Gruppe steht und zwei der Gruppen R, $R_1$ und ($Am_1$) bzw. ($Am_2$) eine Gruppe $-CO_2R_5$, in der $R_5$ für Wasserstoff oder ein Alkalimetallatom steht, und eine Gruppe $-CO_2R_5$, in der $R_5$ für einen $C_1$-$C_{10}$-Alkyl- oder $C_3$-$C_6$-Cycloalkylrest steht, enthalten, wobei die dritte Gruppe von einer Gruppe $-CO_2R_5$, in der $R_5$ für einen $C_1$-$C_{10}$-Alkyl- oder $C_3$-$C_6$-Cycloalkylrest steht, verschieden ist, **dadurch gekennzeichnet, daß** man

- eine Verbindung der Formel:

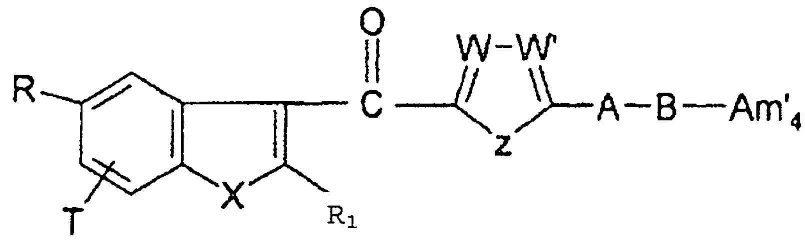

(24)

worin A, B, R, $R_1$, T, W, W', X und Z die gleiche Bedeutung wie in Anspruch 1 besitzen, $Am'_4$ für eine Gruppe ($Am_1$) oder ($Am_2$) gemäß der in Anspruch 1 angegebenen Definition steht und R, $R_1$ und ($Am_1$) bzw. ($Am_2$) so beschaffen sind, daß eine davon eine Gruppe $-CO_2R''_5$, in der $R''_5$ für einen $C_1$-$C_{10}$-Alkyl- oder $C_3$-$C_6$-Cycloalkylrest steht, enthält und eine andere davon eine Benzyloxycarbonylgruppe enthält,
wobei die dritte Gruppe von einer Gruppe $-CO_2R_5$, in der $R_5$ für einen $C_1$-$C_{10}$-Alkyl- oder $C_3$-$C_6$-Cycloalkylrest steht, verschieden ist, in Gegenwart eines geeigneten Katalysators hydriert,

- eine Verbindung der Formel:

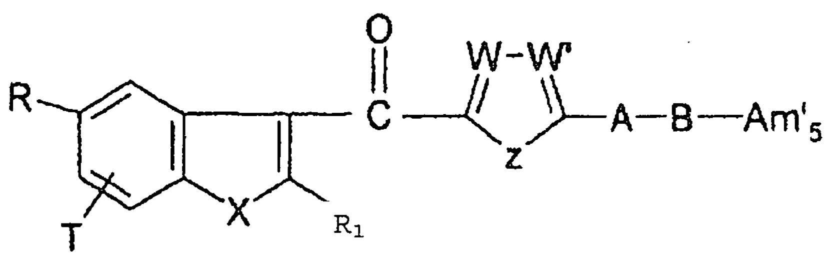

(25)

worin A, B, R, $R_1$, T, W, W', X und Z die gleiche Bedeutung wie in Anspruch 1 besitzen, $Am'_5$ für eine Gruppe ($Am_1$) oder ($Am_2$) gemäß der in Anspruch 1 angegebenen Definition steht und R, $R_1$ und ($Am_1$) bzw. ($Am_2$) so beschaffen sind, daß eine davon eine Gruppe $-CO_2R''_5$, in der $R''_5$ die gleiche Bedeutung wie oben besitzt, enthält und eine andere davon eine t-Butoxycarbonylgruppe enthält, wobei die dritte Gruppe von einer Gruppe $-CO_2R_5$, in der $R_5$ für einen $C_1$-$C_{10}$-Alkyl- oder $C_3$-$C_6$-Cycloalkylrest steht, verschieden ist, in Gegenwart von Trifluoressigsäure hydrolysiert,
wobei man die gewünschten Verbindungen der Formel (1), in der zwei der Gruppen R, $R_1$ und ($Am_1$) bzw. ($Am_2$) eine Gruppe $-CO_2R_5$, in der $R_5$ für eine $C_1$-$C_{10}$-Alkyl- oder $C_3$-$C_6$-Cycloalkylgruppe steht, und eine Gruppe $-CO_2R_5$, in der $R_5$ für Wasserstoff steht, enthalten, erhält, welche man gegebenenfalls mit einem geeigneten basischen Agens behandeln kann, wodurch man die gewünschten Verbindungen der Formel (1), in der zwei der Gruppen R,

$R_1$ und ($Am_1$) bzw. ($Am_2$) eine Gruppe -$CO_2R_5$, in der $R_5$ für ein Alkalimetall steht, und eine Gruppe -$CO_2R_5$, in der $R_5$ für eine $C_1$-$C_{10}$-Alkyl- oder $C_3$-$C_6$-Cycloalkylgruppe steht, enthalten, in Form der freien Base erhält, welche man gegebenenfalls mit einer starken Säure behandelt, wobei man die gewünschten Verbindungen der Formel (1), in der zwei der Gruppen R, $R_1$ und ($Am_1$) bzw. ($Am_2$) eine Carboxylgruppe und eine Gruppe -$CO_2R_5$, in der $R_5$ für eine $C_1$-$C_{10}$-Alkyl- oder $C_3$-$C_6$-Cycloalkylgruppe steht, enthalten, in Form der freien Base erhält,
wobei man die so gebildeten freien Basen gegebenenfalls zur Bildung eines pharmazeutisch unbedenklichen Salzes mit einer geeigneten organischen oder anorganischen Säure behandeln kann.

**32.** Verfahren zur Herstellung von Benzofuran- oder Benzothiophenderivaten nach Anspruch 1, worin Y für eine -CO-Gruppe steht, R für eine Cyanogruppe steht und eine der Gruppen $R_1$ und ($Am_1$) bzw. ($Am_2$) eine Carboxylgruppe enthält, **dadurch gekennzeichnet, daß** man eine Verbindung der Formel (1) gemäß Anspruch 1, in der Y für eine -CO-Gruppe steht, R für eine Cyanogruppe steht, A, B, $R_1$, T, W, W', X und Z die gleiche Bedeutung wie in Anspruch 1 besitzen, Am für eine Gruppe ($Am_1$) oder ($Am_2$) gemäß der in Anspruch 1 angegebenen Definition stehen und $R_1$, ($Am_1$) und ($Am_2$) so beschaffen sind, daß eine davon eine Gruppe -$CO_2R''_5$, worin $R''_5$ einen $C_1$-$C_{10}$-Alkyl- oder $C_3$-$C_6$-Cycloalkylrest bedeutet, enthält mit Tributylzinnoxid behandelt, wodurch man die gewünschten Verbindungen in Form der freien Base erhält, welche man dann gegebenenfalls zur Bildung eines pharmazeutisch unbedenklichen Salzes mit einer organischen oder anorganischen Säure umsetzen kann.

**33.** Verfahren zur Herstellung von Benzofuran- oder Benzothiophenderivaten nach Anspruch 1, worin Y für die Gruppe

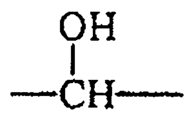

steht, **dadurch gekennzeichnet, daß** man eine Verbindung der Formel (1) gemäß Anspruch 1, in der Y für eine -CO-Gruppe steht und A, B, Am, R, $R_1$, W, W', X und Z die gleiche Bedeutung wie in Anspruch 1 besitzen, mit einem Alkalimetallborhydrid reduziert, wodurch man die gewünschten Verbindungen in Form der freien Base erhält, welche man dann gegebenenfalls zur Bildung eines pharmazeutisch unbedenklichen Salzes mit einer organischen oder anorganischen Säure umsetzen kann.

**34.** Verfahren zur Herstellung von Benzofuran- oder Benzothiophenderivaten nach Anspruch 1, worin Y für die Gruppe

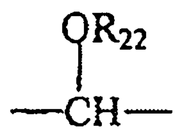

worin $R_{22}$ einen $C_1$-$C_4$-Alkylrest bedeutet, steht, **dadurch gekennzeichnet, daß** man eine Verbindung der Formel (1) gemäß Anspruch 1, in der Y für eine -CHOH-Gruppe steht und A, B, Am, R, $R_1$, W, W', X und Z die gleiche Bedeutung wie in Anspruch 1 besitzen, mit einem Alkalimetallalkoholat und dann mit einem Halogenid der Formel:

$R_{23}$-Hal

worin Hal für ein Halogenatom steht und $R_{23}$ für einen $C_1$-$C_4$-Alkylrest steht, umsetzt, wodurch man die gewünschten Verbindungen in Form der freien Base erhält, welche man dann gegebenenfalls zur Bildung eines pharmazeutisch unbedenklichen Salzes mit einer organischen oder anorganischen Säure umsetzen kann.

**35.** Verfahren zur Herstellung von Benzofuran- oder Benzothiophenderivaten nach Anspruch 1, worin Y für die Gruppe

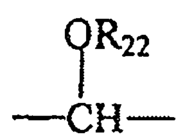

worin $R_{22}$ einen Acylrest der Formel $-CO-R_{23}$, worin $R_{23}$ für einen $C_1$-$C_4$-Alkylrest steht, bedeutet, steht, **dadurch gekennzeichnet, daß** man eine Verbindung der Formel (1) gemäß Anspruch 1, in der Y für eine -CHOH-Gruppe steht und A, B, Am, R, $R_1$, W, W', X und Z die gleiche Bedeutung wie in Anspruch 1 besitzen, mit einem Acylhalogenid der Formel:

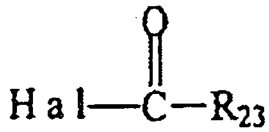

(27)

worin Hal für ein Halogenatom steht und $R_{23}$ für einen $C_1$-$C_4$-Alkylrest steht, umsetzt, wodurch man die gewünschten Verbindungen in Form der freien Base erhält, welche man dann gegebenenfalls zur Bildung eines pharmazeutisch unbedenklichen Salzes mit einer organischen oder anorganischen Säure umsetzen kann.

**36.** Verfahren zur Herstellung von Benzofuran- oder Benzothiophenderivaten nach Anspruch 1, worin Y für eine $-CH_2$-Gruppe steht, **dadurch gekennzeichnet, daß** man eine Verbindung der Formel (1) gemäß Anspruch 1, in der Y für eine -CHOH-Gruppe steht und A, B, Am, R, $R_1$, W, W', X und Z die gleiche Bedeutung wie in Anspruch 1 besitzen, mit einem Alkalimetallborhydrid und in Anwesenheit von Trifluoressigsäure reduziert, wodurch man die gewünschten Verbindungen in Form der freien Base erhält, welche man dann gegebenenfalls zur Bildung eines pharmazeutisch unbedenklichen Salzes mit einer geeigneten organischen oder anorganischen Säure umsetzen kann.

**37.** Medikament, **dadurch gekennzeichnet, daß** es ein Benzofuran- oder Benzothiophenderivat oder ein pharmazeutisch unbebdenkliches Salz davon gemäß einem der Ansprüche 1 bis 12 umfaßt.

**38.** Pharmazeutische oder veterinärmedizinische Zusammensetzung, **dadurch gekennzeichnet, daß** sie als Wirkstoff mindestens ein Benzofuran- oder Benzothiophenderivat oder ein pharmazeutisch unbedenkliches Salz davon gemäß einem der Ansprüche 1 bis 12 in Kombination mit einem pharmazeutischen Vehikel oder einem geeigneten Trägerstoff enthält.

**39.** Pharmazeutische oder veterinärmedizinische Zusammensetzung nach Anspruch 38 zur Behandlung von pathologischen Syndromen des Herz-Kreislauf-Systems.

**40.** Pharmazeutische oder veterinärmedizinische Zusammensetzung nach Anspruch 38 oder 39 zur Behandlung von Angina pectoris, Hypertonie, atrialer, ventrikulärer oder supraventrikulärer Arrhythmie, Hirnkreislaufinsuffizienz, Herzinsuffizienz und Myokardinfarkt mit oder ohne Komplikation durch Herzinsuffizienz oder zur Prävention von Postinfarkt-Mortalität.

**41.** Pharmazeutische oder veterinärmedizinische Zusammensetzung nach einem der Ansprüche 38 bis 40, **dadurch gekennzeichnet, daß** sie 50 bis 500 mg Wirkstoff enthält.

**42.** Verwendung mindestens eines Benzofuran- oder Benzothiophenderivats oder eines pharmazeutisch unbedenklichen Salzes davon gemäß einem der Ansprüche 1 bis 12 zur Herstellung eines Arzneimittels zur Behandlung von pathologischen Syndromen des Herz-Kreislauf-Systems.